# EUROPEAN PATENT SPECIFICATION

(11) **EP 0 573 573 B1**
(45) Date of publication and mention of the grant of the patent: **29.09.1999**
(21) Application number: 92907769.1
(22) Date of filing: 28.02.1992
(51) Int. Cl.: C08G 59/20, C08G 59/50, G02F 1/35

(54) **MESOGENIC GLYCIDYL AMINES**
Mesogene Glycidylamine
AMINES DE GLYCIDYLE MESOGENIQUES

(30) Priority: 01.03.1991 US 663499
(43) Date of publication of application: 15.12.1993
(73) Proprietor: THE DOW CHEMICAL COMPANY, Midland, Michigan 48674 (US)
(72) Inventor: HEFNER, Robert, E., Jr., Lake Jackson, TX 77566 (US); EARLS, Jimmy, D., Lake Jackson, TX 77566 (US)
(74) Representative: Casalonga, Axel
(86) International application number: US9201513
(87) International publication number: WO9215630

(56) References cited:
- EP-A- 0 252 359
- EP-A- 0 379 055
- EP-A- 0 430 142
- EP-A- 0 478 918
- BE-A- 628 786
- GB-A- 1 158 384

## Description

The present invention concerns particular glycidyl amines of mono and polyamines containing one or more mesogenic moieties, curable compositions and cured compositions thereof.

Glycidyl amines are a specialized class of thermosettable resins with utility in a myriad of applications, notably coatings, adhesives, encapsulants. moldings, laminates, castings, electrical insulation, weatherable coatings, sealants, impregnants. plasticizers, fibers, foams, and any application where a compound containing more than one vicinal epoxide group is suitably employed. The art describes numerous incremental improvements in the physical, mechanical, thermal and/or chemical resistant properties possessed by certain polyglycidyl amines relative to their polyglycidyl ether counterparts. This notwithstanding, substantial room for improvement in one or more of the aforesaid properties of polyglycidyl amines is desirable for each of the aforementioned applications.

The present invention provides a method for improving the properties of mono and polyglycidyl amines as well as the curable and cured compositions thereof by incorporating one or more mesogenic moieties into the structure of said glycidyl amines. These glycidyl amines exhibit ordering of the molecular chains in the melt phase and/or in the advanced compositions thereof. This morphology is susceptible to orientation during processing which can result in enhanced unidirectional mechanical properties. This is not possible to any significant extent with the conventional (non-mesogenic) glycidyl amines. The mesogenic structures incorporated into the structure of the glycidyl amines and the polymer chains of the resultant polymers thereof are believed to be responsible for the improvement in properties.

One aspect of the present invention pertains to mesogenic glycidyl amines containing one or more mesogenic moieties represented by the following Formulas I to VIII 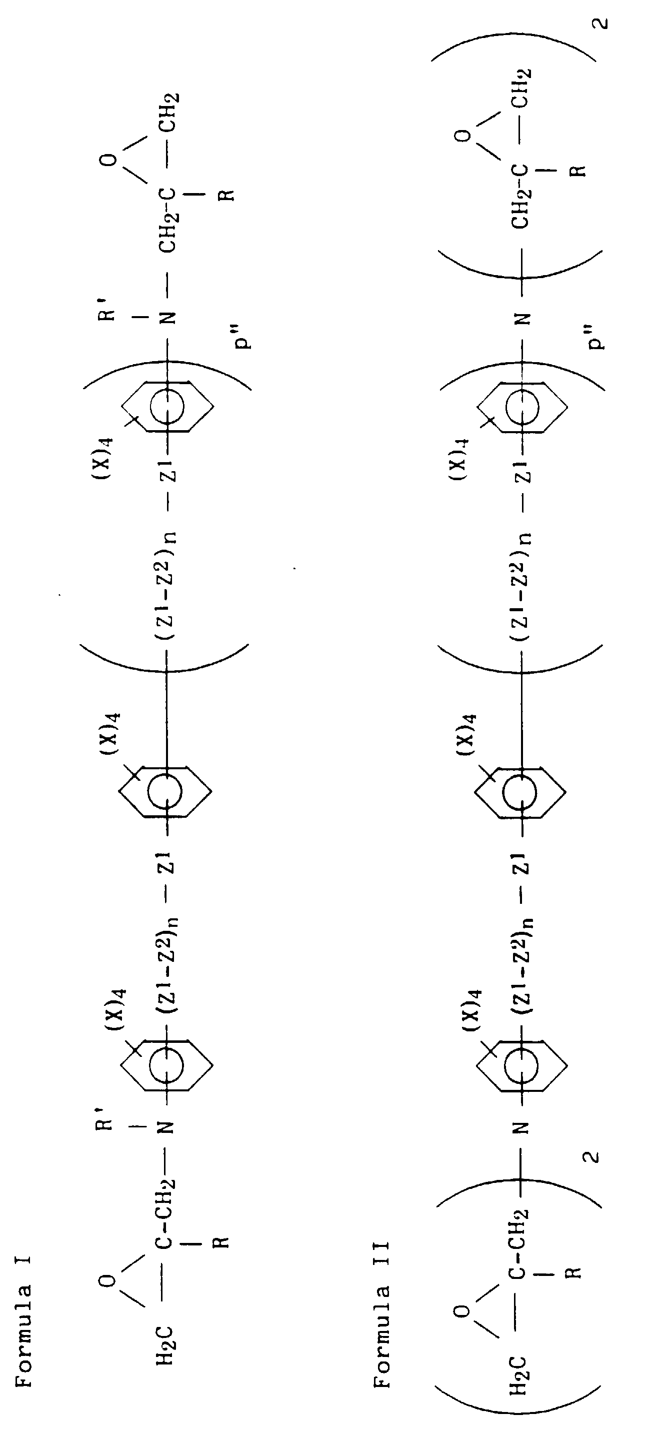 wherein at least 80 percent of the -(Z¹-Z²)n-Z¹-linkages and the glycidyl amine groups are in the para position with respect to each other; each R and R¹ is independently hydrogen or an aliphatic hydrocarbon group having from 1 to 4 carbon atoms; each R' is independently a hydrocarbyl group having suitably from 1 to 12, more suitably from 1 to 6, most suitably from 1 to 2, carbon atoms; each X is independently hydrogen, a hydrocarbyl or hydrocarbyloxy group having suitably from 1 to 12, more suitably from 1 to 6, most suitably from 1 to 4, carbon atoms, a halogen atom (preferably chlorine or bromine), -NO₂, or -C≡N; each Z¹ is independently a direct single bond, -CR¹=CR¹-, -CR¹=CR¹-CR¹=CR¹-, -CR¹=N-N=CR¹-, -CR¹=CR¹-CO-O-(CHR¹)_{p'}-, -CR¹=CR¹-O-CO-(CHR¹)_{p'}-, -(CHR¹)_{p'}-O-CO-CR¹=CR¹-, -(CHR¹)_{p'}-CO-O-CR¹=CR¹-, -CR¹=CR¹-CO-O-, -O-CO-CR¹=CR¹-, -CO-NR¹-, -NR¹-CO-, -CO-NR¹-NR¹-CO-, -C≡C-, -C≡C-C≡C-. -CO-S-, -S-CO-, -CR¹=N-, -N=CR¹-, -O-CO-, -CO-O-, -CR¹=CR¹-CO-, -CO-CR¹=CR¹-, -CR¹=CR¹-O-CO-, -CO-O-CR¹=CR¹-, -CH₂-CH₂-CO-O-, -O-CO-CH₂-CH₂-, -N=N-, 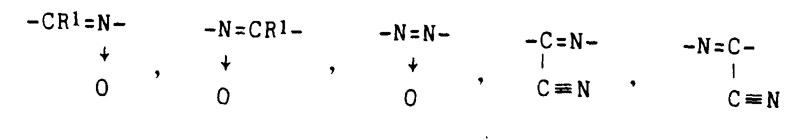 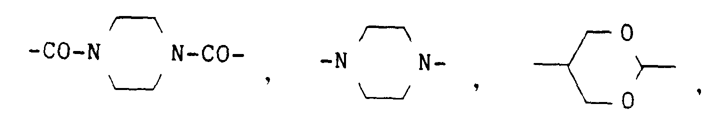 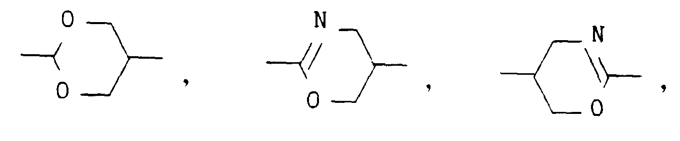 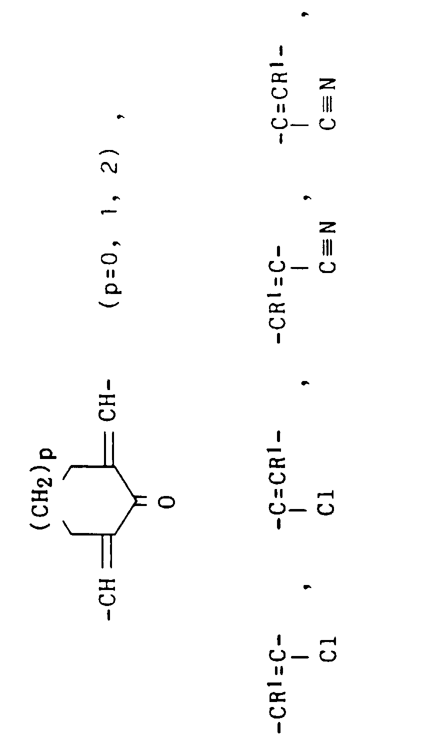 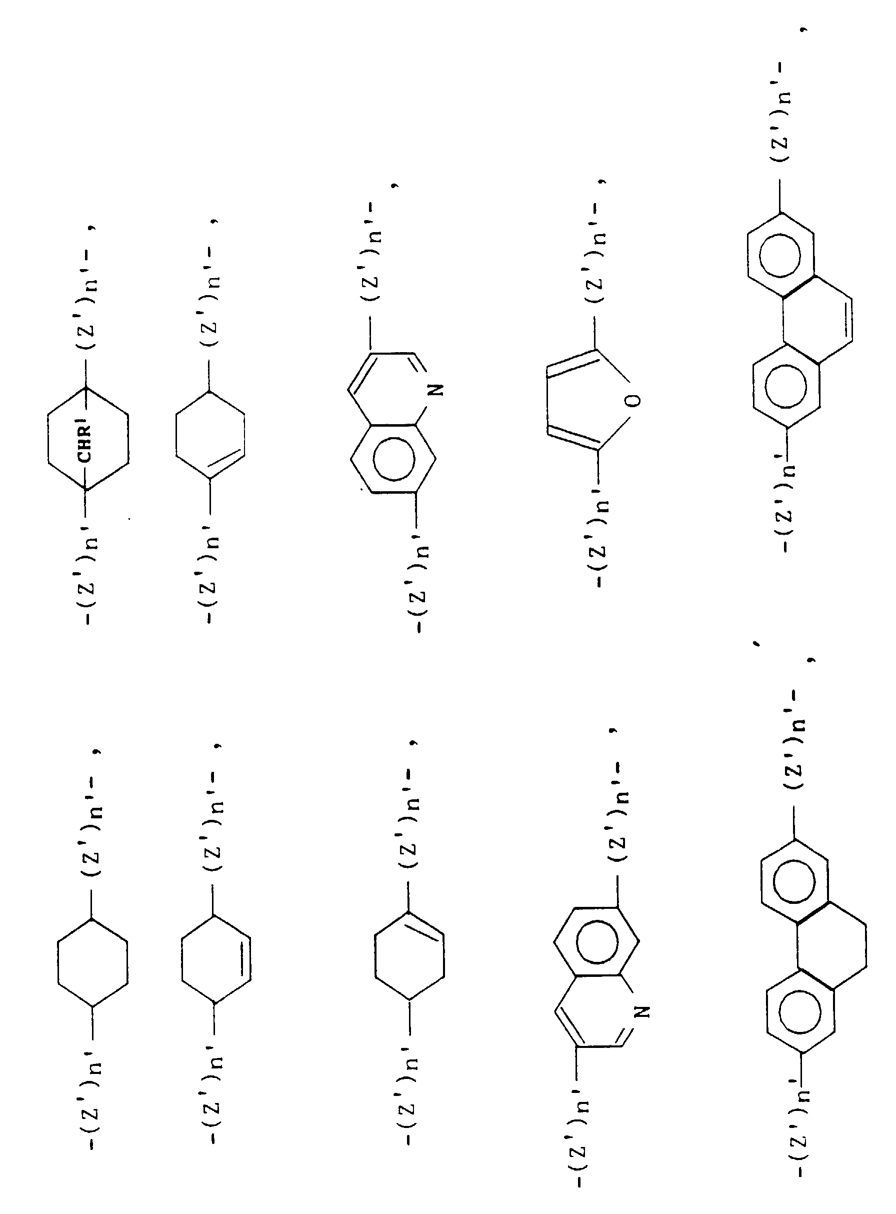 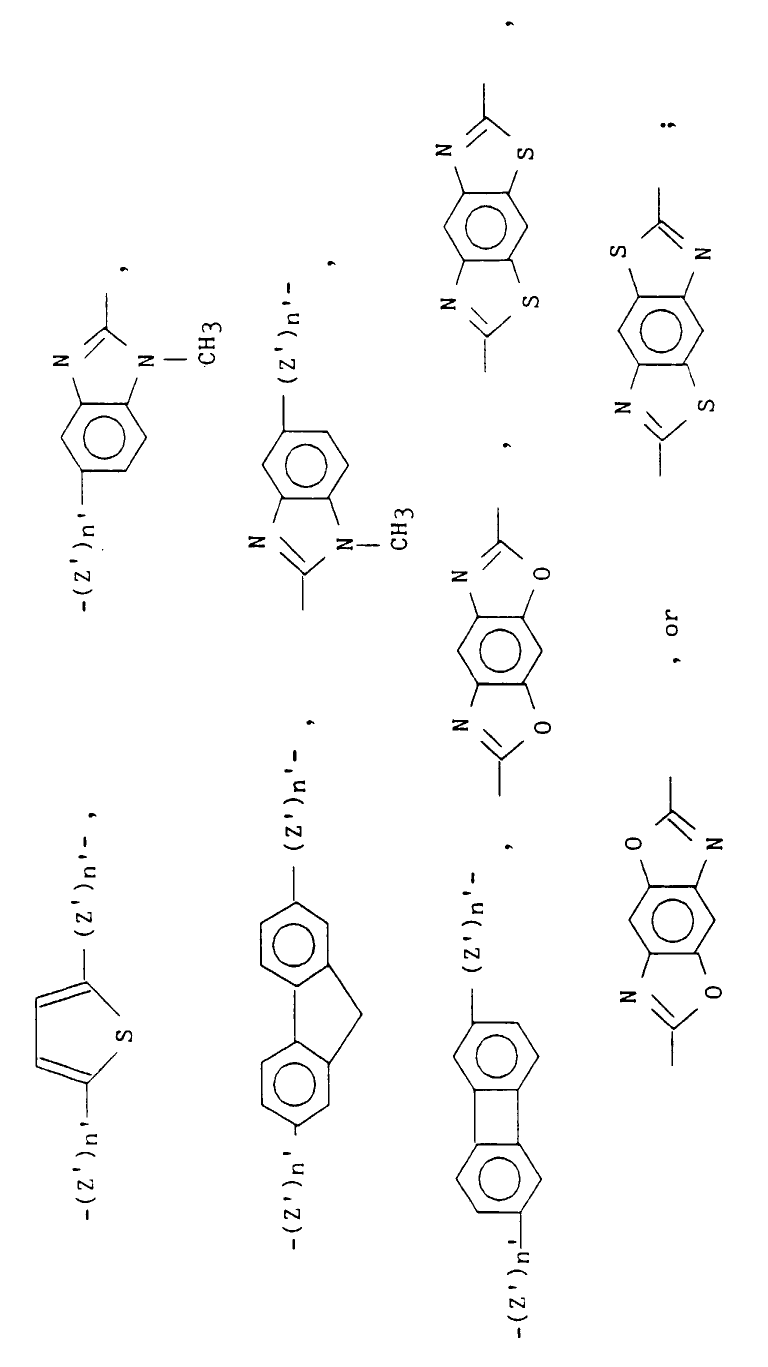 Z² is a group represented by a cyclic or bicyclic ring system containing from 5 to 12 carbon atoms and may be cycloaliphatic, polycycloaliphatic, aromatic or a combination thereof; n is 0 to 2; p' is 1 or 2; p" has a value of zero to 100; each Z' is independently a -CO-, -O-CO-, -CO-O-, -CO-NR¹-, or -NR¹-CO- group and each n' independently has a value of zero or one; with the proviso that the polyglycidyl amine of Formula II is not the tetraglycidyl amine of 4,4'-diaminobiphenyl (R is H, X is H, n=0, p"=, Z¹ is a direct single bond) or the tetraglycidyl amine of 3,3'-dimethyl-4,4'-diaminobiphenyl (R is H, X in the 3,3' position is -CH₃ and H in all other positions, n=0, p"=0, Z¹ is a direct single bond) and that in Formula II n and p" are not zero at the same time. 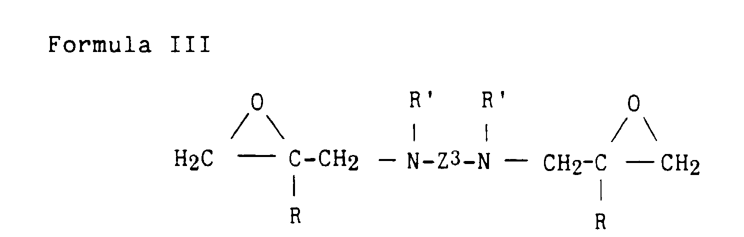 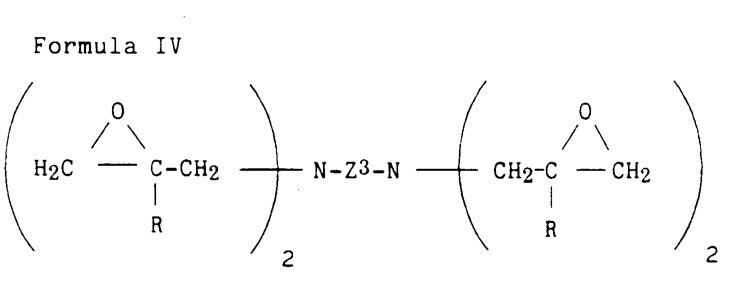 wherein Z³ is 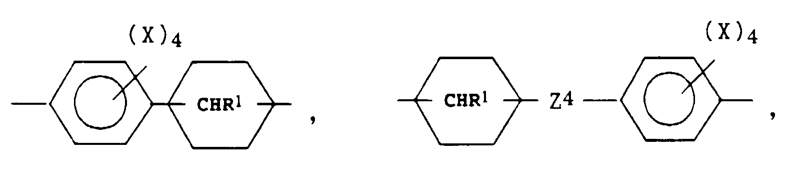 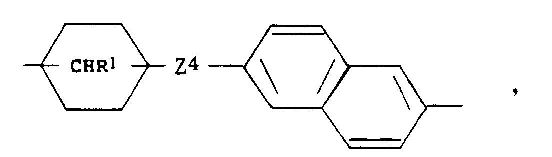 or 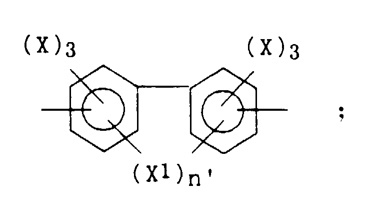 and Z⁴ is -CO-O-, -O-CO-, -NR¹-CO- or -CO-NR¹-; X¹ is a hydrocarbyl group having from 1 to 10, preferably from 1 to 4, carbon atoms which can contain one or more heteroatoms selected from N, O, or S and may be saturated or unsaturated; each R and R¹ is independently hydrogen or an aliphatic hydrocarbon group having from 1 to 4 carbon atoms: each R' is independently a hydrocarbyl group having suitably from 1 to 12, more suitably from 1 to 6, most suitably from 1 to 2, carbon atoms; each X is independently hydrogen, a hydrocarbyl or hydrocarbyloxy group having suitably from 1 to 12, more suitably from 1 to 6, most suitably from 1 to 4, carbon atoms, a halogen atom (preferably chlorine or bromine), -NO₂, or -C=N; and each n' is independently zero or one. 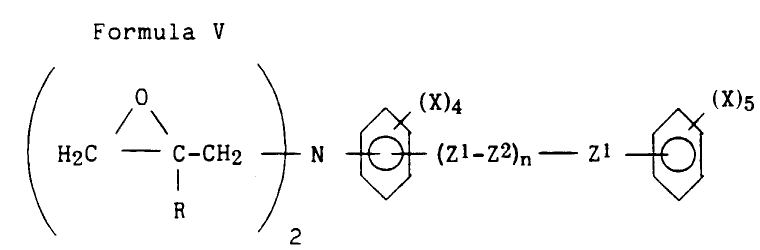 wherein at least 80 percent of the -(Z¹-Z²)ₙ-Z¹- linkages and the glycidyl amine groups are in the para position with respect to each other; R, R¹, X, Z¹, Z², Z', n, p' and n' are as hereinbefore defined. 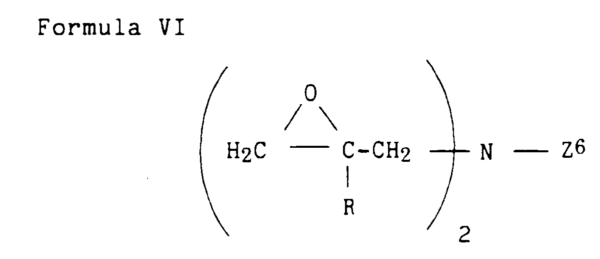 wherein R, R¹, X, X¹, Z⁴ and n' are as hereinbefore defined; and Z⁶ is 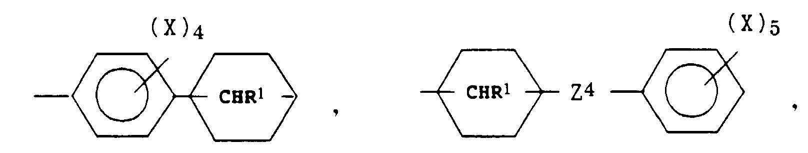 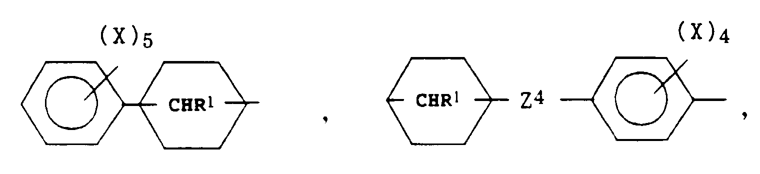 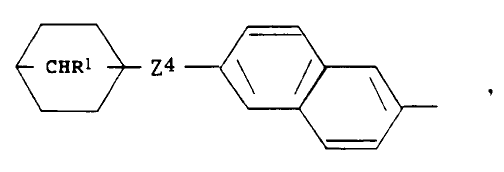 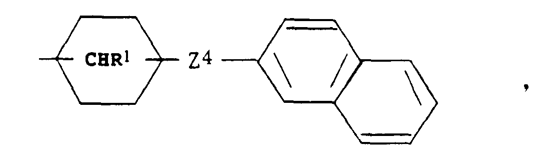 or 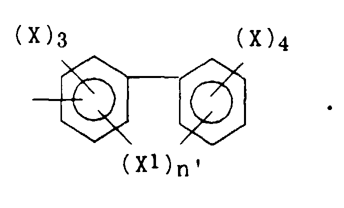 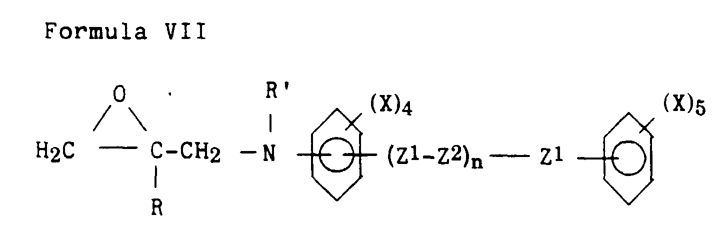 wherein at least 80 percent of the -(Z¹-Z²)ₙ-Z¹- linkages and the glycidyl amine groups are in the para position with respect to each other; R, R', R¹, X, Z¹, Z², Z', n, p' and n' are as hereinbefore defined. 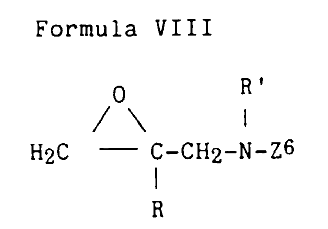 wherein R, R', R¹, X, X¹, Z³, Z⁴ and n' are as hereinbefore defined and Z⁶ is 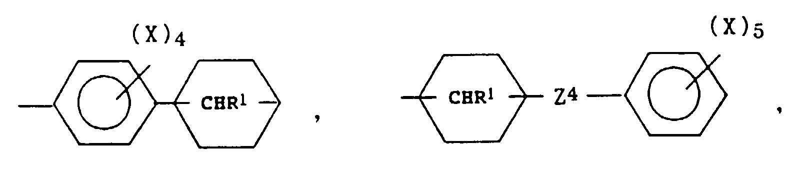 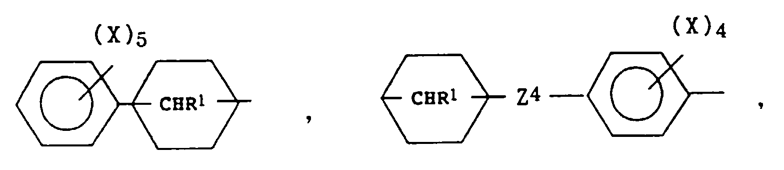 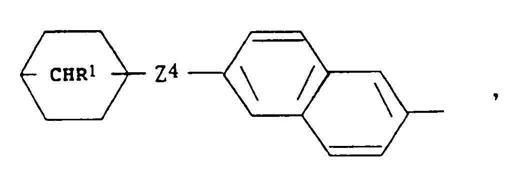 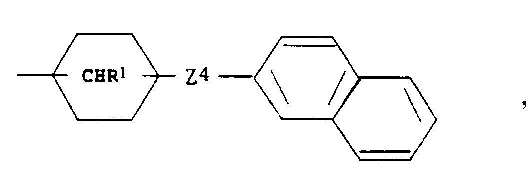 or

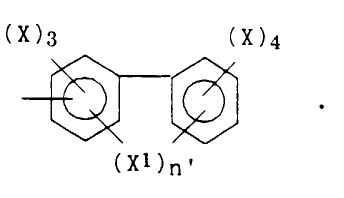

Another aspect of the present invention pertains to advanced polyglycidyl amine compositions prepared by reacting
(A) one or more of the polyglycidyl amines containing one or more mesogenic moieties, said polyglycidyl amines being those represented by Formulas I-VI, with the proviso that the polyglycidyl amine of Formula II may include the tetraglycidyl amine of 4,4'-diaminobiphenyl or the tetraglycidyl amine of 3,3'-dimethyl-4,4'-diaminobiphenyl; with
(B) at least one compound having an average of more than one active hydrogen atom per molecule; and wherein components (A) and (B) are employed in quantities which provide a ratio of active hydrogen atoms per epoxide group of from 0.01:1 to 0.95:1, more suitably from 0.05:1 to 0.8:1, most suitably from 0.1:1 to 0.5:1;
with the proviso that when component (A) contains polyglycidyl amine compounds containing an average of more than two glycidyl groups per molecule, such compounds are present in an amount such that less than 15, preferably less than 10, more preferably less than 5 percent of the glycidyl groups are contributed by the compounds containing an average of more than two glycidyl groups per molecule.

Another aspect of the present invention pertains to phenoxy type resin compositions prepared by the advancement reaction of
(A) one or more of the polyglycidyl amines containing one or more mesogenic moieties, said polyglycidyl amines being those represented by either Formulas I, III, V or VI; with
(B) at least one compound having an average of more than one active hydrogen atom per molecule; and
wherein components (A) and (B) are employed in quantities which provide a ratio of active hydrogen atoms per epoxide group of from 0.96:1 to 1.05:1.

Another aspect of the present invention pertains to blends of (A) one or more of the polyglycidyl amines or monoglycidyl amine compounds containing one or more mesogenic moieties which polyglycidyl amines or monoglycidyl amine compounds are represented by the aforementioned Formulas I-VIII with the proviso that the polyglycidyl amine of Formula II may include the tetraglycidyl amine of 4,4'-diaminobiphenyl or the tetraglycidyl amine of 3,3'-dimethyl-4,4'-diamino biphenyl; and (B) one or more polyepoxides represented by the following Formulas IX-XV; 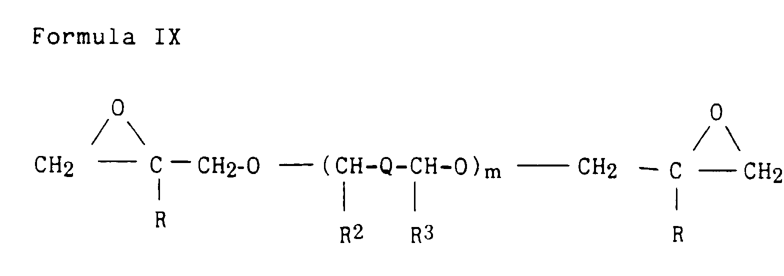 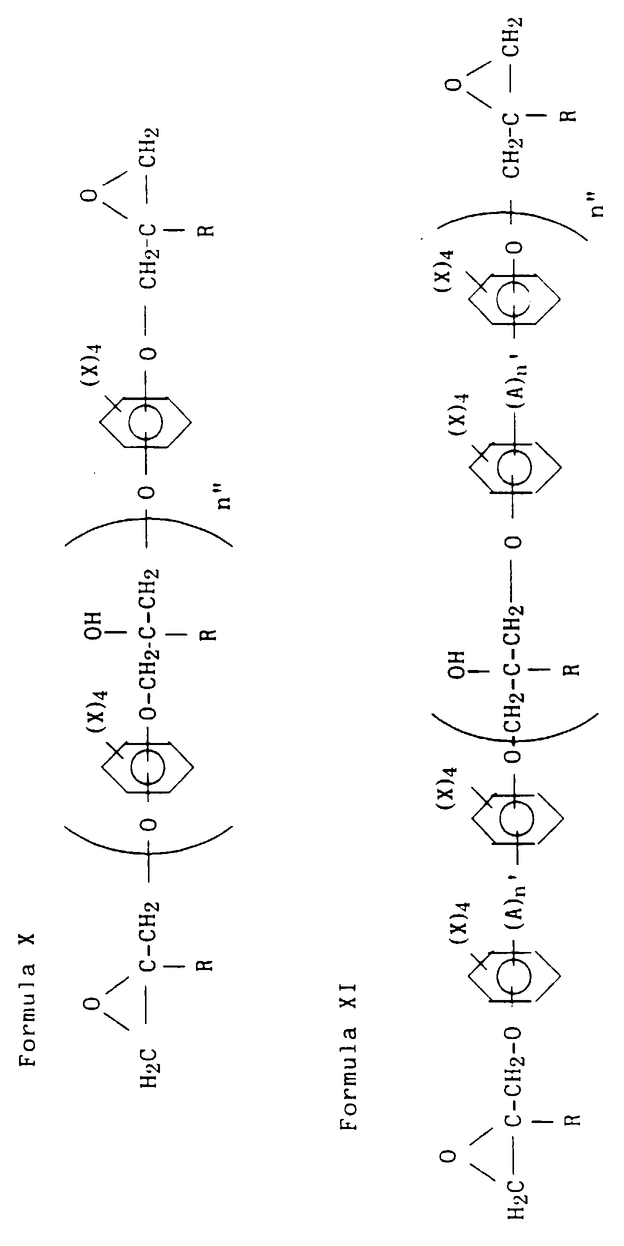 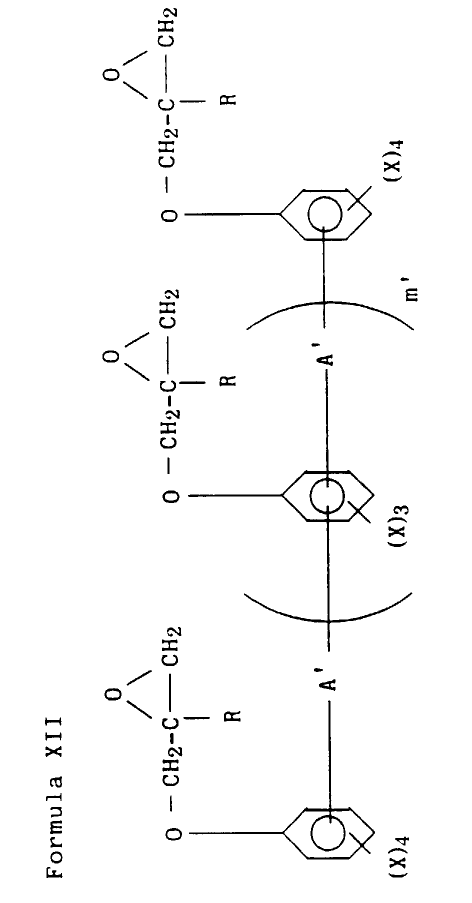 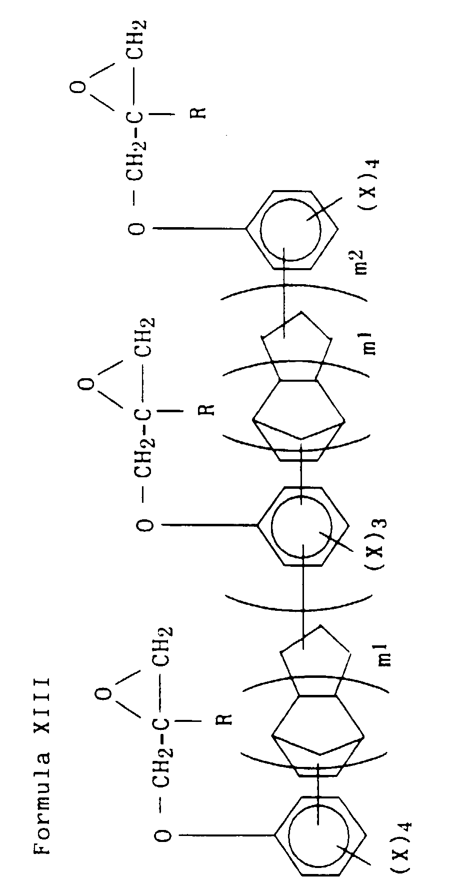 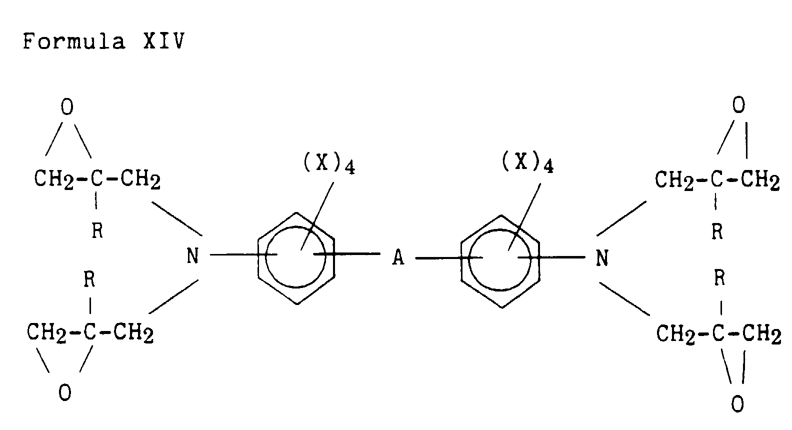 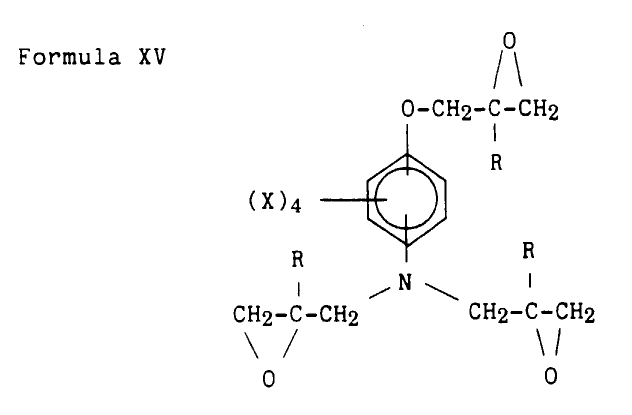 wherein each A is independently a divalent hydrocarbyl group having from 1 to 12, preferably from 1 to 6, more preferably from 1 to 3, carbon atoms, -O-, -S-, -S-S-,-SO-, -SO₂-, or -CO-; each A' is independently a divalent hydrocarbon group having from 1 to 6, preferably from 1 to 3, carbon atoms; Q is a single bond, -CH₂-S-CH₂-, -(CH₂)_{n¹}-, or 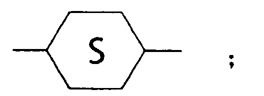 each R is independently hydrogen or an alkyl group having from 1 to 4 carbon atoms; each R² and R³ is independently hydrogen, a hydrocarbyl or halohydrocarbyl group having from 1 to 6, preferably from 1 to 3, more preferably from 1 to 2, carbon atoms; each X is independently hydrogen, a hydrocarbyl or hydrocarbyloxy group having from 1 to 12, preferably from 1 to 6, most preferably from 1 to 4, carbon atoms, a halogen atom,-NO₂ or - C≡N;
m has a value from 1 to 10, preferably from 1 to 4, more preferably from 1 to 2; m' has an average value from 0.01 to 12, preferably from 1 to 6, more preferably from 1 to 3; m¹ has an average value from 1 to 12, preferably from 1 to 6, more preferably from 1 to 3; m² has a value from 1 to 12, preferably from 2 to 6, more preferably from 2 to 3; n' has a value of zero or 1; n" has an average value from zero to 3, preferably from zero to 1.5, more preferably from zero to 0.5, and n¹ has an average value from 1 to 10; and wnerein component (A) is present in an amount suitably from 1 to 99, more suitably from 10 to 80, most suitably from 10 to 50, percent by weight based upon the combined weight of components (A) and (B) and component (B) is present in an amount suitably from 99 to 1, more suitably from 90 to 20, most suitably from 90 to 50, percent by weight based upon the combined weight of components (A) and (B).

Another aspect of the present invention pertains to blends of (A) one or more of the advanced polyglycidyl amines containing one or more mesogenic moieties which advanced polyglycidyl amines are prepared by reacting (1) one or more polyglycidyl amines represented by Formulas I -VI, with the proviso that the polyglycidyl amine of Formula II may include the tetraglycidyl amine of 4,4'-diaminobiphenyl or the tetraglycidyl amine of 3,3'-dimethyl-4,4'-diaminobiphenyl, and (2) at least one compound having an average of more than one active hydrogen atom per molecule; and (B) one or more polyepoxides represented by Formulas IX-XV; and wherein component (A) is present in an amount suitably from 1 to 99, more suitably from 10 to 80, most suitably from 10 to 50, percent by weight based upon the combined weight of components (A) and (B) and component (B) is present in an amount suitably from 99 to 1, more suitably from 90 to 20, most suitably from 90 to 50, percent by weight based upon the combined weight of components (A) and (B).

Another aspect of the present invention pertains to curable compositions comprising at least one polyglycidyl amine containing one or more mesogenic moieties represented by Formulas I-VI and a curing amount of a suitable curing agent therefor.

Another aspect of the present invention pertains to curable compositions comprising
(A) at least one polyglycidyl amine containing one or more mesogenic moieties, said polyglycidyl amine being represented by either Formulas I-VI, with the proviso that the polyglycidyl amine of Formula II may include the tetraglycidyl amine of 4,4'-diaminobiphenyl or the tetraglycidyl amine of 3,3'-dimethyl-4,4'-diaminobiphenyl;
(B) at least one of the aforementioned monoglycidyl amine compounds containing one or more mesogenic moieties, said monoglycidyl amine compounds being represented by Formulas VII or VIII, and
(C) a curing amount of a suitable curing agent therefor; wherein component (A) is present in an amount suitably from 1 to 99, more suitably from 50 to 90, most suitably from 70 to 90, percent by weight based upon the combined weight of components (A) and (B) and component (B) is present in an amount suitably from 99 to 1, more suitably from 50 to 10, most suitably from 30 to 10, percent by weight based upon the combined weight of components (A) and (B).

Another aspect of the present invention pertains to curable compositions comprising
(A) one or more of the advanced polyglycidyl amines resulting from reacting
   (1) at least one of the polyglycidyl amines containing one or more mesogenic moieties, said polyglycidyl amines being those represented by Formulas I-VI, with the proviso that the polyglycidyl amine of Formula II may include the tetraglycidyl amine of 4,4'-diaminobiphenyl or the tetraglycidyl amine of 3,3'-dimethyl-4,4'-diaminobiphenyl; with
   (2) at least one compound having an average of more than one active hydrogen atom per molecule; wherein components (A1) and (A2) are employed in quantities which provide a ratio of active hydrogen atoms to epoxide groups suitably from 0.01:1 to 0.95:1, more suitably from 0.05:1 to 0.8:1, most suitably from 0.1:1 to 0.5:1; and
(B) a curing amount of a suitable curing agent for component (A).

Another aspect of the present invention pertains to curable compositions comprising a blend of
(A) at least one of the polyglycidyl amines or monoglycidyl amine compounds containing one or more mesogenic moieties represented by Formulas I-VIII, with the proviso that the polyglycidyl amine of Formula II may include the tetraglycidyl amine of 4,4'-diaminobiphenyl or the tetraglycidyl amine of 3,3'-dimethyl-4,4'-diaminobiphenyl;
(B) at least one of the polyepoxide resins represented by Formulas IX-XV; and
(C) a curing amount of a suitable curing agent therefor; wherein component (A) is present in an amount suitably from 1 to 99, more suitably from 10 to 80, most suitably from 10 to 50, percent by weight based upon the combined weight of components (A) and (B) and component (B) is present in an amount suitably from 99 to 1, more suitably from 90 to 20, most suitably from 90 to 50, percent by weight based upon the combined weight of components (A) and (B).

Another aspect of the present invention pertains to curable compositions comprising a blend of
(A) at least one of the advanced polyglycidyl amines containing one or more mesogenic moieties prepared by reacting (1) one or more polyglycidyl amines represented by Formulas I-VI, with the proviso that the polyglycidyl amine of Formula II may include the tetraglycidyl amine of 4,4'-diaminobiphenyl or the tetraglycidyl amine of 3,3'-dimethyl-4,4'-diaminobiphenyl; with (2) at least one compound having an average of more than one active hydrogen atom per molecule; wherein components (1) and (2) are employed in quantities which provide a ratio of active hydrogen atoms per epoxide group suitably from 0.01:1 to 0.95:1, more suitably from 0.05:1 to 0.8:1, most suitably from 0.1:1 to 0.5:1;
(B) at least one of the polyepoxide resins represented by Formulas IX-XV; and
(C) a curing amount of a suitable curing agent therefor; wherein component (A) is present in an amount suitably from 1 to 99, more suitably from 10 to 80, most suitably from 10 to 50, percent by weight based upon the combined weight of components (A) and (B) and component (B) is present in an amount suitably from 99 to 1, more suitably from 90 to 20, most suitably from 90 to 50, percent by weight based upon the combined weight of components (A) and (B).

A further aspect of the present invention pertains to products resulting from curing the aforementioned curable compositions.

A further aspect of the present invention pertains to products resulting from the application of an electric field or magnetic field or drawing and/or shear forces before and/or during curing or processing of the aforementioned compositions.

A still further aspect of the present invention pertains to products resulting from the application of an electric field or magnetic field or drawing and/or shear forces before and/or during curing or processing of a curable composition comprising (A) at least one polyglycidyl amine containing one or more mesogenic moieties said polyglycidyl amines being those represented by Formulas I-VI, with the proviso that the polyglycidyl amine of Formula II may include the tetraglycidyl amine of 4,4'-diaminobiphenyl or the tetraglycidyl amine of 3,3'-dimethyl-4,4'-diaminobiphenyl; and (B) a curing amount of at least one suitable curing agent for component (A).

The term "mesogenic" as is used herein designates compounds containing one or more rigid rod-like structural units which have been found to favor the formation of liquid crystal phases in the case of low molar mass substances. Thus the mesogen or mesogenic moiety is that structure responsible for molecular ordering.

The mono and polyglycidyl amine compositions of the present invention can be prepared by any suitable method known to those skilled in the art. One such suitable method includes reaction of a mono or diamine and an epihalohydrin to form the corresponding halohydrin amine of said mono or diamine followed by dehydrohalogenation of the resultant halohydrin amine. In the preparation of the mono or polyglycidyl amine of a mono or polyamine, the amine containing compound is typically reacted with an epihalohydrin in the presence or absence of a suitable catalyst and in the presence or absence of a suitable solvent at a temperature suitably from 0°C to 150°C, more suitably from 20°C to 100°C, most suitably from 40°C to 80°C; at pressures suitably from 30 mm Hg vacuum to 100 psia., more suitably from 65 mm Hg vacuum to 50 psia., most suitably from atmospheric pressure to 20 psia.; and for a time sufficient to complete the reaction, usually from 1 to 48, more usually from 1 to 12, most usually from 1 to 6 hours. This initial reaction, unless the catalyst is an alkali metal or alkaline earth metal hydroxide employed in stoichiometric quantities, produces a halohydrin amine intermediate which is then reacted with a basic acting compound to convert the vicinal halohydrin groups to epoxide groups. When the catalyst is an alkali metal or alkaline earth metal hydroxide employed in stoichiometric quantities, then a subsequent dehydrohalogenation step is not required. Reaction of the halohydrin amine intermediate and basic acting compounds in the presence or absence of a suitable solvent is typically conducted at a temperature suitably from 0°C to 100°C, more suitably from 20°C to 80°C, most suitably from 25°C to 60°C; at pressures suitably from 30 mm Hg vacuum to 100 psia., more suitably from 45 mm Hg vacuum to 50 psia., most suitably from 60 mm Hg vacuum to atmospheric pressure; and for a time sufficient to complete the dehydrohalogenation reaction, usually from 10 minutes to 12 hours, more usually from 15 minutes to 6 hours, most usually from 20 minutes to 1 hour. The resultant product is a glycidyl amine compound.

Suitable epihalohydrins which can be employed to prepare the mono and polyglycidyl amines of the present invention include, for example, those represented by the following Formula XVI 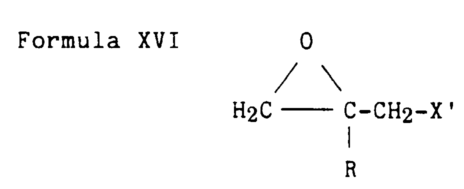 wherein R is as previously defined and X' is a halogen. Particularly suitable such epihalohydrins include, for example, epichlorohydrin, epibromohydrin, epiiodohydrin, methylepichlorohydrin, methylepibromohydrin, methylepiiodohydrin, or any combination thereof.

Suitable amine containing compounds which can be employed to prepare the mono and polyglycidyl amines of the present invention include, for example, those represented by the following Formulas XVII-XXIV

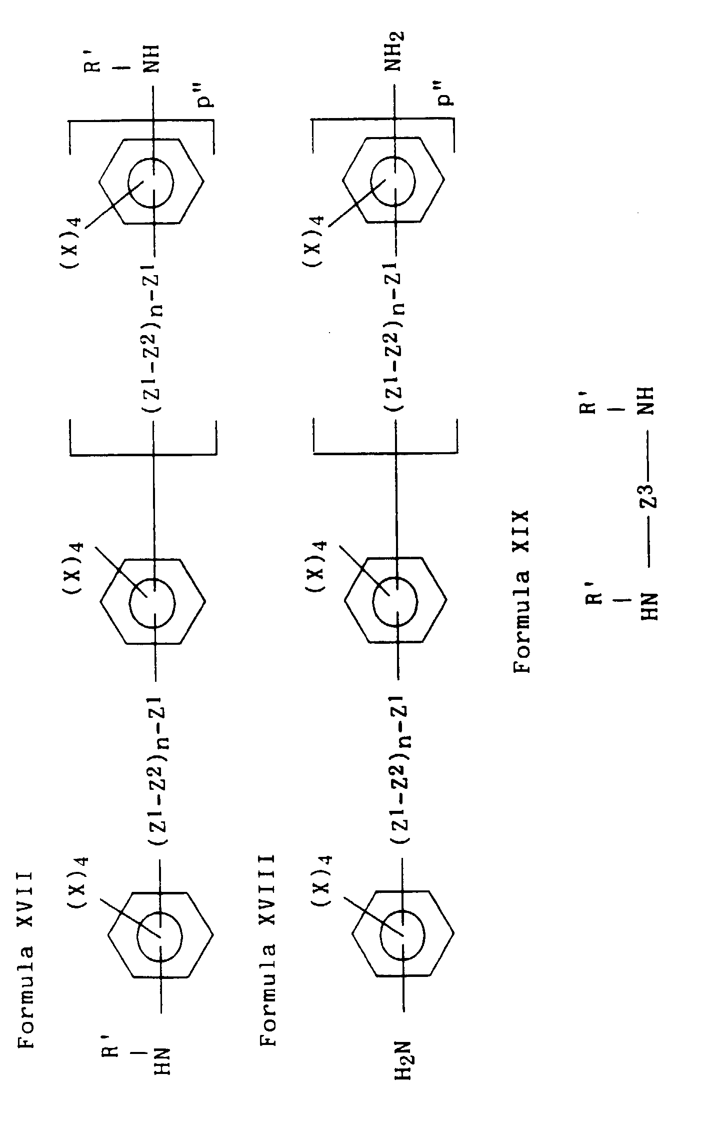

Formula XX H₂N ―Z³― NH₂

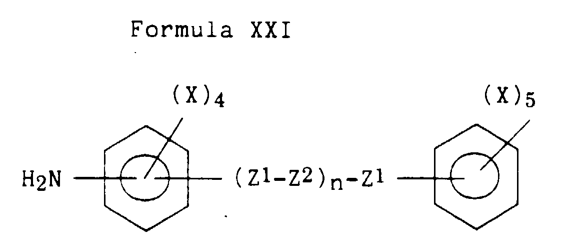

Formula XXII H₂―N―Z⁶

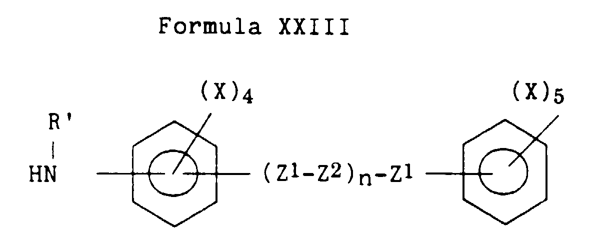

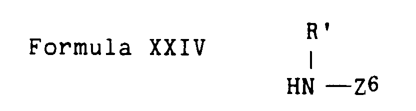

wherein at least 80 percent of the -(Z¹-Z²)ₙ-Z¹- linkages and the amine groups are in the para position with respect to each other; wherein R', R¹, X, X¹, Z¹, Z², Z³, Z⁴, Z⁶, n, n', and p" are as previously defined.

Particularly suitable amine containing compounds include, for example, 4,4'-diamino-α-methylstilbene, 4,4'-diaminobenzanilide, 4,4'-diamino-2,2'-dimethylazoxybenzene, 4,4'-diaminostilbene, 4,4'-diaminoazobenzene, 4,4'-diaminoazoxybenzene, 4,4'-diamino-α-cyanostilbene, 4,4'-diaminodiphenylacetylene, N,N'-bis(4-aminophenyl)terephthalamide, 4,4'-diamino-3,3',5,5'-tetramethylstilbene, 4,4'-diamino-3,3',5,5'-tetrabromostilbene, 4,4'-diamino-3,3',5,5'-tetramethyl-α-methylstilbene, N-biphenyl-4-aminobenzamide, N-2-naphthyl-4-aminobenzamide, N-phenyl-4-aminobenzamide, N-(4'-aminophenyl)benzamide, 4-aminostilbene, 4-amino-α-methylstilbene, 4-aminoazobenzene, 4-amino-α-cyanostilbene, 4-aminoazoxybenzene, 4,4'-diaminodiphenylazomethine, 4-amino-4'-methoxystilbene, N,N'-dimethyl-4,4'-diaminobiphenyl, N,N'-dimethyl-3,3'-dimethyl-4,4'-diaminobiphenyl, N,N'-dimethyl-4,4'-diamino-a-methylstilbene, N,N'-diethyl-4,4'-diamino-α-methylstilbene, N,N'-dimethyl-4,4'-diaminobenzanilide, N,N'-dimethyl-4,4'-diaminostilbene, N,N'-dimethyl-4,4'-diaminoazoxybenzene, N,N'-dimethyl-4,4'-diamino-α-cyanostilbene, N,N'-dimethyl-4,4'-diamino-α-chlorostilbene, N,N'-dimethyl-4,4'-diaminodiphenylacetylene, N,N'-dimethyl-4,4'-diamino-3,3',5,5'-tetramethylstilbene, N,N'-diethyl-4,4'-diamino-α,α'-dimethylstilbene, N-methyl-4-aminostilbene. N-ethyl-4-aminostilbene, N-methyl-4-amino-α-methylstilbene, N-methyl-4-aminoazobenzene, N-methyl-4-amino-α-cyanostilbene. N-methyl-4-aminoazoxybenzene, N-methyl-4-aminobenzamide, N-methyl-4-amino-4'-methoxystilbene, N,N'-dimethyl-4-aminobenzamide, N-methyl-4-amino-α-chlorostilbene, N,N'-dimethyl-4,4'-diaminodiphenylazomethine, or any combination thereof.

Suitable catalysts which can be employed to prepare the mono and polyglycidyl amines of the present invention include, for example, ammonium halides such as, for example, benzyltrimethylammonium chloride, benzyltrimethylammonium bromide, tetrabutylammonium chloride, tetrabutylammonium bromide, tetraoctylammonium chloride, tetraoctylammonium bromide, tetramethylammonium chloride, tetramethylammonium bromide, or any combination thereof.

Suitable basic acting compounds which can be employed to prepare the mono and polyglycidyl amines of the present invention include, for example, alkali metal or alkaline earth metal hydroxides, carbonates, bicarbonates or any combination thereof. Particularly suitable such compounds include, for example, sodium hydroxide, potassium hydroxide, lithium hydroxide, calcium hydroxide, barium hydroxide, magnesium hydroxide, manganese hydroxide, sodium carbonate, potassium carbonate, lithium carbonate, calcium carbonate, barium carbonate, magnesium carbonate, manganese carbonate, sodium bicarbonate, potassium bicarbonate, lithium bicarbonate, calcium bicarbonate, barium bicarbonate, magnesium bicarbonate, manganese bicarbonate, or any combination thereof. Most preferred is sodium hydroxide or potassium hydroxide.

Suitable solvents which can be employed herein include, for example, alcohols, glycols, aliphatic hydrocarbons, aromatic hydrocarbons, glycol ethers, amides, sulfoxides, sulfones, or any combination thereof. Particularly suitable solvents include, for example, methanol, ethanol, isopropanol, hexane, heptane, octane, nonane, decane, toluene. xylene, ethylene glycol, propylene glycol, ethylene glycol methyl ether, ethylene glycol ethyl ether, ethylene glycol n-butyl ether, ethylene glycol phenyl ether, propylene glycol methyl ether, propylene glycol phenyl ether, tripropylene glycol methyl ether, diethylene glycol methyl ether, diethylene glycol ethyl ether, diethylene glycol n-butyl ether, diethylene glycol phenyl ether, butylene glycol methyl ether, N,N-dimethylformamide, N-methylpyrrolidinone, N,N-dimethylacetamide, dimethylsulfoxide, sulfolane, or any combination thereof.

The solvent, if used, is usually employed in amounts suitably from 5 to 95, more suitably from 20 to 60, most suitably from 30 to 40, percent by weight based upon the combined weight of solvent and epihalohydrin.

Suitable compounds having an average of more than one active hydrogen atom per molecule which can be employed to prepare the advanced resin compositions of the present invention include, for example, bisphenols, thiobisphenols, dicarboxylic acids and compounds containing one primary amine or amide group or two secondary amine groups such as those represented by the following Formulas XXV or XXVI; 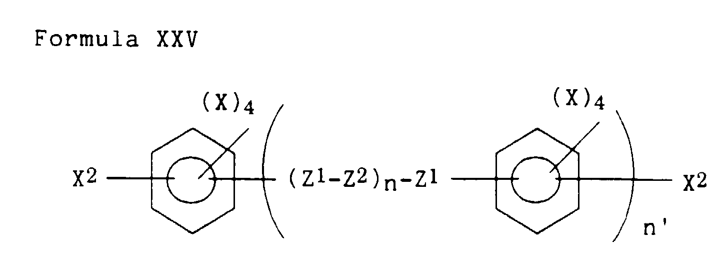 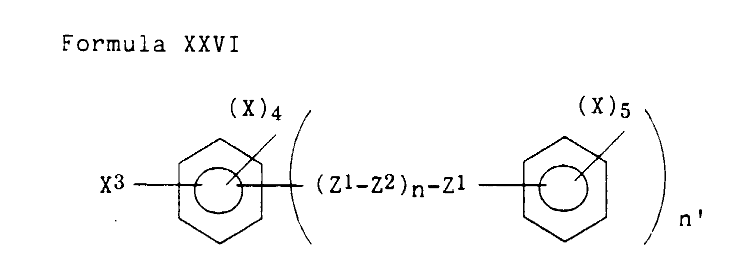 wherein X² is independently a hydroxyl, carboxylic acid, -SH, or -NHR' group; X³ is -NH₂, NH₂-SO₂-, NH₂-CO-, or NH₂-Z⁵-O-; Z⁵ is an alkyl or cycloalkyl group having from 1 to 12 carbon atoms; and wherein R', Z¹, X, Z', R¹, Z², n and n' are as hereinbefore defined.

The advancement of the polyglycidyl amines containing one or more mesogenic moieties with compounds having an average of more than one active hydrogen per molecule is employed to linearly chain extend the resin. This linear chain extension is required for some mesogen-containing resin compositions in order to obtain liquid crystal character. The advancement of the mesogenic polyglycidyl amine resins can also be used to increase the temperature range in which a particular resin is liquid crystalline and to control the degree of crosslinking during the final curing stage.

The polyglycidyl amine containing one or more mesogenic moieties and the compound having an average of more than one active hydrogen atom per molecule are reacted in amounts which provide suitably from 0.01:1 to 0.95:1, more suitably from 0.05:1 to 0.9:1, most suitably from 0.10:1 to 0.50:1 active hydrogen atoms per epoxy group.

Particularly suitable compounds having an average of more than one active hydrogen atom per molecule which can be employed herein include hydroxyl-containing compounds, carboxylic acid-containing compounds and primary amine-containing compounds. These compounds include, for example, those represented by Formulas XXV and XXVI.

Particularly suitable hydroxyl-containing compounds include, for example, hydroquinone, bisphenol A, 4,4'-dihydroxydiphenylmethane, 4,4'-thiodiphenol, 4,4'-sulfonyldiphenol, 4,4'-dihydroxydiphenyl oxide, 4,4'-dihydroxybenzophenone, 1,1-bis(4-hydroxyphenyl)-1-phenylethane, 3,3',5,5'-tetrachorobisphenol A, 3,3'-dimethoxybisphenol A, 4,4'-dihydroxybiphenyl, 4,4'-dihydroxy-α,α'-diethylstilbene, 4,4'-dihydroxy-α-methylstilbene, 4,4'-dihydroxybenzanilide, 4,4'-dihydroxy-2,2'-dimethylazoxybenzene, 4,4'-dihydroxy-α-cyanostilbene, bis(4-hydroxyphenyl)terephthalate, N,N'-bis(4-hydroxyphenyl)terephthalamide, bis(4'-hydroxybiphenyl)terephthalate, 4,4'-dihydroxyphenylbenzoate, bis(4'-hydroxyphenyl)-1,4-benzenediimine, 4,4"-dihydroxybiphenylbenzoate, 1,4-bis(4'-hydroxyphenyl-1'-carboxamide)benzene, 1,4-bis(4'-hydroxyphenyl-1'-carboxy)benzene, 4,4'-bis(4"-hydroxyphenyl-1"-carboxy)biphenyl, or any combination thereof.

Particularly suitable carboxylic acid-containing compounds include, for example, terephthalic acid, 4,4'-benzanilide dicarboxylic acid. 4,4'-phenylbenzoate dicarboxylic acid, 4,4'-stilbenedicarboxylic acid, 4,4'-dicarboxybiphenyl 4,4'-dicarboxydiphenylazomethine, or any combination thereof.

Particularly suitable primary amine-containing compounds include, for example, aniline, 4'-sulfonamido-N-phenyl benzamide, 4'-sulfonamido-N-phenyl-4-chlorobenzamide, 4-amino-1-phenylbenzoate, 4-amino-N-phenylbenzamide, N-phenyl-4-aminophenyl-1-carboxamide, phenyl-4-aminobenzoate, biphenyl-4-aminobenzoate, 1-phenyl-4'-aminophenylterephthalate, or any combination thereof.

Particularly suitable bis(secondary amine)containing compounds include, for example, N,N'-dimethyl-4,4'-diaminodiphenylmethane, N,N'-diethyl-4,4'-diaminodiphenylmethane, N,N'-dimethyl-4,4'-diaminobiphenyl, N,N'-dimethyl-3,3'-dimethyl-4,4'-diaminobiphenyl, N,N'-dimethyl-4,4'-diaminostilbene, N,N'-dimethyl-4,4'-diaminodiphenylsulfone, N,N'-dimethyl-4,4'-diaminobenzanilide, N,N'-dimethyl-4,4'-diamino-N-methylbenzanilide,or any combination thereof.

The advancement reaction can be conducted in the presence of a suitable advancement catalyst such as, for example, phosphines, quaternary ammonium compounds, phosphonium compounds, tertiary amines, or any combination thereof. Particularly suitable catalysts include, for example, ethyltriphenylphosphonium chloride, ethyltriphenylphosphonium bromide, ethyltriphenyl-phosphonium iodide, ethyltriphenylphosphonium diacetate (ethyltriphenylphosphonium acetate·acetic acid complex), ethyltriphenylphosphonium phosphate, tetrabutylphosphonium chloride, tetrabutylphosphonium bromide, tetrabutylphosphonium iodide. tetrabutylphosphonium diacetate (tetrabutylphosphonium acetate·acetic acid complex), butyltriphenylphosphonium tetrabromobisphenate, butyltriphenylphosphonium bisphenate, butyltriphenylphosphonium bicarbonate, benzyltrimethylammonium chloride, tetramethylammonium hydroxide, triethylamine, tripropylamine, tributylamine, 2-methylimidazole, benzyldimethylamine, or any combination thereof. Many of these catalysts are described in U. S. Patent Nos. 3,306.872; 3,341,580; 3,379,684; 3,477,990; 3,547,881; 3,637,590; 3.843,605; 3,948,855; 3,956,237; 4,048,141; 4,093,650; 4,131,633; 4,132,706; 4,171,420; 4,177,216 and 4,366,295.

The amount of advancement catalyst depends, of course, upon the particular reactants and catalyst employed; however, it is usually employed in quantities of from 0.03 to 3, preferably from 0.03 to 1.5, most preferably from 0.05 to 1.5 percent by weight based upon the weight of the epoxy-containing compound.

The advancement reaction can be conducted at atmospheric, superatmospheric or subatmospheric pressures at temperatures of from 20°C to 260°C. preferably from 80°C to 240°C, more preferably from 100°C to 200°C. The time required to complete the advancement reaction depends upon the temperature employed. Higher temperatures require shorter periods of time whereas lower temperatures require longer periods of time. Generally, however, times of from 5 minutes to 24 hours, preferably from 30 minutes to 8 hours, more preferably from 30 minutes to 3 hours are suitable.

If desired, the advancement reaction can be conducted in the presence of one or more solvents. Suitable such solvents include, for example, glycol ethers, aliphatic and aromatic hydrocarbons, aliphatic ethers, cyclic ethers, ketones, amines, amides. or any combination thereof. Particularly suitable solvents include, for example, toluene, benzene, xylene, methyl ethyl ketone, methyl isobutyl ketone, diethylene glycol methyl ether, dipropylene glycol methyl ether, dimethylformamide, dimethyl-sulfoxide, N-methylpyrrolidinone, tetrahydrofuran, propylene glycol methyl ether, or any combination thereof. The solvents can be employed in amounts of from zero to 80 percent, preferably from 20 percent to 60 percent, more preferably from 30 percent to 50 percent by weight based upon the weight of the reaction mixture.

When the polyglycidyl amine containing one or more mesogenic moieties and the compound having an average of more than one active hydrogen atom per molecule are reacted in amounts which provide from 0.96:1 to 1.05:1 active hydrogen atoms per epoxy group, a relatively high molecular weight substantially thermoplastic resinous product is produced. These thermoplastic resin compositions contain little, if any, curable residual epoxide functionality and may even contain an active hydrogen functionality, depending upon which component is employed in excess, the polyglycidyl amine or the active hydrogen containing compound. These phenoxy type resins may thus be processed using the typical processing methods employed with conventional thermoplastic resins, such as, for example, injection molding or extrusion. Thermosetting may, however, be induced, for example, via reaction of all or a part of the backbone secondary aliphatic hydroxyl groups produced in the aforesaid advancement reaction, with a curing agent therefor. One class of suitable curing agents includes, for example, the di or polyisocyanates, as well as the blocked di or polyisocyanates which can be induced to react with the secondary hydroxyl groups providing urethane crosslinks between the resin chains. An example of a specific diisocyanate especially useful herein is 4,4'-diisocyanatodiphenylmethane. If desired. the reaction can be conducted in the presence of a suitable catalyst such as, for example, those catalysts described herein for use in the advancement reaction.

The compositions of the present invention containing an average of more than one vicinal epoxy group per molecule can be cured with any suitable curing agent for curing epoxy-containing resins such as, for example, primary and secondary polyamines, carboxylic acids and anhydrides thereof, aromatic hydroxyl containing compounds, imidazoles, guanidines, urea-aldehyde resins, melamine-aldehydes resins, alkoxylated urea-aldehyde resins, alkoxylated melamine-aldehyde resins, aliphatic amines, cycloaliphatic amines, aromatic amines, or any combination thereof. Said curing agents may contain one or more mesogenic moieties or may be substantially free of said mesogenic moieties. Particularly suitable curing agents include, for example, diaminodiphenylmethanes, dicyandiamide, ethylene diamine, diethylenetriamine, triethylenetetramine, tetraethylenepentamine, urea-formaldehyde resins, melamine-formaldehyde resins, methylolated urea-formaldehyde resins, methylolated melamine-formaldehyde resins, phenol-formaldehyde novolac resins, cresol-formaldehyde novolac resins, sulfanilamide, diaminodiphenylsulfones, diethyltoluenediamines, t-butyltoluenediamines. bis-4-aminocyclohexylmethane, isophoronediamine, diaminocyclohexanes, hexamethylenediamine, piperazine, aminoethylpiperazine, 2,5-dimethyl-2,5-hexanediamine, 1,12-dodecanediamine, tris-3-aminopropylamine, 4,4'-diaminostilbene, 4,4'-diaminobenzanilide, 3,3'-dimethyl-4,4'-diaminobiphenyl, or any combination thereof.

The curing agents are employed in amounts which will effectively cure the composition: however, these amounts will depend upon the particular polyglycidyl amine and curing agent employed. Generally, suitable amounts include, for example, from 0.95:1 to 1.2:1 equivalents of curing agent per equivalent of polyglycidyl amine.

The monoglycidyl amines containing one or more mesogenic moieties of the present invention can be employed as reactive diluents for the polyglycidyl amines of the present invention as well as for polyglycidyl amines substantially free of mesogenic moieties, or epoxy resins. For polyglycidyl amines free of mesogenic moieties, the monoglycidyl amines provide a means of incorporating mesogenic moieties into the composition so as to enhance one or more properties when cured.

The mesogenic polyglycidyl amines of the present invention can also be employed for the purpose of improving the properties of epoxy resins substantially free of mesogenic moieties. Generally, suitable amounts of mesogenic polyglycidyl amines are from 1 to 99, more suitably from 10 to 80, most suitably from 10 to 50 weight percent based on the total weight of the combined resins. Representative of the epoxy resins free of mesogenic moieties include, for example, the diglycidyl ethers of resorcinol, bisphenol A, 4,4'-dihydroxydiphenylmethane, 3,3',5,5'-tetrabromobisphenol A, 4,4'-thiodiphenol, 4,4'-sulfonyldiphenol, 4,4'-dihydroxydiphenyl oxide, 4,4'-dihydroxybenzophenone, 1,1-bis(4-hydroxyphenyl)-1-phenylethane, 3,3',5,5'-tetrachlorobisphenol A, 3,3'-dimethoxybisphenol A; the triglycidyl ether of tris(hydroxyphenyl)methane; the polyglycidyl ether of a phenol or substituted phenolaldehyde condensation product (novolac); the polyglycidyl ether of a dicyclopentadiene or an oligomer thereof and phenol condensation product; the advancement reaction products of the aforesaid di- and polyglycidyl ethers with aromatic di- or polyhydroxyl- or carboxylic acid- containing compounds including, for example, bisphenol A (4,4'-isopropylidenediphenol), o-, m-, p-dihydroxybenzene, 2,4-dimethylresorcinol, 4-chlororesorcinol, tetramethylhydroquinone, 1,1-bis(4-hydroxyphenyl)ethane, 1,1-bis(4-hydroxyphenyl)-1-phenylethane, 4,4'-dihydroxydiphenyl ether, 3,3',5,5'-tetramethyldihydroxydiphenyl ether, 3,3',5,5'-dichlorodihydroxydiphenyl ether, 4,4'-bis-(p-hydroxyphenyl isopropyl)diphenyl ether, 4,4'-bis-(p-hydroxyphenoxy)benzene, 4,4'-bis(p-hydroxyphenoxy)diphenyl ether, 4,4'-bis(4(4-hydroxyphenoxy)phenyl sulfone)diphenyl ether, 4,4'-dihydroxydiphenyl sulfone, 4,4'-dihydroxydiphenyl sulfide, 4,4'-dihydroxydiphenyl disulfide, 2,2'-dihydroxydiphenyl sulfone, 4,4'-dihydroxydiphenyl methane, 1,1-bis(p-hydroxyphenyl)cyclohexane, 4,4'-dihydroxybenzophenone, phloroglucinol, pyrogallol, 2,2',5,5'-tetrahydroxydiphenyl sulfone, tris(hydroxyphenyl)methane, dicyclopentadiene diphenol, tricyclopentadiene diphenol; or any combination thereof.

Before and/or during processing and/or curing of the polyglycidyl amine compositions into a part, electric or magnetic fields or shear stresses can be applied for the purpose of orienting the liquid crystal moieties contained or developed therein which in effect improves the mechanical properties. As specific examples of these methods, Finkelmann, et al, Macromol. Chem., 180, 803-806 (March 1979) induced orientation in thermotropic methacrylate copolymers containing mesogenic side chain groups decoupled from the main chain via flexible spacers in an electric field. Orientation of mesogenic side chain groups decoupled from the polymer main chain via flexible spacers in a magnetic field has been demonstrated by Roth and Kruecke, Macromol. Chem., 187, 2655-2662 (November 1986). Magnetic field induced orientation of mesogenic main chain containing polymers has been demonstrated by Moore, et al, ACS Polymeric Material Sciences and Engineering, 52, 84-86 (April-May 1985). Magnetic and electric field orientation of low molecular weight mesogenic compounds is discussed by W. R. Krigbaum in Polymer Liquid Crystals, pages 275-309 (1982) published by Academic Press, Inc.

In addition to orientation by electric or magnetic fields, polymeric mesophases can be oriented by shear forces which are induced by drawing and/or flow through dies, orifices, and mold gates. A general discussion for orientation of thermotropic liquid crystal polymers by this method is given by S. K. Garg and S. Kenig in High Modulus Polymers, pages 71-103 (1988) published by Marcel Dekker, Inc. For the mesomorphic systems based on the polyglycidyl amine compositions, this shear orientation can be produced by processing methods such as injection molding, extrusion. pultrusion, filament winding, filming and prepreging.

The mesogenic polyglycidyl amines of the present invention can be blended with other materials such as solvents or diluents, fillers, pigments, dyes, flow modifiers, thickeners, reinforcing agents, mold release agents, wetting agents, stabilizers, fire retardant agents, surfactants, or any combination thereof.

These additives are added in functionally equivalent amounts, e.g., the pigments and/or dyes are added in quantities which will provide the composition with the desired color; however, they are suitably employed in amounts of from zero to 20, more suitably from 0.5 to 5, most suitably from 0.5 to 3 percent by weight based upon the weight of the total blended composition.

Solvents or diluents which can be employed herein include, for example, hydrocarbons, ketones, glycol ethers, aliphatic ethers, cyclic ethers, amines, amides, or any combination thereof. Particularly suitable solvents or diluents include, for example, toluene, benzene, xylene. methyl ethyl ketone, methyl isobutyl ketone, diethylene glycol methyl ether, dipropylene glycol methyl ether, dimethylformamide, N-methylpyrrolidinone, tetrahydrofuran, propylene glycol methyl ether, or any combination thereof.

The modifiers such as thickeners, flow modifiers or other suitable such additives can be suitably employed in amounts of from zero to 10, more suitable from 0.5 to 6, most suitably from 0.5 to 4 percent by weight based upon the weight of the total composition.

Reinforcing materials which can be employed herein include natural and synthetic fibers in the form of woven fabric, mats, monofilament, multifilament, unidirectional fibers, rovings, random fibers or filaments, inorganic fillers or whiskers, hollow spheres, or other suitable such materials. Suitable reinforcing materials include, glass, ceramics, nylon, rayon, cotton, aramid. graphite, polyalkylene terephthalates, polyethylene, polypropylene, polyamines, or any combination thereof.

Suitable fillers which can be employed herein include, for example, inorganic oxides, ceramic microspheres, plastic microspheres, glass microspheres, inorganic whiskers, CaCO₃, or any combination thereof.

The fillers can be employed in amounts suitable from zero to 95, more suitably from 10 to 80, most suitable from 40 to 60 percent by weight based upon the weight of the total composition.

The following examples are illustrative of the present invention, but are not to be construed as to limiting its scope in any manner.

### EXAMPLE 1

### A. Synthesis of -Dinitrostilbene

Nitrobenzyl chloride (74.5 grams, 0.434 mole), ethanol (587 milliliters) and acetone (25.22 grams, 0.434 mole) are added to a reactor and stirred under a nitrogen atmosphere with cooling to provide a 22°C mixture. Dropwise addition of a solution of sodium hydroxide (19.11 grams, 0.478 mole) in ethanol (352 milliliters) commences and was completed over the next 25 minutes inducing a reaction temperature increase to 34°C. After an additional three minutes, heating commences and a reaction temperature of 77°C at reflux was achieved 12 minutes later. After an additional 6 hour of refluxing, the reactor contents are added to deionized water (3.5 liters) followed by acidification of the product slurry with hydrochloric acid to a pH of 2 with mixing. The resultant precipitated product was recovered by filtration, washed with deionized water (250 milliliters), then added to dimethylsulfoxide (450 milliliters) and heated to 140°C to provide a solution. Recrystallization was accomplished by gradual cooling and holding of the dimethylsulfoxide solution at 5°C overnight followed by filtration to recover the crystalline precipitate. A second recrystallization was completed by adding the wet filter cake to acetone (150 milliliters) followed by heating to a boil. Recrystallization was accomplished by gradual cooling and holding of the acetone solution at 5°C overnight followed by filtration to recover the crystalline precipitate. The recovered filter cake was dried in a vacuum oven at 110°C and 5 mm Hg to a constant weight of 39.2 grams. The product was recovered as brilliant yellow colored needlelike crystals. Additional solids precipitated from the dimethylsulfoxide mother liquor from the initial recrystallization, but no attempt was made to recover and process this material. Fourier transform infrared spectrophotometric analysis of a potassium chloride pellet of the product confirmed the product structure: 1503 and 1337 cm⁻¹ conjugated nitro group absorbance, with the 1503 cm⁻¹ conjugated nitro group absorbance masking the aromatic ring absorbance. Proton nuclear magnetic resonance spectroscopy further confirmed the product structure.

### B. Synthesis of 4,4'-Diaminostilbene

A portion (20.27 grams, 0.075 mole) of 4,4'-dinitrostilbene from A above, concentrated hydrochloric acid (150 milliliters) and ethanol (300 milliliters) are added to a beaker and heated to provide a 60°C stirred solution. Over the next eight hour period, 325 mesh powdered iron was added to the reaction mixture in aliquots until a total of 33.51 grams (0.60 mole) had been added. After completion of the iron addition, the mixture was heated for 16 hours at 60°C then diluted with ethanol (250 milliliters) and deionized water (250 milliliters). After cooling to 25°C, the product was filtered. The resultant solution was treated with 50% aqueous sodium hydroxide sufficient to induce precipitation of a pale tan colored product which was recovered by filtration. The recovered filter cake was washed with deionized water (500 milliliters) then dried in a vacuum oven at 100°C and 2 mm Hg to a constant weight of 11.3 grams. The product was recovered as a pale tan colored solid. Fourier transform infrared spectrophotometric analysis of a potassium chloride pellet of the product confirmed the product structure: Disappearance of the absorbances observed for the conjugated nitro group, appearance of -NH₂ stretching absorbances at 3436 (3462 shoulder) and 3356 (3376 shoulder) cm⁻¹, a -NH₂ bending absorbance at 1616 cm⁻¹ (1603 cm⁻¹ shoulder due to aromatic ring absorbance), an out-of-plane C-H deformation at 970 cm⁻¹ due to the trans substituted ethylene group and a C-H out-of-plane bending vibration at 832 cm⁻¹ due to the para-disubstituted aromatic rings. Proton nuclear magnetic resonance spectroscopy further confirmed the product structure.

### C. Synthesis of 4,4'-Diacetamidostilbene

4,4'-Diaminostilbene (8.9 grams, 0.0847 -NH₂ equivalent) from B above, was ground to a fine powder and added to a beaker containing stirred, 90°C deionized water (1500 milliliters). After two minutes, aqueous 36.5% hydrochloric acid (8.47 grams, 0.0847 mole) was added to the stirred, 90°C suspension. The solution which formed within five minutes was cooled to 60°C. Acetic anhydride (10.61 grams, 0.1039 mole) was added to the stirred solution and mixed therein for 30 seconds before addition of an aqueous sodium acetate solution (prepared by dissolution of anhydrous sodium acetate [13.89 grams, 0.1693 mole] in deionized water [41.7 grams]). After thoroughly stirring the white slurry, it was maintained at 4°C for 16 hours, followed by filtration to remove the precipitated product. The recovered filter cake was washed with two portions (250 milliliters) of deionized water then dried in a vacuum oven at 80°C and 2 mm Hg to a constant weight of 11.44 grams. The product was recovered as a tan colored crystalline powder. Fourier transform infrared spectrophotometric analysis of a potassium chloride pellet of the product confirmed the product structure: disappearance of the absorbances observed for the -NH₂ group, appearance of solid state >NH stretching absorbances at 3309 (3396 and 3190 shoulders) cm⁻¹, a solid state amide I carbonyl stretching absorbance at 1669 cm⁻¹, a solid state amide II carbonyl stretching absorbance at 1516 cm⁻¹ (masking the aromatic ring absorbance), an out-of-plane C-H deformation at 959 cm⁻¹ due to the trans substituted ethylene group and a C-H out-of-plane bending vibration at 832 cm⁻¹ due to the para-disubstituted aromatic rings. Proton nuclear magnetic resonance spectroscopy further confirmed the product structure.

### D. Synthesis of N,N'-dimethyl-4,4'-diacetamidostilbene

4,4'-Diacetamidostilbene (10.35 grams, 0.0694 -NH-CO- equivalent) from C above, tetra-n-butylammonium hydrogen sulfate (2.36 grams, 0.0069 mole), powdered 99% sodium hydroxide (9.71 grams, 0.2429 mole), anhydrous powdered potassium carbonate (19.42 grams, 0.1405 mole) and benzene (200 milliliters) are added to a reactor and stirred under a nitrogen atmosphere to provide a 21°C mixture. After two minutes, dimethyl sulfate (13.13 grams, 0.1041 mole) was added to the stirred mixture then heating was initiated. After nine minutes, the mixture reached a reflux temperature of 81°C. After 363 minutes at the 81°C reflux, Fourier transform infrared spectrophotometric analysis of a sample of the organic layer of the reaction mixture revealed that full conversion to the N,N'-dimethyl derivative had occurred. Sixteen minutes later, additional benzene (200 milliliters) and deionized water (100 grams) are added to the reactor and thoroughly mixed with the reaction mixture. The reactor contents are added to a separatory funnel and the aqueous layer which separated was removed and discarded. The benzene solution was washed with two portions of deionized water (100 milliliters), dried over anhydrous sodium sulfate, then rotary evaporated to remove solvent. The recovered powder was then dried in a vacuum oven at 80°C and 2 mm Hg to a constant weight of 7.55 grams. The product was recovered as a white crystalline powder. Fourier transform infrared spectrophotometric analysis of a film of the product on a potassium chloride plate confirmed the product structure: disappearance of the absorbances observed for the secondary amide group, appearance of a tertiary amide carbonyl stretching absorbance at 1656 cm⁻¹, a C-H stretching absorbance at 2878 cm⁻¹ due to the >N-CH₃ groups, an out-of-plane C-H deformation at 972 cm⁻¹ due to the trans substituted ethylene group and a C-H out-of-plane bending vibration at 846 (839 shoulder) cm⁻¹ due to the para-disubstituted aromatic rings. Proton nuclear magnetic resonance spectroscopy further confirmed the product structure.

### E. Hydrolysis of N,N'-dimethyl-4,4'-diacetamidostilbene

N,N'-dimethyl-4,4'-diacetamidostilbene (7.50 grams, 0.0460 -N-(CH3)-CO- equivalent) from D above was added to a reactor and dissolved with stirring under a nitrogen atmosphere in ethanol (170 milliliters). Concentrated hydrochloric acid (9.07 grams, 0.0920 mole) was added to the resultant 25°C solution inducing a maximum exotherm of 34°C one minute later. The stirred reaction mixture was allowed to cool to 25°C over the next forty eight minutes then heating to 50°C commences. After eighty nine minutes at 50°C, additional concentrated hydrochloric acid (9.07 grams) was added to the solution. After an additional 149 minutes at 50°C, additional concentrated hydrochloric acid (9.07 grams) was added to the solution and heating to 75°C commences. After seventy six minutes at 75°C, additional concentrated hydrochloric acid (18.14 grams) was added to the solution. After 22.5 hours at the 75°C temperature, Fourier transform infrared spectrophotometric analysis of a sample of the solution as a neat film on a potassium chloride plate revealed that complete hydrolysis of the amide linkages had occurred concurrent with the formation of the amine hydrochloride salt of the diamine thus produced: disappearance of the tertiary amide carbonyl stretching absorbance, appearance of -NH₃⁺ stretching absorbances at 2665 and 2446 cm⁻¹, a NH₃⁺ bending absorbance at 1603 cm⁻¹ (masking the aromatic ring absorbance). At this time, deionized water (500 grams) was added to the reactor and thoroughly mixed with the reaction mixture. The reactor contents are cooled to 25°C then 50% aqueous sodium hydroxide was added with mixing to a pH of 9 followed by filtration of the precipitated product. The recovered filter cake was washed with two portions (100 milliliters) of deionized water then dried in a vacuum oven at 80°C and 2 mm Hg to a constant weight of 5.02 grams. The product was recovered as a light yellow colored crystalline powder. Fourier transform infrared spectrophotometric analysis of a potassium bromide pellet of the product confirmed the product structure: disappearance of the tertiary amide carbonyl stretching absorbance, appearance of a >NH stretching absorbance at 3416 (3389 shoulder) cm⁻¹, a C-H stretching absorbance at 2818 cm⁻¹ due to the >N-CH₃ groups, an out-of-plane C-H deformation at 965 cm⁻¹ due to the trans substituted ethylene group and a C-H out-of-plane bending vibration at 826 cm⁻¹ due to the para-disubstituted aromatic rings. Proton nuclear magnetic resonance spectroscopy further confirmed the product structure.

### F. Epoxidation of N,N'-dimethyl-4,4'-diaminostilbene

N,N'-dimethyl-4,4'-diaminostilbene (2.383 grams, 0.02 >NH equivalent) from E above. epichlorohydrin (7.402 grams, 0.08 mole) and ethylene glycol (0.991 gram, 0.0320 hydroxyl equivalent) are added to a reactor and heated to 50°C with stirring under a nitrogen atmosphere. After 3 hours at the 50°C temperature, cooling to 35°C commenced. After seven minutes the 35°C temperature was achieved and sodium hydroxide (0.952 gram, 0.0238 mole) dissolved in deionized water (1.04 grams, 52% of total solution) was added to the reactor. A maximum exotherm of 42°C occurs within one minute with the reaction temperature reduced back to 35°C within an additional minute via cooling of the reactor exterior. After an additional eighteen minutes at 35°C, the reaction mixture was recovered and rotary evaporated at 55°C and 2 mm Hg final vacuum to remove residual epichlorohydrin. The solids remaining after rotary evaporation are thoroughly mixed with methylethylketone (100 milliliters) then extracted with deionized water (150 milliliters). The mixture was added to a separatory funnel and the aqueous layer which separated was removed and discarded. The methylethylketone solution was washed with two portions (50 milliliters) of deionized water, then rotary evaporated at 55°C and 1 mm Hg final vacuum for 90 minutes. The product was recovered as a light yellow colored crystalline powder with an epoxide equivalent weight (EEW) of 192.19 (corrected for titrated contribution of amine nitrogen).

### EXAMPLE 2

### Characterization of the Diglycidyl Amine of N,N'-dimethyl-4,4'-diaminostilbene by Optical Microscopy and Differential Scanning Calorimetry

A portion (12.50 milligrams) of the diglycidyl amine of N,N'-dimethyl-4,4'-diaminostilbene from Example 1-F was analyzed by differential scanning calorimetry using a heating rate of 10°C per minute and a temperature range of 0 to 130°C under a stream of nitrogen flowing at 35 cubic centimeters per minute. The results are given in Table I.

**Table I**

| Cycle Designation | Observed Transition Temperatures (°C) midpoint/ range | Enthalpy (j/g) | Comments |
|---|---|---|---|
| First heating (0 to 130°C) | 33/33-46 | 0.78 | single endotherm |
| | 75 and 103/46-112 | 43.8 | endotherm with shoulder at 75°C |
| First cooling (130 to 0°C | 55.5/43-67 | 33.8 | single exotherm |

Analysis of the diglycidyl amine of N,N'-dimethyl-4,4'-diaminostilbene via crosspolarized light microscopy was completed using a microscope equipped with a programmable hot stage using a heating rate of 10°C per minute. The results are given in Table II.

**Table II**

| Cycle Designation | Observed Transition Temperatures (°C) | Comments |
|---|---|---|
| First heating (30 to 135 °C) | 30 | Birefringent crystalline solid. |
| | 102 | Melting observed. |
| | 103 | Crystalline dispersion. |
| | 119 | Isotropization. |
| First cooling (135 to 30°C) | 135 | Isotropic fluid. |
| | 74 | Crystallization begins. |
| | 64 | Birefringent crystalline solid. |

### EXAMPLE 3 Copolymerization of Diglycidyl Amine of N,N'-dimethyl-4,4'-diaminostilbene and N,N'-dimethyl-4,4'-diaminostilbene and Analysis via Optical Microscopy and Differential Scanning Calorimetry.

A portion (0.2485 grams) of the diglycidyl amine of N,N'-dimethyl-4,4'-diaminostilbene from Example 1-F above was combined with an equivalent amount (0.1541 grams) of N,N'-dimethyl-4,4'-diaminostilbene from Example 1-E above. These compounds are ground together to form a fine, homogeneous powder. A sample of this mixture was placed on a hot stage which had been heated to 100°C and then observed via optical microscopy under crosspolarized light at 70X magnification. At the 100°C temperature, a birefringent crystalline dispersion occurred in less than one minute. Heating to 140°C was then resumed and complete isotropization occurs at 132°C. After ten minutes at 140°C, the resin exhibits opacity at the edges of the coverslip and small birefringent domains throughout. After an additional twelve minutes at 140°C, the resin was still fluid and shear applied to the resin aligns the birefringent domains in a direction perpendicular to the direction of the shear. After an additional sixteen minutes at 140°C, the resin becomes an opaque, birefringent solid with a nematic texture. Further heating to 180°C reduced the number of nematic domains present with oriented domains still observable. After cooling to 25°C, a highly birefringent, opaque solid with a nematic texture was observed.

A second sample of the mixture was placed on a hot stage which had been heated to 140°C and then observed via optical microscopy under crosspolarized light at 70X magnification. At the 140°C temperature, an isotropic fluid containing a trace of birefringent domains was observed after three minutes. At this time, cooling at a rate of 10°C per minute was initiated. Once 120°C was achieved, this temperature was held. After eleven minutes at 120°C the resin still appears as an isotropic fluid containing a trace of birefringent domains. After an additional six minutes at 120°C, a second birefringent phase forms and the resin becomes opaque and viscous. At this time, application of shear to the resin induces alignment of some of the birefringent domains in the direction of the shear. After an additional sixteen minutes at 120°C, the resin becomes an opaque, birefringent solid with a nematic texture with a diminished number of oriented domains observable. After an additional twenty seven minutes at 120°C, cooling to 25°C commences to provide a highly birefringent, opaque solid with a nematic texture.

A third sample (10.10 milligrams) of the mixture was analyzed by differential scanning calorimetry using a heating rate of 10°C per minute and a temperature range of 30 to 280°C under a stream of nitrogen flowing at 35 cubic centimeters per minute. The results are given in Table III.

**Table III**

| Cycle Designation | Observed Transition Temperatures (°C) midpoint/range | Enthalpy (j/g) | Comments |
|---|---|---|---|
| First heating (30 to 280°C) | 82/50-126 | 33.5 | single endotherm |
| | 189/127-243 | 162.3 | broad exotherm |

### EXAMPLE 4 Preparation of a Neat Resin Casting of the Diglycidyl Amine of N,N'-dimethyl-4,4'-diaminostilbene Cured with N,N'-dimethyl-4,4'-diaminostilbene and Analysis via Optical Microscopy and Differential Scanning Calorimetry.

The powder mixture of the diglycidyl amine of N,N'-dimethyl-4,4'-diaminostilbene and N,N'-dimethyl-4,4'-diaminostilbene remaining from Example 3 was transferred to an aluminum cup. The aluminum cup containing this mixture was placed in an oven which had been heated to 140°C. After one minute at 140°C, a partial melt was observed. After an additional nine minutes, a melt was obtained. At this time, the oven temperature was reduced to 120°C. After an additional seventeen minutes, the resin becomes viscous and immobile. After an additional fifteen minutes, the resin gelled to an opaque solid which was then held at the 120°C temperature for an additional five hours and fifteen minutes. Thin sections of the demolded casting exhibited a high level of birefringence and a nematic texture when observed via optical light microscopy under crosspolarized light at 70X magnification.

Differential scanning calorimetry of a portion (25.0 milligrams) of the casting using a heating rate of 10°C per minute and a temperature range of 30 to 240°C under a stream of nitrogen flowing at 35 cubic centimeters per minute provided the results in Table IV.

**Table IV**

| Cycle Designation | Observed Transition Temperatures (°C) midpoint/range | Enthalpy (j/g) | Comments |
|---|---|---|---|
| First heating (30 to 240°C) | 183/165-196 | 3.2 | single endotherm |
| | 217/196-229 | 19.4 | single endotherm |

Analysis of a portion of the casting via crosspolarized light microscopy was completed using a microscope equipped with a programmable hot stage using a heating rate of 10°C per minute. The results are given in Table V.

**Table V**

| Cycle Designation | Observed Transition Temperatures (°C) | Comments |
|---|---|---|
| First heating (30 to 135°C) | 30 | Solid |
| | 170 | First melting observed |
| | 178 | Isotropic liquid containing birefringent domains. |
| | 216 | Isotropization. |

### EXAMPLE 5

### A. Synthesis of 3,3'-Dimethyl-4,4'-diacetamidobiphenyl

3,3'-Dimethyl-4,4'-diaminobiphenyl (12.74 grams, 0.12 -NH₂ equivalent) was ground to a fine powder and added to a beaker containing stirred, 90°C deionized water (1200 milliliters). After two minutes, aqueous 36.5% hydrochloric acid (12.00 grams, 0.12 mole) was added to the stirred, 90°C suspension. The solution which formed in six minutes was cooled to 70°C. Acetic anhydride (15.04 grams, 0.473 mole) was added to the stirred solution and mixed therein for 20 seconds before addition of an aqueous sodium acetate solution (prepared by dissolution of anhydrous sodium acetate [19.69 grams, 0.24 mole] in deionized water [60.0 grams]). After thoroughly stirring the white slurry, it was maintained at 4°C for 16 hours, followed by filtration to remove the precipitated product. The recovered filter cake was washed with two portions (150 milliliters) of deionized water then dried in a vacuum oven at 60°C and 5 mm Hg to a constant weight of 16.92 grams. The product was recovered as a white crystalline powder. Fourier transform infrared spectrophotometric analysis of a potassium chloride pellet of the product confirmed the product structure: disappearance of the absorbances observed for the -NH₂ group, appearance of solid state >NH stretching absorbances at 3283 (3183 slight shoulder) cm⁻¹, a solid state amide I carbonyl stretching absorbance at 1656 cm⁻¹, a solid state amide II carbonyl stretching absorbance at 1516 cm⁻¹ (masking the aromatic ring absorbance) and a C-H out-of-plane bending vibration at 819 cm⁻¹ due to the para-disubstituted aromatic rings. Proton nuclear magnetic resonance spectroscopy further confirmed the product structure.

### B. Synthesis of 3,3'-Dimethyl-N,N'-dimethyl-4,4'-diacetamidobiphenyl

3,3'-Dimethyl-4,4'-diacetamidobiphenyl (30.03 grams, 0.2027 -NH-CO- equivalent) prepared using the method of A above, tetra-n-butylammonium hydrogen sulfate (6.79 grams, 0.02 mole), powdered 99% sodium hydroxide (28.0 grams, 0.70 mole), anhydrous powdered potassium carbonate (56.0 grams, 0.4052 mole) and benzene (800 milliliters) were added to a reactor and stirred under a nitrogen atmosphere to provide a 21°C mixture. After two minutes, dimethyl sulfate (37.84 grams, 0.30 mole) was added to the stirred mixture then heating was initiated. After eleven minutes, the mixture reached a reflux temperature of 81°C. After 208 minutes at the 81°C reflux, Fourier transform infrared spectrophotometric analysis of a sample of the organic layer of the reaction mixture, revealed that full conversion to the N,N'-dimethyl derivative had occurred. Sixteen minutes later, additional benzene (400 milliliters) and deionized water (300 grams) were added to the reactor and thoroughly mixed with the reaction mixture. The reactor contents were added to a separatory funnel and the aqueous layer which separated was removed and discarded. The benzene solution was washed with two portions of deionized water (200 milliliters), dried over anhydrous sodium sulfate, then rotary evaporated to remove solvent. The recovered powder was then dried in a vacuum oven at 50°C and 12 mm Hg to a constant weight of 32.61 grams. The product was recovered as a white crystalline powder. Fourier transform infrared spectrophotometric analysis of a film of the product on a potassium chloride plate confirmed the product structure: disappearance of the absorbances observed for the secondary amide group, appearance of a tertiary amide carbonyl stretching absorbance at 1663 cm⁻¹, a C-H stretching absorbance at 2878 cm⁻¹ due to the >N-CH₃ groups and a C-H out-of-plane bending vibration at 826 cm⁻¹ due to the para-disubstituted aromatic rings. Proton nuclear magnetic resonance spectroscopy further confirmed the product structure.

### C. Hydrolysis of 3,3'-Dimethyl-N,N'-dimethyl-4,4'-diacetamidobiphenyl

3,3'-Dimethyl-N,N'-dimethyl-4,4'-diacetamidobiphenyl (32.44 grams, 0.20 -N-(CH3)-CO-equivalent) from B above was added to a reactor and dissolved with stirring under a nitrogen atmosphere in ethanol (600 milliliters). Concentrated hydrochloric acid (157.87 grams, 1.60 moles) was added to the resultant 22°C solution inducing a maximum exotherm of 43°C two minutes later. The stirred reaction mixture was heated to 75°C over the next seven minutes. After 23 hours at 75°C, heating to a 85°C reflux commences. After 96 hours at the 86°C temperature, Fourier transform infrared spectrophotometric analysis of a sample of the solution as a neat film on a potassium chloride plate revealed that complete hydrolysis of the amide linkages had occurred concurrent with the formation of the amine hydrochloride salt of the diamine thus produced: disappearance of the tertiary amide carbonyl stretching absorbance, appearance of -NH₃⁺ stretching absorbances at 2659 and 2446 cm⁻¹, a NH₃⁺ bending absorbance at 1609 cm⁻¹ (masking the aromatic ring absorbance). At this time, deionized water (400 grams) was added to the reactor and thoroughly mixed with the reaction mixture. The reactor contents were cooled to 25°C then 50% aqueous sodium hydroxide was added with mixing to a pH of 9 followed by filtration of the precipitated product. The recovered filter cake was washed with two portions (100 milliliters) of deionized water then dried in a vacuum oven at 40°C and 10 mm Hg to a constant weight of 22.01 grams. The product was recovered as a white crystalline powder. Fourier transform infrared spectrophotometric analysis of a potassium bromide pellet of the product confirmed the product structure: disappearance of the tertiary amide carbonyl stretching absorbance, appearance of a >NH stretching absorbance at 3429 (3369 shoulder) cm⁻¹, a C-H stretching absorbance at 2818 cm⁻¹ due to the >N-CH₃ groups and a C-H out-of-plane bending vibration at 806 cm⁻¹ due to the para-disubstituted aromatic rings. Proton nuclear magnetic resonance spectroscopy further confirmed the product structure.

### D. Epoxidation of 3,3'-Dimethyl-N,N'-dimethyl-4,4'-diaminobiphenyl

3,3'-Dimethyl-N,N'-dimethyl-4,4'-diaminobiphenyl (6.01 grams, 0.05 >NH equivalent) from C above, epichlorohydrin (18.51 grams, 0.20 mole) and ethylene glycol (2.48 gram, 0.08 hydroxyl equivalent) were added to a reactor and heated to 50°C with stirring under a nitrogen atmosphere. After 3 hours at the 50°C temperature, cooling to 35°C commenced. After ten minutes the 35°C temperature was achieved and sodium hydroxide (2.38 grams, 0.0595 mole) dissolved in deionized water (2.58 grams, 52% of total solution) was added dropwise to the reactor over a thirteen minute period and so as to maintain a 35 to 38°C reaction temperature. After an additional fifteen minutes at 35°C, the reaction mixture was recovered and rotary evaporated at 55°C and 2 mm Hg final vacuum to remove residual epichlorohydrin. The solids remaining after rotary evaporation were thoroughly mixed with methylethylketone (250 milliliters) then extracted with deionized water (100 milliliters). The mixture was added to a separatory funnel and the aqueous layer which separated was removed and discarded. The methylethylketone solution was washed with two portions (75 milliliters) of deionized water, then rotary evaporated at 50°C and 1 mm Hg final vacuum for 90 minutes. The product was recovered as a tacky clear amber solid with an epoxide equivalent weight (EEW) of 188.73 (corrected for titrated contribution of amine nitrogen).

## Claims

1. A glycidyl amine-containing composition containing one or more mesogenic moieties represented by the following Formulas I-VIII 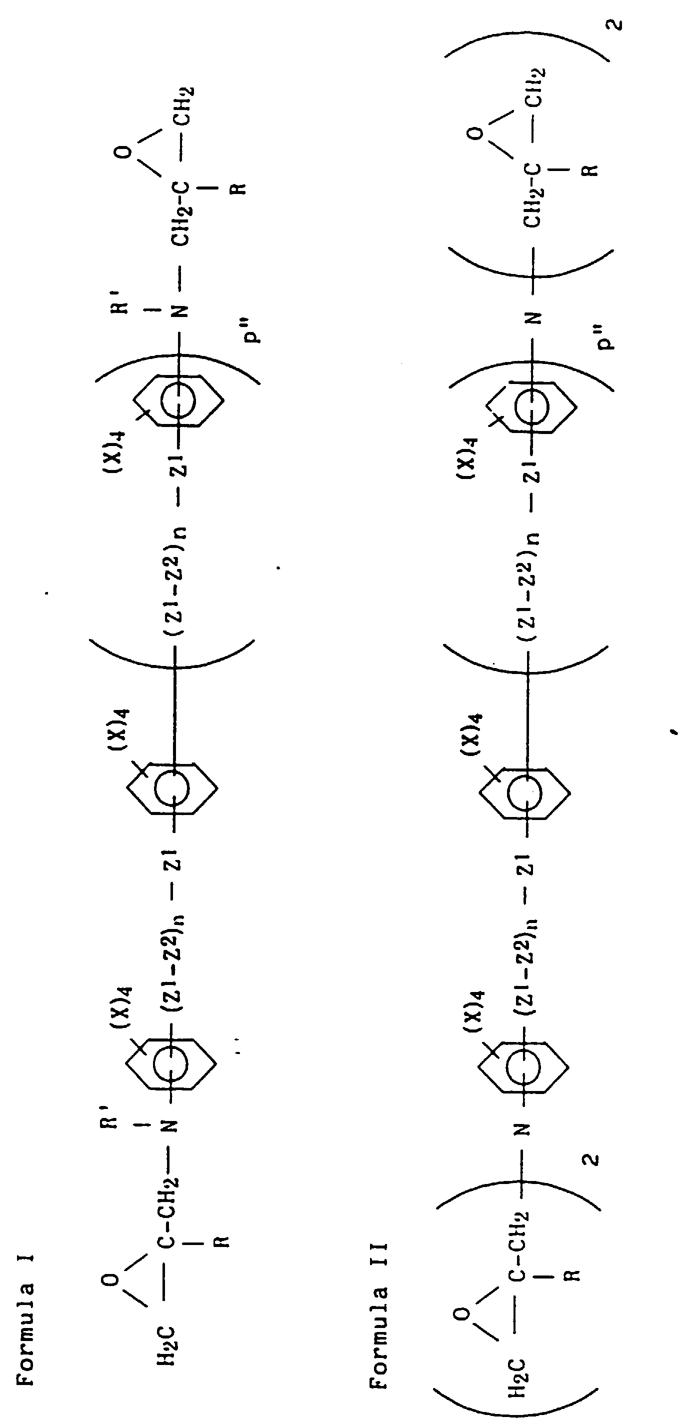 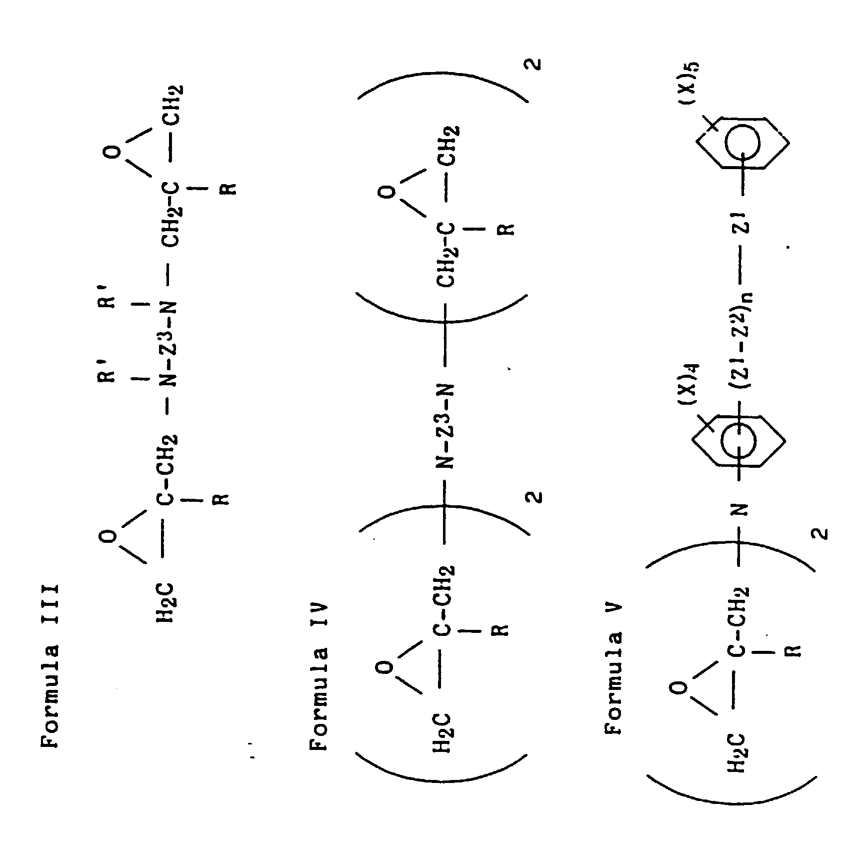 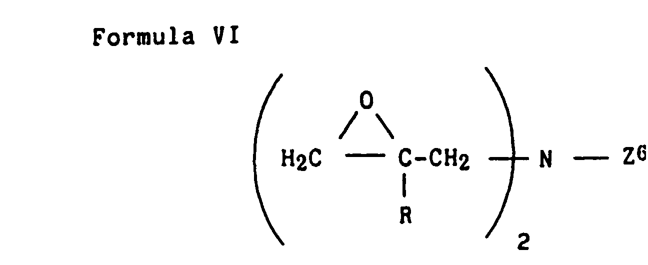 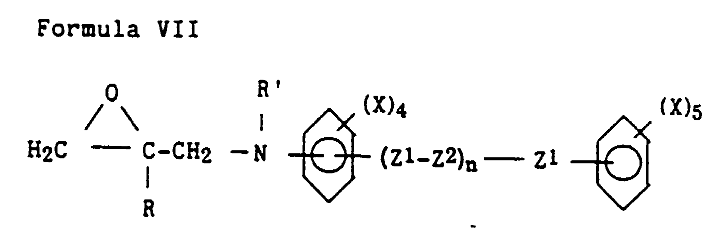 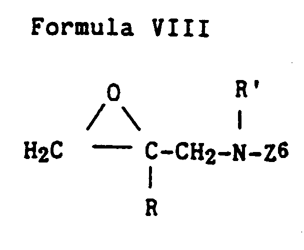 wherein at least 80 percent of the -(Z¹-Z²)ₙ-Z¹- linkages and the glycidyl amine groups are in the para position with respect to each other; each R and R¹ is independently hydrogen or an aliphatic hydrocarbon group having from 1 to 4 carbon atoms; each R' is independently a hydrocarbyl group having from 1 to 12 carbon atoms; each X is independently hydrogen, a hydrocarbyl or hydrocarbolyloxy group having suitably from 1 to 12 carbon atoms, a halogen atom, -NO₂, or -C≡N;
X¹ is a hydrocarbyl group having from 1 to 10 carbon atoms which can contain one or more heteroatoms selected from N, O or S and may be saturated or unsaturated; each Z¹ is independently a direct single bond, -CR¹=CR¹-, -CR¹=CR¹-CR¹=CR¹-, -CR¹=N-N=CR¹, -CR¹=CR¹-CO-O-(CHR¹)_{p'}-, -CR¹=CR¹-O-CO-(CHR¹)_{p'}-, -(CHR¹)_{p'}-O-CO-CR¹=CR¹-, -(CHR¹)_{p'}-CO-O-CR¹=CR¹-, -CR¹=CR¹-CO-O-, -O-CO-CR¹=CR¹-, -CO-NR¹-, -NR¹-CO-, -CO-NR¹-NR¹-CO-, -C≡C-, -C≡C-C≡C-, -CO-S-, -S-CO-, -CR¹=N-, -N=CR¹, -O-CO-, -CO-O-, -CR¹=CR¹-CO-, -CO-CR¹=CR¹-, -CR¹=CR¹-O-CO-, -CO-O-CR¹=CR¹-, -CH₂-CH₂-CO-O-, -O-CO-CH₂-CH₂-, -N=N-, 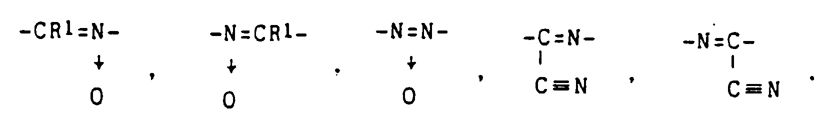 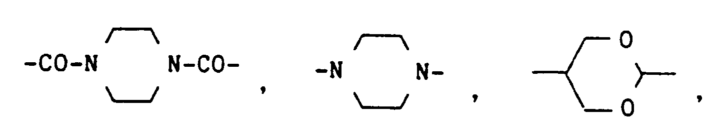 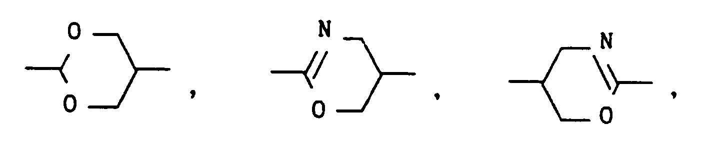 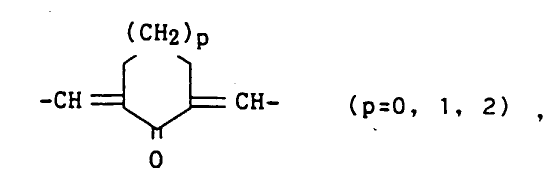 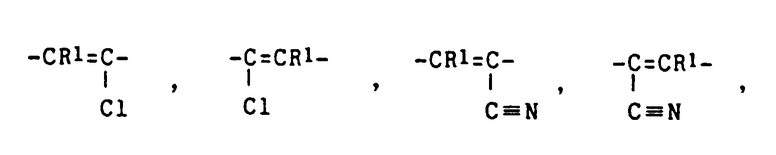 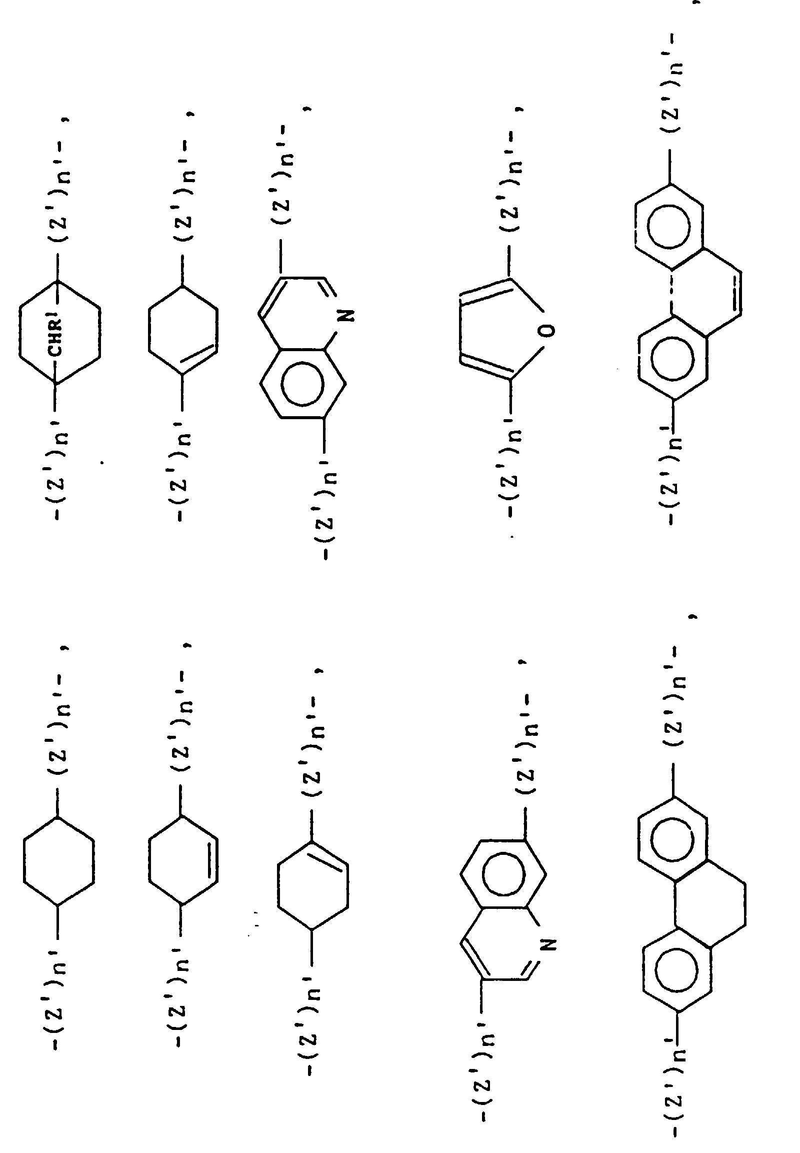 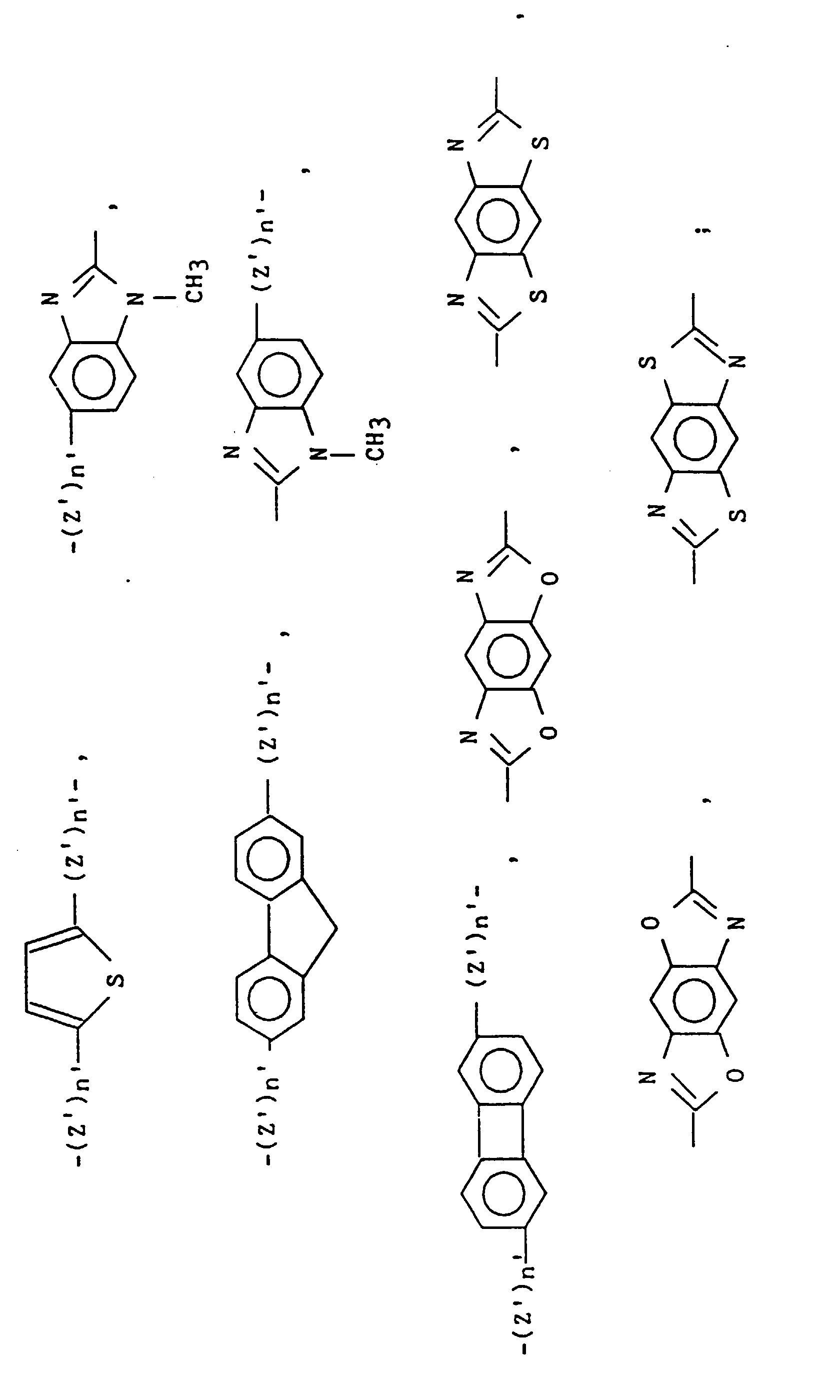 Z² is a group represented by a cyclic or bicyclic ring system containing from 5 to 12 carbon atoms and may be cycloaliphatic, polycycloaliphatic, aromatic or a combination thereof; Z³ is 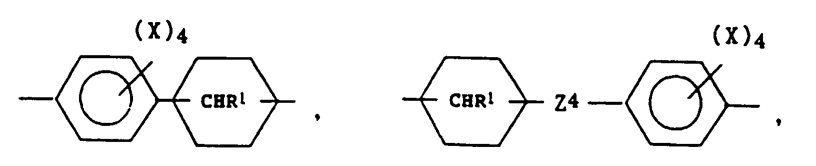 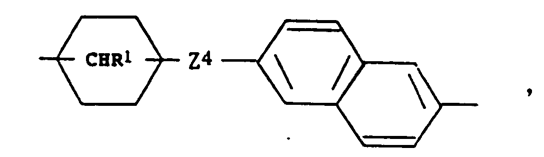 or 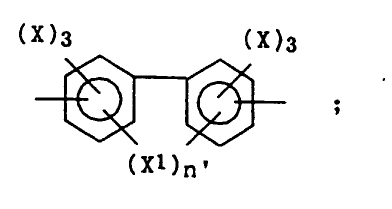 Z⁴ is -CO-O-, -O-CO-, -NR¹-CO- or -CO-NR¹-; Z⁶ is 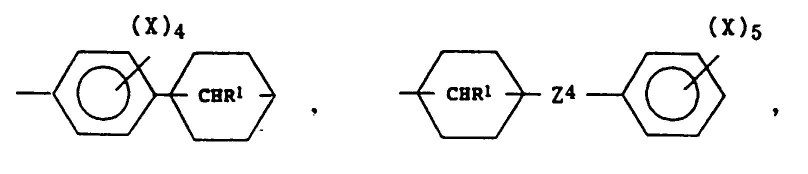 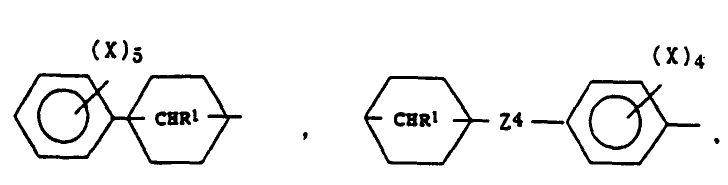 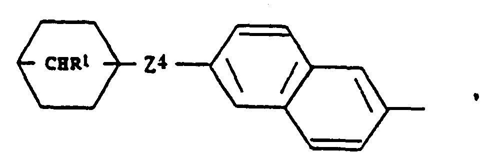 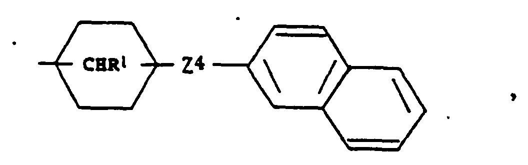 or 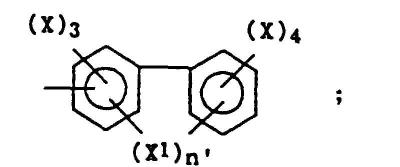 n is 0 to 2; n' is zero or 1; p' is 1 or 2; p" has a value of zero to 100; each Z' is independently a -CO-, -O-CO-, -CO-O-; -CO-NR¹-, or -NR¹-CO- group; with the proviso that the polyglycidyl amine of Formula II is not the tetraglycidyl amine of 4,4'-diaminobiphenyl (R is H, X is H, n=0, p"=0, Z¹ is a direct single bond) or the tetraglycidyl amine of 3,3'-dimethyl-4,4'-diaminobiphenyl (R is H, X in the 3,3' position is -CH₃ and H in all other positions, n=0, p"=0, Z¹ is a direct single bond) and that in Formula II n and p" are not zero at the same time.

2. A polyglycidyl amine composition comprising a diglycidyl amine of N,N'-dimethyl-4,4'-diaminostilbene, N,N'-dimethyl-3,3'-dimethyl-4,4"-diaminobiphenyl, or any combination thereof.

3. An advanced polyglycidyl amine composition prepared by reacting
(a) one or more polyglycidyl amines containing one or more mesogenic moieties, said polyglycidyl amines being those represented by Formulas I-VI, with the proviso that the polyglycidyl amine of Formula II may include the tetraglycidyl amine of 4,4'-diaminobiphenyl or the tetraglycidyl amine of 3,3'-dimethyl-4,4'-diaminobiphenyl; with
(b) at least one compound having an average of more than one active hydrogen atom per molecule; wherein components (a) and (b) are employed in quantities which provide a ratio of active hydrogen atoms per epoxide group of from 0.01:1 to 0.95:1; with the proviso that when component (a) contains polyglycidyl amine compounds containing an average of more than two glycidyl groups per molecule, such compounds are present in an amount such that less than 15 percent of the glycidyl groups are contributed by the compounds containing an average of more than two glycidyl groups per molecule.

4. An advanced polyglycidyl amine composition of Claim 3 wherein
(a) when component (A) is a polyglycidyl amine represented by Formulas I or III, R' has from 1 to 6 carbon atoms and when X is a hydrocarbyl or hydrocarbyloxy group, it has from 1 to 6 carbon atoms and when X is a halogen atom, it is chlorine or bromine;
(b) when component (A) is a polyglycidyl amine represented by Formulas II, IV, V, or VI, and when X is a hydrocarbyl or hydrocarbyloxy group, it has from 1 to 6 carbon atoms and when X is a halogen atom, it is chlorine or bromine;
(c) component (B) is a compound represented by the following Formulas XXV and XXVI 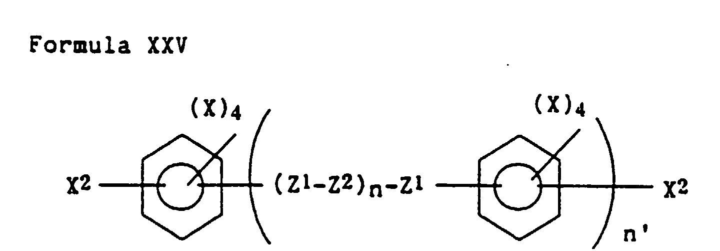 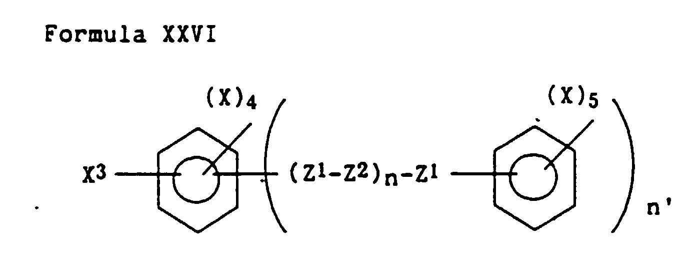 wherein X² is independently a hydroxyl, carboxylic acid, -SH, or -NHR' group; each R' is independently a hydrocarbyl group having from 1 to 12 carbon atoms; X³ is -NH₂, NH₂-SO₂-, NH₂-CO-, or NH₂-Z⁵-O-; Z⁵ is an alkyl or cycloalkyl group having from 1 to 12 carbon atoms; Z¹ can independently be a divalent hydrocarbyl group having from 1 to 10 carbon atoms, -O-, -CO-, -SO-, -SO₂-, -S-, -S-S-, -CR¹=CR¹-, -CR¹=CR¹-CR¹=CR¹-, -CR¹=N-N=CR¹-, -CR¹=CR¹-CO-O-(CHR¹)_{p'}-, -CR¹=CR¹-O-CO-(CHR¹)_{p'}-, -(CHR¹)_{p'}-O-CO-CR¹=CR¹-, -(CHR¹)_{p'}-CO-O-CR¹=CR¹-, -CR¹=CR¹-CO-O-, -O-CO-CR¹=CR¹-, -CO-NR¹-, -NR¹-CO-, -CO-NR¹-NR¹-CO-, -C≡C-, -C≡C-C≡C-, -CO-S-, -S-CO-, -CR¹=N-, -N=CR¹-, -O-CO-, -CO-O-, -CR¹=CR¹-CO-, -CO-CR¹=CR¹-, -CR¹=CR¹-O-CO-, -CO-O-CR¹=CR¹-, -CH₂-CH₂-CO-O-, -O-CO-CH₂-CH₂-, -N=N-, a direct single bond, 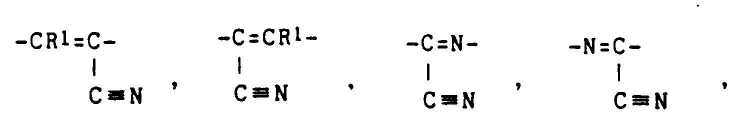 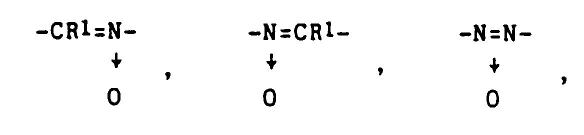 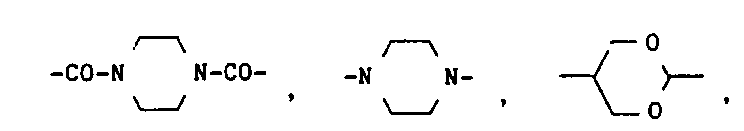 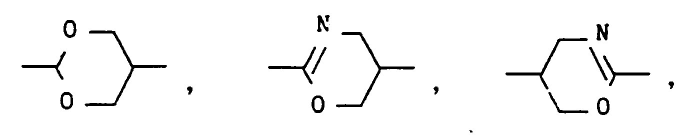 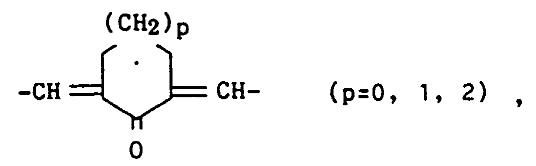 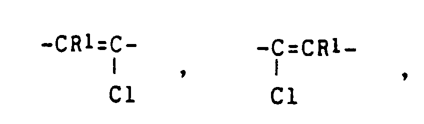 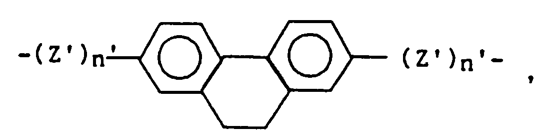 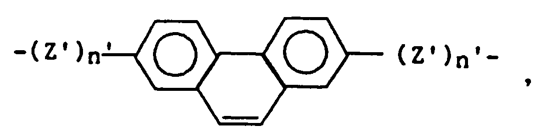 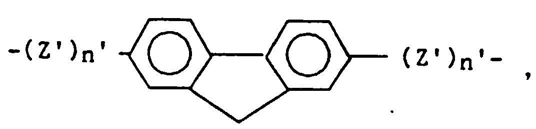 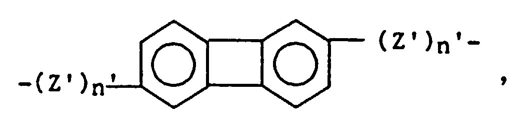 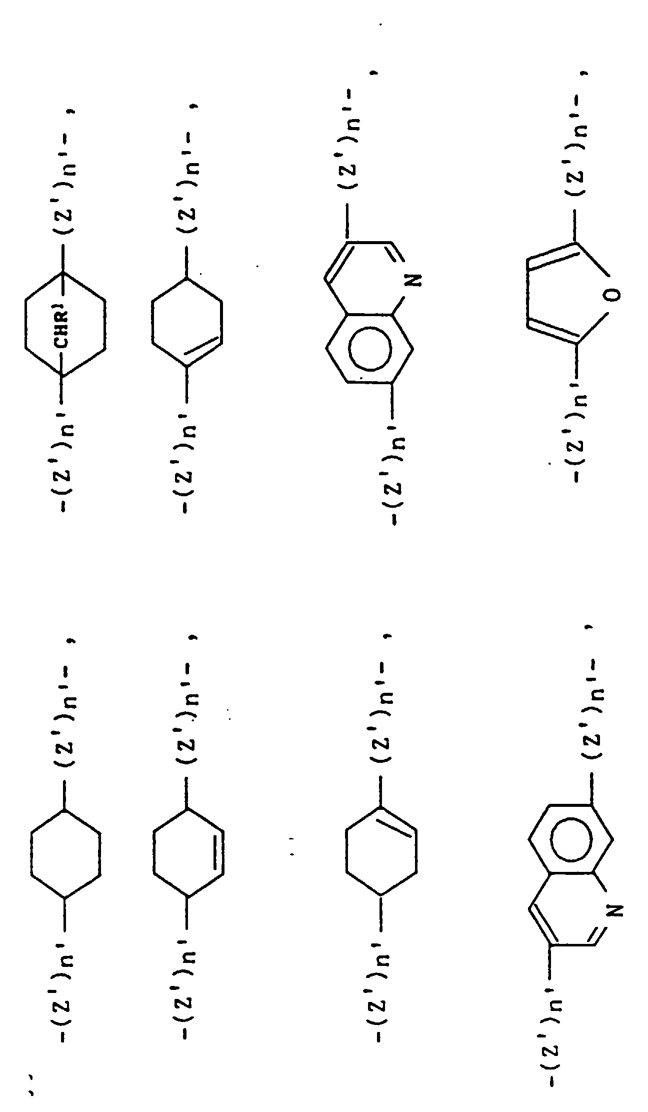 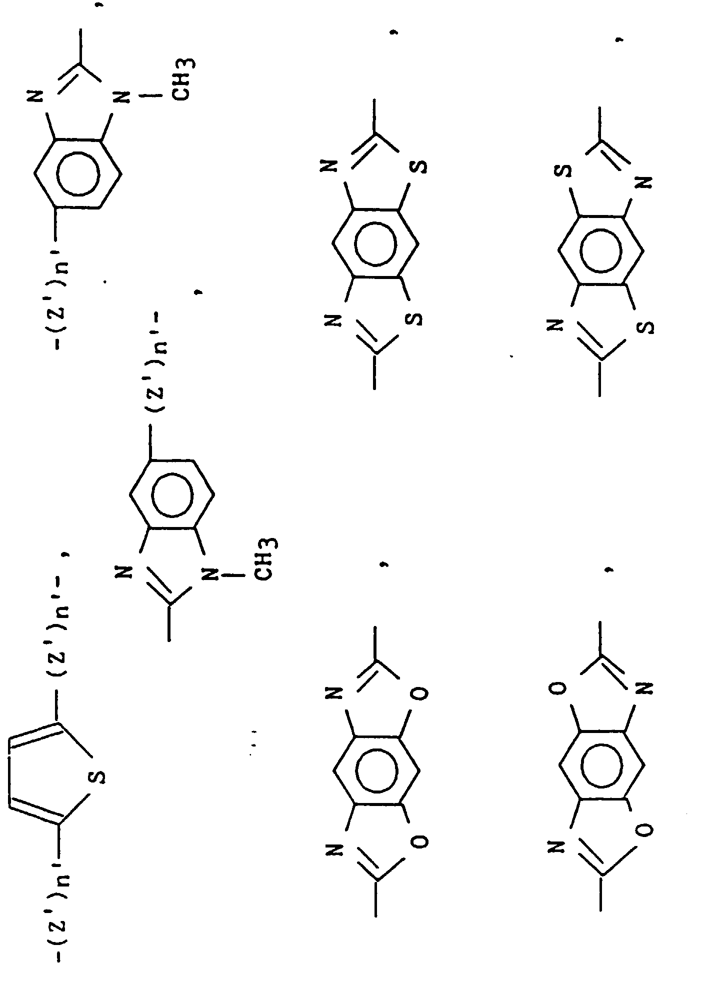 and each X is independently hydrogen. a hydrocarbyl or hydrocarbyloxy group having from 1 to 12 carbon atoms, a halogen atom, -NO₂ or -C≡N; Z² is a group represented by a cyclic or bicyclic ring system containing from 5 to 12 carbon atoms and may be cycloaliphatic, polycycloaliphatic, aromatic or a combination thereof; n is zero to 2; each Z' is independently a -CO-, -O-CO-, -CO-O-, -CO-NR¹-, or -NR¹-CO- group; each R¹ is independently hydrogen or an aliphatic hydrocarbon group having from 1 to 4 carbon atoms and each n' independently has a value of zero or one; or any combination of any two or more compounds represented by the aforementioned Formulas XXV and XXVI; and
(d) components (A) and (B) are employed in amounts which provide a ratio of active hydrogen atoms per epoxide group of from 0.05:1 to 0.8:1; and
with the proviso that when component (A) contains a polyglycidyl amine compound having an average of more than two glycidyl amine groups per molecule, not more than 10 percent of the total amount of glycidyl groups contained in component (A) are contributed by said polyglycidyl amine compound having an average of more than two glycidyl amine groups per molecule.

5. An advanced polyglycidyl amine of Claim 3 or 4 wherein
(a) component (B) is hydroquinone, bisphenol A,
4,4'-dihydroxydiphenylmethane, 4,4'-thiodiphenol, 4,4'-sulfonyldiphenol, 4,4'-dihydroxydiphenyl oxide, 4,4'-dihydroxybenzophenone, 1,1-bis(4-hydroxyphenyl)-1-phenylethane, 3,3',5,5'-tetrachlorobisphenol A, 3,3'-dimethoxybisphenol A, 4,4'-dihydroxybiphenyl, 4,4'-dihydroxy-α,α'-diethylstilbene, 4,4'-dihydroxy-α-methylstilbene, 4,4'-dihydroxybenzanilide, 4,4'-dihydroxy-2,2'-dimethylazoxybenzene, 4,4'-dihydroxy-α-cyanostilbene, bis(4-hydroxyphenyl)terephthalate, N,N'-bis(4-hydroxyphenyl)terephthalamide, bis(4'-hydroxybiphenyl)terephthalate, 4,4'-dihydroxyphenylbenzoate, bis(4'-hydroxyphenyl)-1,4-benzenediimine, 4,4"-dihydroxybiphenylbenzoate, 1,4-bis(4'-hydroxyphenyl-1'-carboxamide)benzene, 1,4-bis(4'-hydroxyphenyl-1'-carboxy)benzene, 4,4'-bis(4"-hydroxyphenyl-1"-carboxy)biphenyl, terephthalic acid, 4,4'-benzanilide dicarboxylic acid, 4,4'-phenylbenzoate dicarboxylic acid, 4,4'-stilbenedicarboxylic acid, 4,4'-dicarboxybiphenyl, 4,4'-dicarboxydiphenylazomethine, aniline, 4'-sulfonamido-N-phenylbenzamide, 4'-sulfonamido-N'-phenyl-4-chlorobenzamide, 4-amino-1-phenylbenzoate, 4-amino-N-phenylbenzamide, N-phenyl-4-aminophenyl-1-carboxamide, phenyl-4-aminobenzoate, biphenyl-4-aminobenzoate, 1-phenyl-4'-aminophenylterephthalate, N,N'-dimethyl-4,4'-diaminodiphenylmethane, N,N'-diethyl-4,4'-diaminodiphenylmethane, N,N'-dimethyl-4,4'-diaminobiphenyl, N,N'-dimethyl-3,3'-dimethyl-4,4'-diaminobiphenyl, N,N'-dimethyl-4,4'-diaminostilbene, N,N'-dimethyl-4,4'-diaminodiphenyl sulfone. N,N'-dimethyl-4,4'-diaminobenzanilide, N,N'-dimethyl-4,4'-diamino-N-methylbenzanilide, and any combination of any two or more of such compounds: and
(b) components (A) and (B) are employed in amounts which provide a ratio of active hydrogen atoms per epoxide group of from 0.1:1 to 0.5:1; and
with the proviso that when component (A) contains a polyglycidyl amine compound having an average of more than two glycidyl amine groups per molecule, not more than 5 percent of the total amount of glycidyl groups contained in component (A) are contributed by said polyglycidyl amine compound having an average of more than two glycidyl amine groups per molecule.

6. A blend comprising
(A) one or more polyglycidyl amine or monoglycidyl amine compounds containing one or more mesogenic moieties represented by Formulas I-VIII with the proviso that the polyglycidyl amine of Formula II can include the tetraglycidyl amine of 4,4'-diaminobiphenyl or the tetraglycidyl amine of 3,3'-dimethyl-4,4'-diaminobiphenyl; and
(B) one or more polyepoxide compounds which are substantially free of mesogenic moieties.

7. A blend of Claim 6 wherein:
(a) when component (A) is a polyglycidyl amine represented by Formulas I or III, R' has from 1 to 6 carbon atoms, and when X is a hydrocarbyl or hydrocarbyloxy group, it has from 1 to 6 carbon atoms and when X is a halogen atom. it is chlorine or bromine;
(b) when component (A) is a polyglycidyl amine represented by Formulas II, IV, V, or VI, and when X is a hydrocarbyl or hydrocarbyloxy group, it has from 1 to 6 carbon atoms and when X is a halogen atom, it is chlorine or bromine;
(c) when component (A) is a monoglycidyl amine represented by Formulas VII and VIII, R' has from 1 to 6 carbon atoms, and when X is a hydrocarbyl or hydrocarbyloxy group, it has from 1 to 6 carbon atoms and when X is a halogen atom, it is chlorine or bromine;
(d) component (B) is an epoxy resin represented by the following Formulas IX-XV 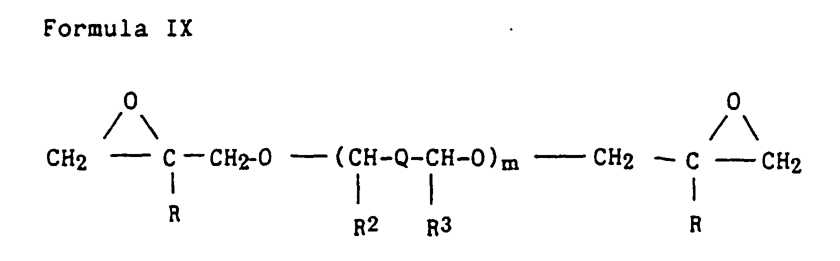 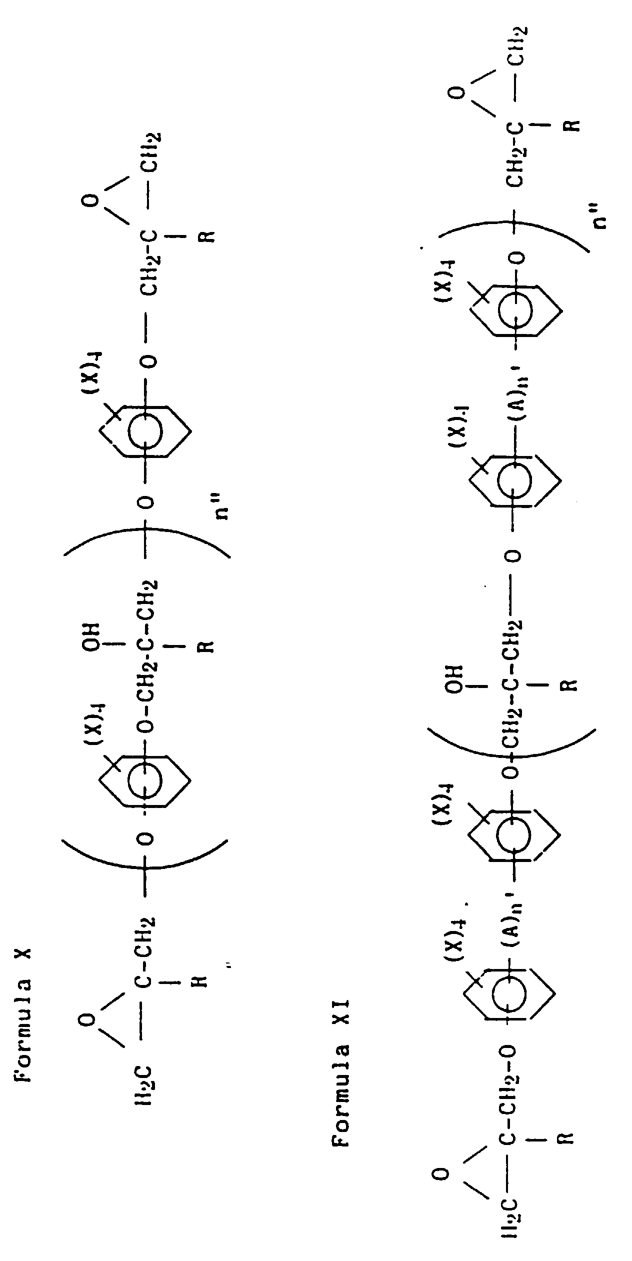 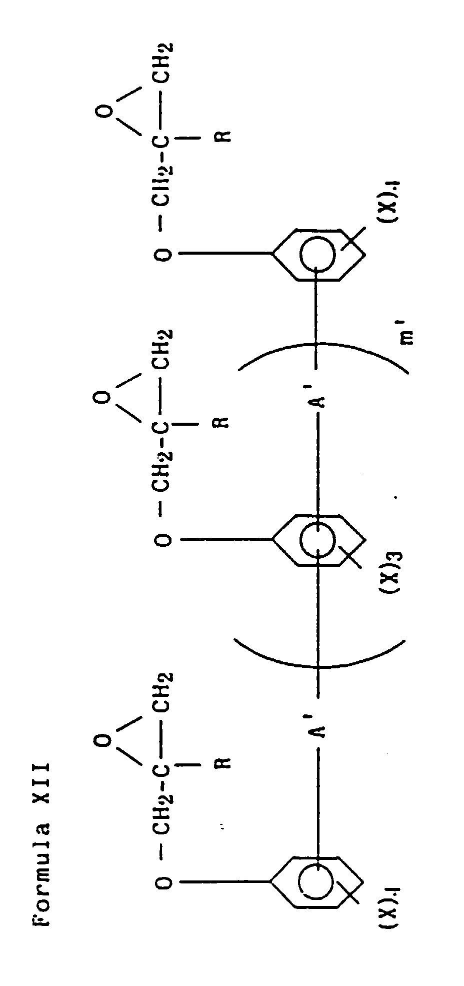 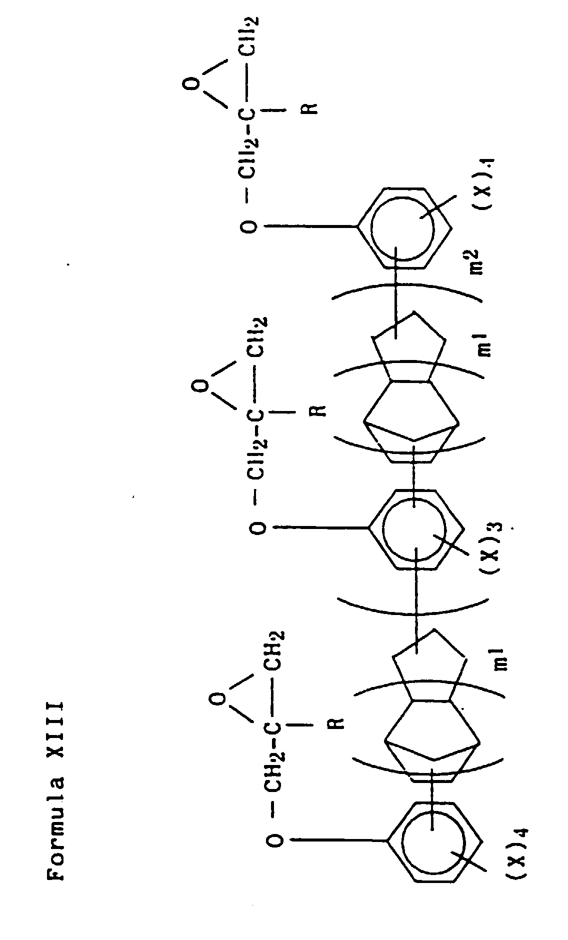 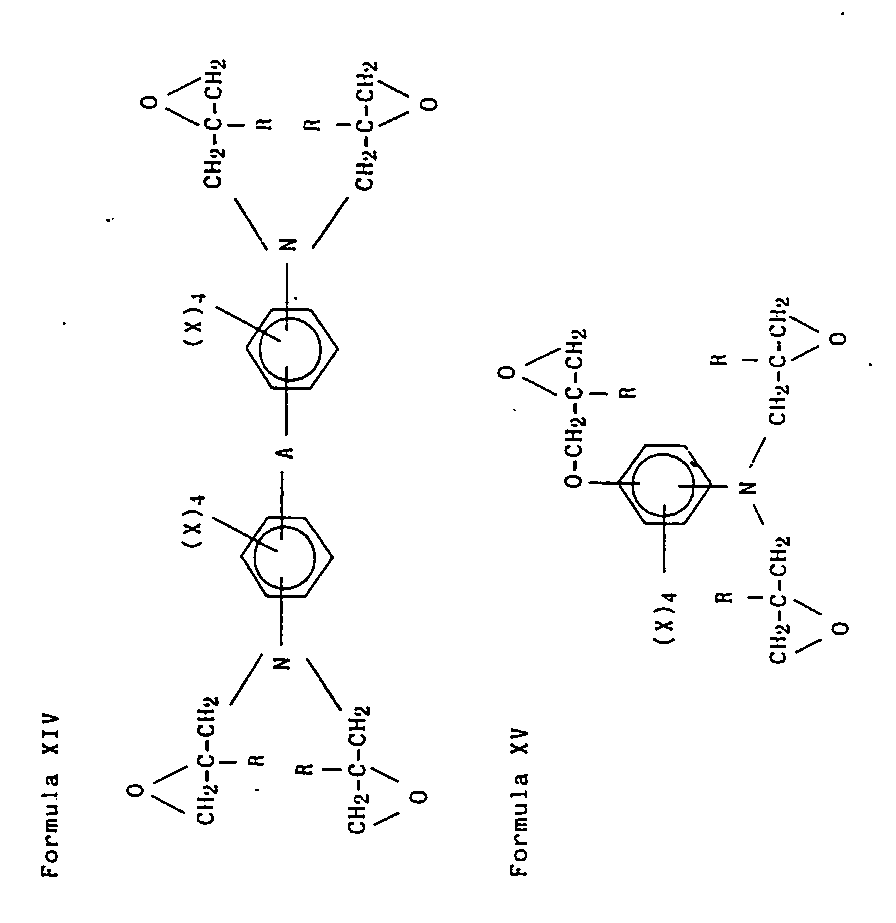 wherein each A is independently a divalent hydrocarbyl group having from 1 to 12 carbon atoms, -O-, -S-, -S-S-. -SO-, -SO₂-, or -CO-; each A' is independently a divalent hydrocarbon group having from 1 to 6 carbon atoms: Q is a single bond. -CH₂-S-CH₂-, -(CH₂)_{n¹}-, or 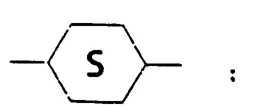 each R is independently hydrogen or an alkyl group having from 1 to 4 carbon atoms; each R² and R³ is independently nydrogen, a hydrocarbyl or halohydrocarbyl group having from 1 to 6 carbon atoms; each X is independently hydrogen, a hydrocarbyl or hydrocarbyloxy group having from 1 to 12, carbon atoms, a halogen atom, -NO₂ or -C≡N; m has a value from 1 to 10; m' has an average value from 0.01 to 12; m¹ has an average value from 1 to 12; m² has a value from 1 to 12; n' has a value of zero or 1; n" has an average value from zero to 3 and n¹ has an average value from 1 to 10; and
(e) component (A) is present in an amount of from 1 to 99 percent by weight based on the combined quantities of components (A) and (B): and component (B) is present in an amount of from 99 to 1 percent by weight based on the combined quantities of components (A) and (B).

8. A blend of Claim 7 wherein component (A) is a diglycidyl amine of N,N'-dimethyl-4,4'-diaminostilbene, N,N'-dimethyl-4,4'-diaminobiphenyl or combination thereof, and component (B) is a diglycidyl ether of bisphenol A, a diglycidyl ether of 4,4'-dihydroxydiphenylmethane. a diglycidyl ether of 4,4'-dihydroxybenzophenone , a diglycidyl ether of 4,4'-dihydroxythiodiphenol, a diglycidyl ether of 1,1-bis(4-hydroxylphenyl)-1-phenylethane (bisphenol AP), or any combination thereof.

9. A blend comprising
(A) an advanced polyglycidyl amine composition prepared by reacting
(1) one or more polyglycidyl amines containing one or more mesogenic moieties. said polyglycidyl amines being those represented by Formulas I-. VI, with the proviso that the polyglycidyl amine of Formula II can include the tetraglycidyi amine of 4,4'-diaminobiphenyl or the tetraglycidyl amine of 3,3'-dimethyl-4,4'-diaminobiphenyl; with
(2) at least one compound having an average of more than one active hydrogen atom per molecule: and wherein components (1) and (2) are employed in quantities which provide a ratio of active hydrogen atoms per epoxide group of from 0.01:1 to 0.95:1, with the proviso that when component (A1) contains a polyglycidyl amine compound having an average of more than two glycidyl amine groups per molecule, not more than 15 percent of the total amount of glycidyl groups contained in component (A1) are contributed by said polyglycidyl amine compound having an average of more than two glycidyl amine groups per molecule; and
(B) one or more polyepoxides which are substantially free of mesogenic moieties.

10. A blend of claim 9 wherein
(a) component (2) is hydroquinone, bisphenol A, 4,4'-dihydroxydiphenylmethane, 4,4'-thiodiohenol, 4,4'-sulfonyldiphenol, 4,4'-dihydroxydiphenyl oxide, 4,4'-dihydroxybenzophenone, 1,1-bis(4-hydroxyphenyl)-1-phenylethane, 3,3',5,5'-tetrachlorobisphenol A, 3,3'-dimethbxybisphenol A, 4,4'-dihydroxybiphenyl, 4,4'-dihydroxy-α,α'-diethylstilbene, 4,4'-dihydroxy-α-methylstilbene, 4,4'-dihydroxybenzanilide, 4,4'-dihydroxy-2,2'-dimethylazoxybenzene, 4,4'-dihydroxy-α-cyanostilbene, bis(4-hydroxyphenyl)terephthalate, N,N'-bis(4-hydroxyphenyl)terephthalamide, bis(4'-hydroxybiphenyl)terephthalate, 4,4'-dihydroxyphenylbenzoate, bis(4'-hydroxyphenyl)-1,4-benzenediimine, 4,4"-dihydroxybiphenylbenzoate, 1,4-bis(4'-hydroxyphenyl-1'-carboxamide)benzene, 1,4-bis(4'-hydroxyphenyl-1'-carboxy)benzene, 4,4'-bis(4"-hydroxyphenyl-1"-carboxy)biphenyl, terephthalic acid, 4,4'-benzanilide dicarboxylic acid, 4,4'-phenylbenzoate dicarboxylic acid, 4,4'-stilbenedicarboxylic acid, 4,4'-dicarboxybiphenyl, 4,4'-dicarboxydiphenylazomethine, aniline, 4'-sulfonamido-N-phenylbenzahide, 4'-sulfonamido-N'-phenyl-4-chlorobenzamide, 4-amino-1-phenylbenzoate, 4-amino-N-phenylbenzamide, N-phenyl-4-aminophenyl-1-carboxamide, phenyl-4-aminobenzoate, biphenyl-4-aminobenzoate, 1-phenyl-4'-aminophenylterephthalate, N,N'-dimethyl4,4'-diaminodiphenylmethane, N,N'-diethyl-4,4'-diaminodiphenylmethane, N,N'-dimethyl-4,4'-diaminobiphenyl, N,N'-dimethyl-3,3'-dimethyl-4,4'-diaminobiphenyl, N,N'-dimethyl-4,4'-diaminostilbene, N,N'-dimethyl-4,4'-diaminodiphenyl sulfone, N,N'-dimethyl-4,4'-diaminobenzanilide, N,N'-dimethyl4,4'-diamino-N-methylbenzanilide, and any combination of any two or more of such compounds;
(b) components (1) and (2) are employed in amounts which provide a ratio of active hydrogen atoms per epoxide group of from 0.1:1 to 0.5:1.;
(c) component (A) is present in an amount of from 10 to 50 percent by weight based on the combined quantities of components (A) and (B); and
(d) component (B) is present in an amount of from 90 to 50 percent by weight based on the combined quantities of components (A) and (B).

11. A blend comprising
(A) one or more polyglycidyl amines according to one or more of the formulae I to VI of claim 1 containing an average of more than one vicinal epoxide group per molecule and one or more mesogenic moieties per molecule; and
(B) one or more monoglycidyl amine compounds according to one or both of the formulae VII and VIII of claim 1 containing only one vicinal epoxide group per molecule and one or more mesogenic moieties per molecule.

12. A blend of Claim 11 wherein component
(A) is present in an amount of from 10 to 50 percent by weight based upon the combined weight of components (A) and (8) and component (B) is present in an amount of from 90 to 50 percent by weight based upon the combined weight of components (A) and (B).

13. A curable composition comprising at least one composition of Claim 1, 2, 3, 4, 5, 6, 7, 8, 9, 10, 11 or 12 and a curing amount of a suitable curing agent therefor.

14. The product resulting from curing the curable composition of Claim 13.

15. The product resulting from orienting a curable composition of Claim 14 either prior to curing, during curing or both prior to and during curing.

16. The product of Claim 15 wherein said orientation is accomplished by means of an electric or magnetic field, or by the application of drawing or shear forces.

17. A phenoxy type resin prepared by reacting
(A) one or more polyglycidyl amines containing one or more mesogenic moieties, said polyglycidyl amines being those represented by Formulas I, III, V or VI;
(B) at least one compound having an average of more than one active hydrogen atom per molecule: wherein components (A) and (B) are employed in quantities which provide a ratio of active hydrogen atoms per epoxide group of from 0.96:1 to 1.05:1.

## Patentansprüche

1. Glycidylamin enthaltende Zusammensetzung, enthaltend eine oder mehrere mesogene Einheiten, welche durch die folgenden Formeln I - VIII wiedergegeben werden: 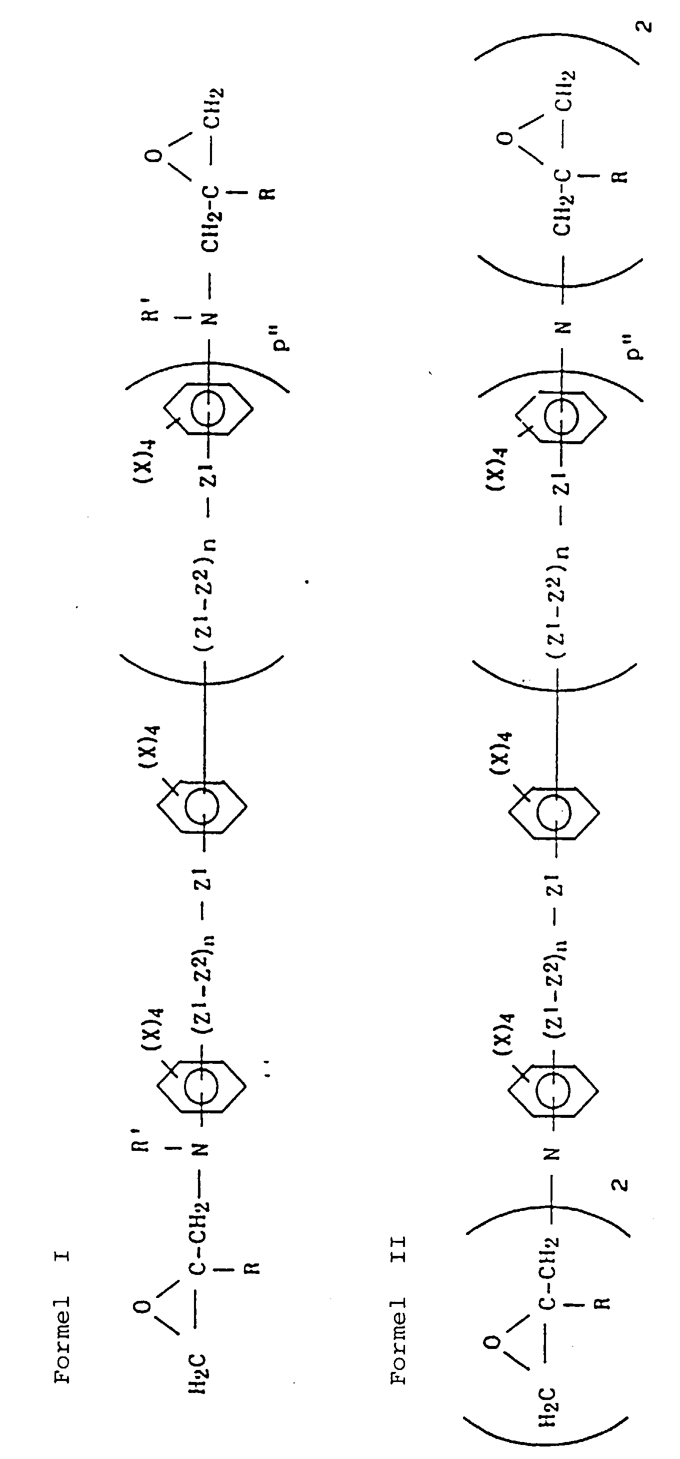 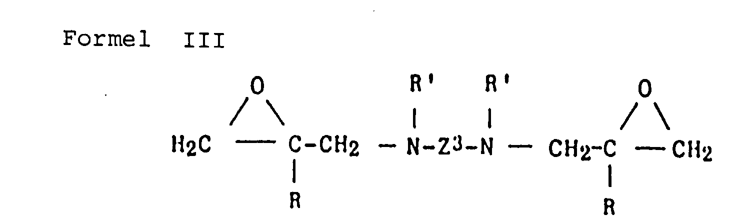 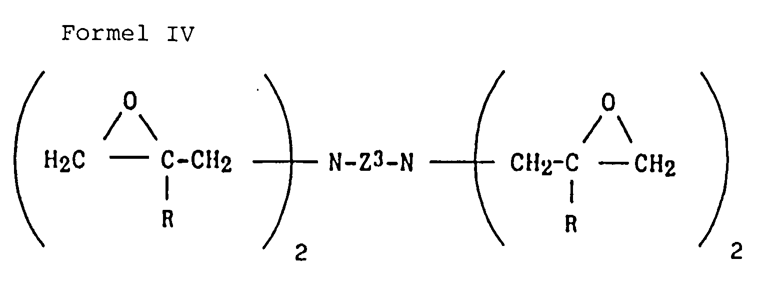 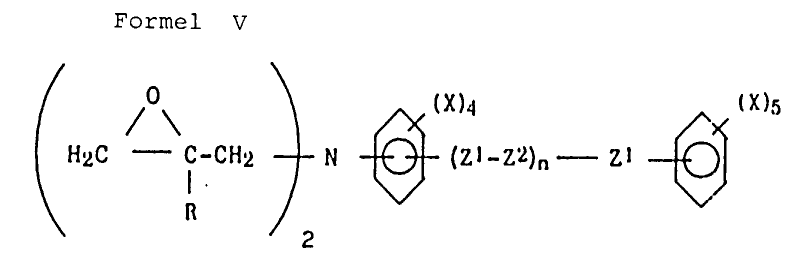 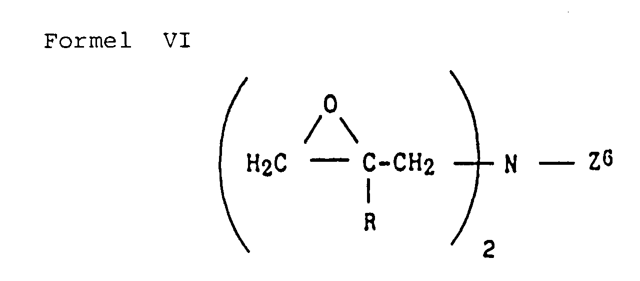 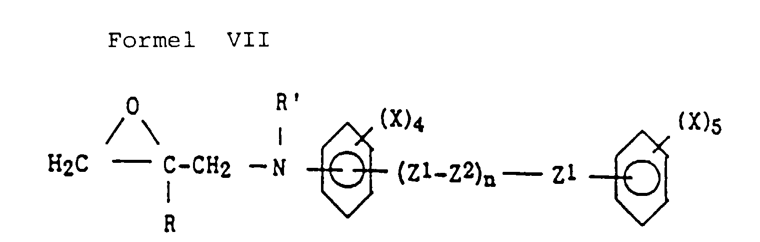 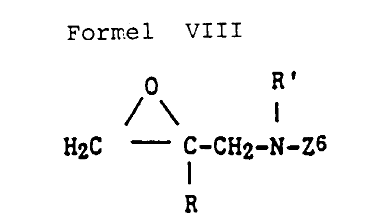 worin wenigstens 80 % der -(Z¹-Z²)ₙ-Z¹-Bindungen und die Glycidylamingruppen in der para-Stellung zueinander stehen; jedes R und R¹ unabhängig Wasserstoff oder eine aliphatische Kohlenwasserstoffgruppe mit 1 bis 4 Kohlenstoffatomen ist; jedes R' unabhängig eine Hydrocarbylgruppe mit 1 bis 12 Kohlenstoffatomen ist; jedes X unabhängig Wasserstoff, eine Hydrocarbyl- oder Hydrocarbyloxygruppe mit geeigneterweise 1 bis 12 Kohlenstoffatomen, ein Halogenatom,-NO₂ oder -C≡N ist; X¹ eine Hydrocarbylgruppe mit 1 bis 10 Kohlenstoffatomen ist, welche eine oder mehrere unter N, O oder S ausgewählte Heteroatome enthalten kann und gesättigt oder ungesättigt sein kann; jedes Z¹ unabhängig eine direkte Einzelbindung, -CR¹=CR¹-, -CR¹=CR¹-CR¹=CR¹-, -CR¹=N-N=CR¹-, -CR¹=CR¹-CO-O-(CHR¹)_{p'}-, -CR¹=CR¹-O-CO-(CHR¹)_{p'}-, -(CHR¹)_{p'}-O-CO-CR¹=CR¹-, (CHR¹)_{p'}-CO-O-CR¹=CR¹-, -CR¹=CR¹-CO-O-, -O-CO-CR¹=CR¹-, -CO-NR¹-, -NR¹-CO-, -CO-NR¹-NR¹-CO-, -C≡C-, -C≡C-C≡C-, -CO-S-, -S-CO-, -CR¹=N-, -N=CR¹-, -O-CO-, -CO-O-, -CR¹=CR¹-CO-, -CO-CR¹=CR¹-, -CR¹=CR¹-O-CO-, -CO-O-CR¹=CR¹-,-CH₂-CH₂-CO-O-, -O-CO-CH₂-CH₂-, -N=N-, 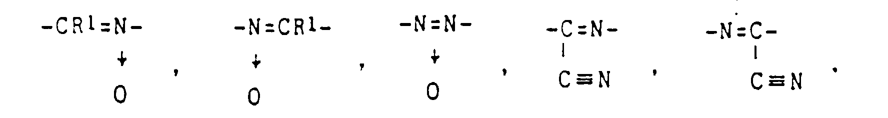 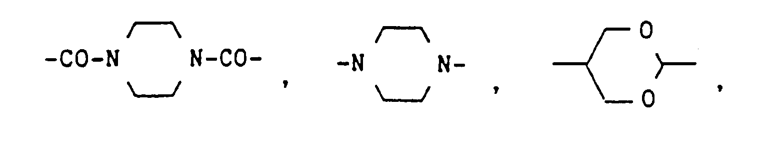 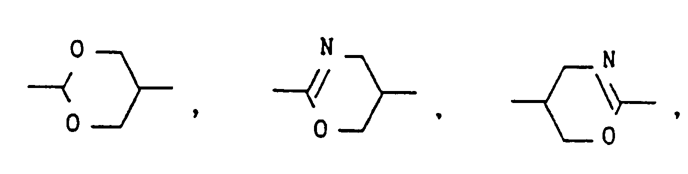 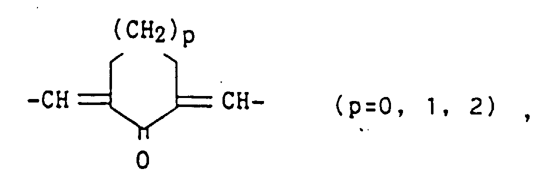 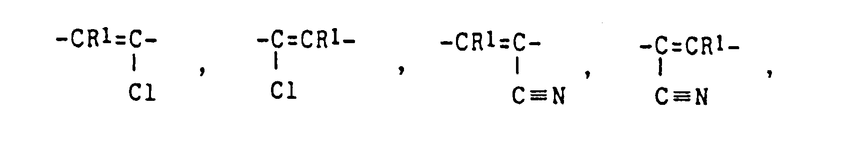 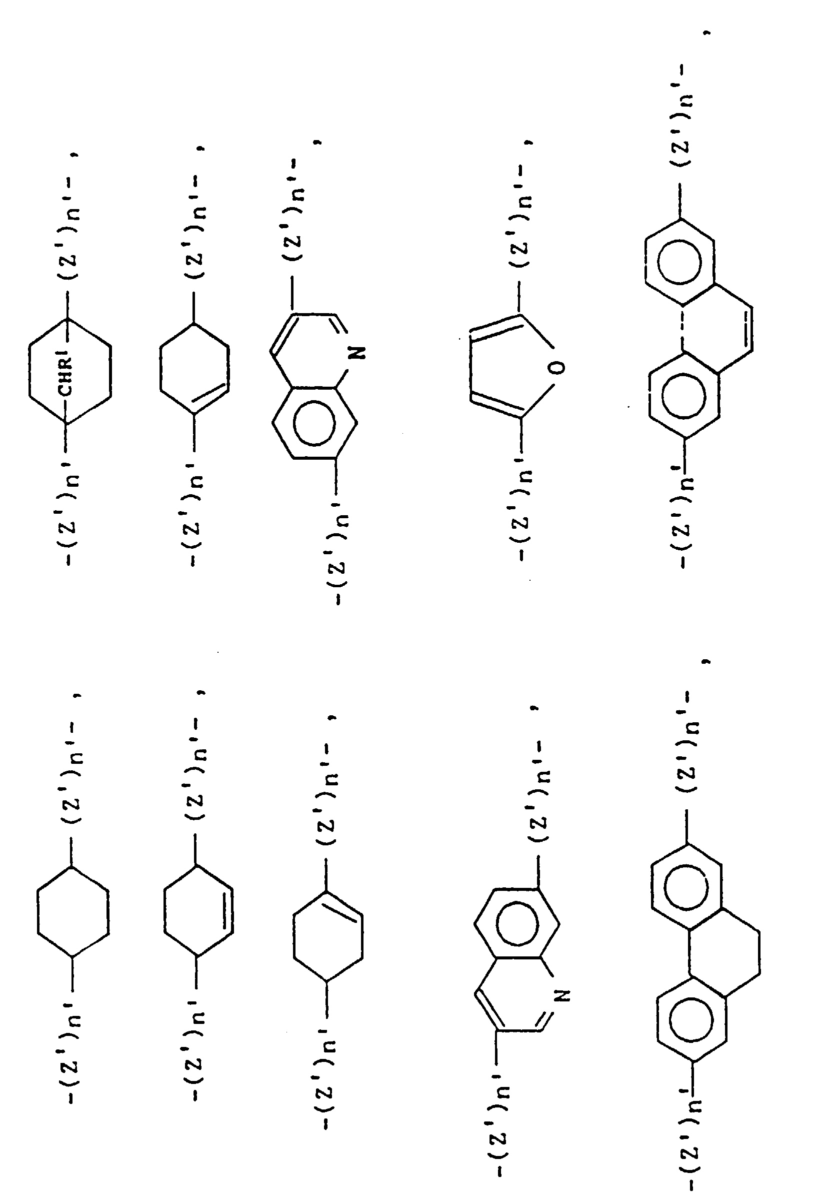 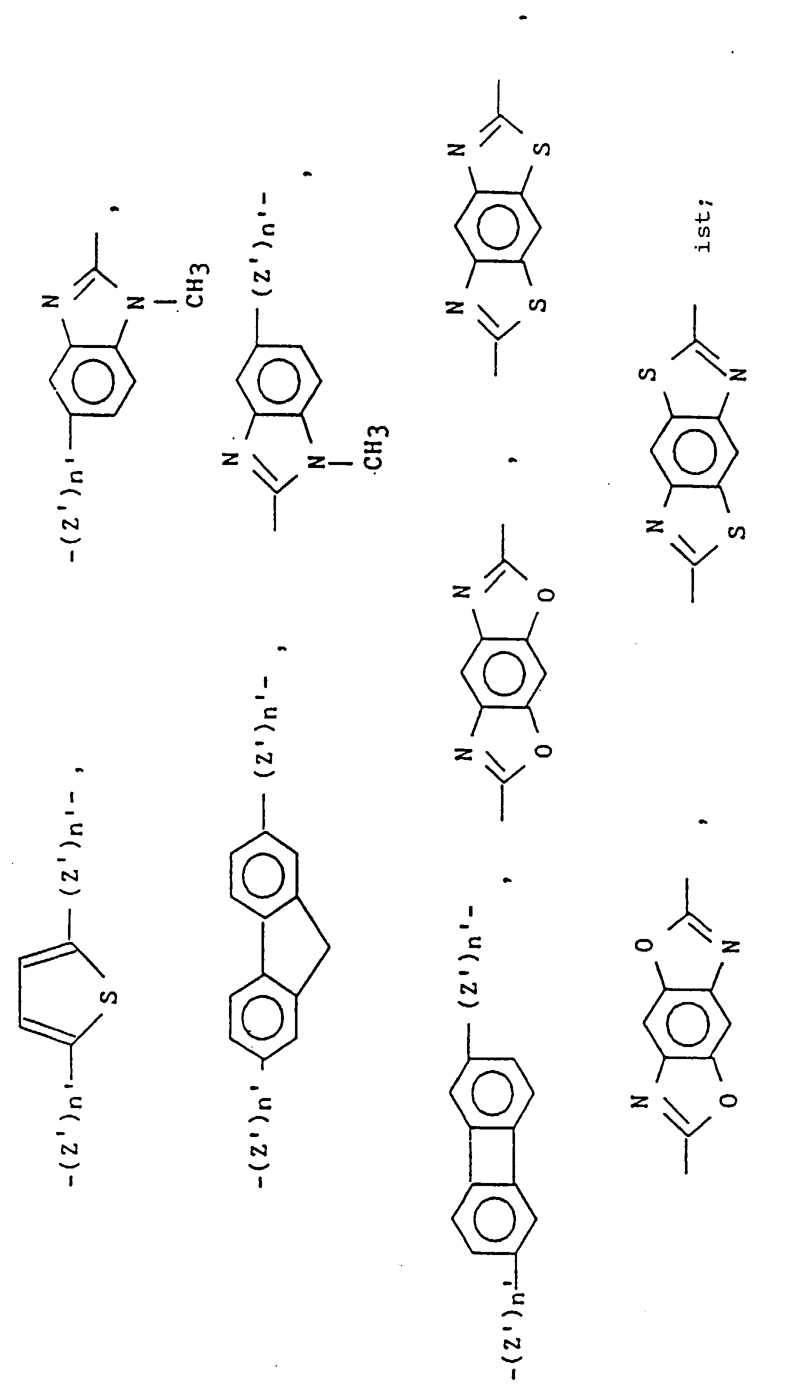 Z² eine Gruppe ist, die durch ein cyclisches oder bicyclisches Ringsystem, das 5 bis 12 Kohlenstoffatome enthält und cycloaliphatisch, polycycloaliphatisch, aromatisch oder eine Kombination hiervon sein kann, wiedergegeben wird;
Z³ 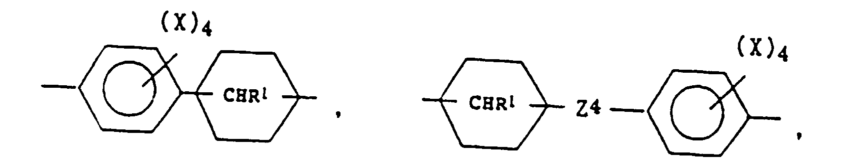 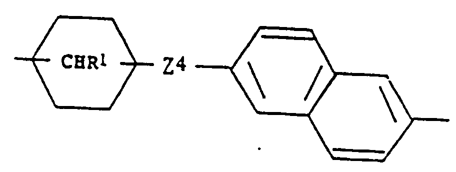 oder 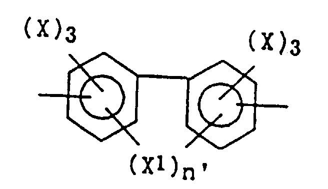 ist ;
Z⁴ = -CO-O-, -O-CO-, -NR¹-CO- oder -CO-NR¹- ist;
Z⁶ 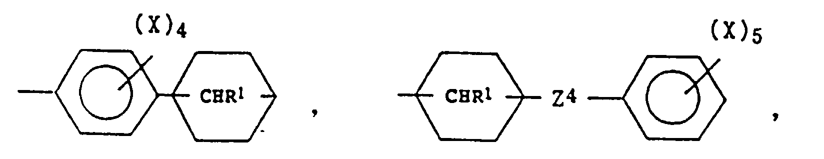 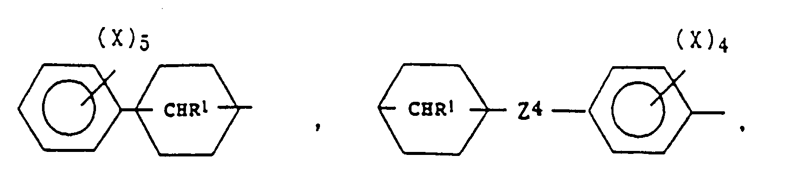 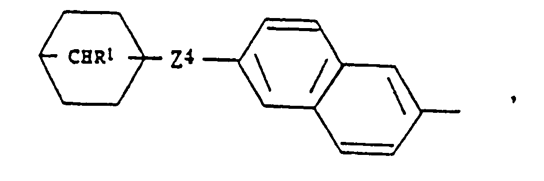 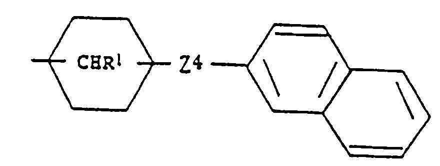 oder 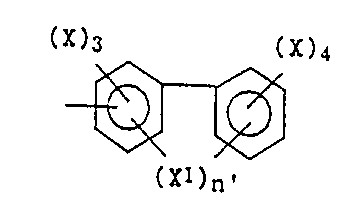 ist ;
n = 0 bis 2 ist; n' = null oder 1 ist; p' = 1 oder 2 ist; p" einen Wert von null bis 100 besitzt; jedes Z' unabhängig eine -CO-, -O-CO-, -CO-O-, -CO-NR¹- oder -NR¹-CO-Gruppe ist, mit der Maßgabe, daß das Polyglycidylamin der Formel II nicht das Tetraglycidylamin von 4,4'-Diaminobiphenyl (R = H, X = H, n = 0, p" = 0, Z¹ = eine direkte Einzelbindung) oder das Tetraglycidylamin von 3,3'-Dimethyl-4,4'-diaminobiphenyl (R = H, X in der 3,3'-Stellung = -CH₃ und H in allen anderen Stellungen, n = 0, p" = 0, Z¹ = eine direkte Einzelbindung) ist, und daß in Formel II n und p" zugleich nicht null sind.

2. Polyglycidylaminzusammensetzung, umfassend ein Diglycidylamin von N,N'-Dimethyl-4,4'-diaminostilben, N,N'-Dimethyl-3,3'-dimethyl-4,4'-diaminobiphenyl oder eine beliebige Kombination hiervon.

3. Verlängerte Polyglycidylaminzusammensetzung, hergestellt durch Umsetzung von:
(a) einem oder mehreren Polyglycidylaminen, die eine oder mehrere mesogene Einheiten enthalten, wobei diese Polyglycidylamine solche durch die Formeln I - VI wiedergegebenen sind, mit der Maßgabe, daß das Polyglycidylamin der Formel II das Tetraglycidylamin von 4,4'-Diaminobiphenyl oder das Tetraglycidylamin von 3,3-Dimethyl-4,4'-diaminobiphenyl einschließen kann, mit
(b) wenigstens einer Verbindung, die einen Durchschnitt von mehr als einem aktiven Wasserstoffatom pro Molekül aufweist,
worin die Komponenten (a) und (b) in Mengen verwendet sind, welche ein Verhältnis von aktiven Wasserstoffatomen pro Epoxidgruppe von 0,01:1 bis 0,95:1 ergeben, mit der Maßgabe, daß, falls die Komponente (a) Polyglycidylaminverbindungen mit einem Durchschnitt von mehr als zwei Glycidylgruppen pro Molekül enthält, solche Verbindungen in einer Menge vorhanden sind, daß weniger als 15 % der Glycidylgruppen durch die Verbindungen geliefert werden, welche einen Durchschnitt von mehr als zwei Glycidylgruppen pro Molekül enthalten.

4. Verlängerte Polyglycidylaminzusammensetzung nach Anspruch 3, worin:
(a) wenn die Komponente (A) ein durch die Formeln I oder III wiedergegebenes Polyglycidylamin ist, R' von 1 bis 6 Kohlenstoffatome aufweist, und wenn X eine Hydrocarbyl- oder Hydrocarbyloxygruppe ist, sie von 1 bis 6 Kohlenstoffatome aufweist, und wenn X ein Halogenatom ist, es Chlor oder Brom ist;
(b) wenn die Komponente (A) ein durch die Formeln II, IV, V oder VI wiedergegebenes Polyglycidylamin ist, und wenn X eine Hydrocarbyl- oder Hydrocarbyloxygruppe ist, sie von 1 bis 6 Kohlenstoffatomen aufweist, und wenn X ein Halogenatom ist, es Chlor oder Brom ist;
(c) Komponente (B) eine Verbindung ist, welche durch die folgenden Formeln XXV und XXVI wiedergegeben wird: 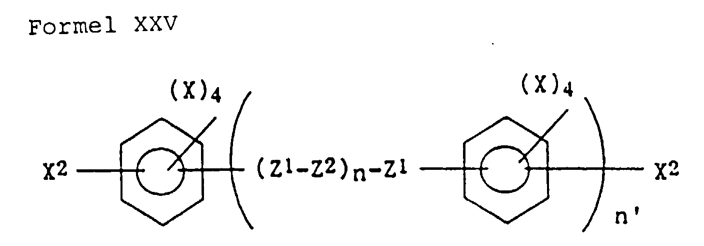 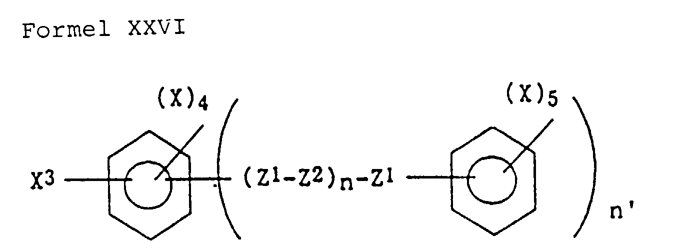 worin X² unabhängig eine Hydroxyl-, Carbonsäure-, -SH- oder -NHR-Gruppe ist; jedes R' unabhängig eine Hydrocarbylgruppe mit 1 bis 12 Kohlenstoffatomen ist; X³ = -NH₂, NH₂-SO₂-, NH₂-CO- oder NH₂-Z⁵-O- ist; Z⁵ eine Alkyl- oder Cycloalkylgruppe mit 1 bis 12 Kohlenstoffatomen ist; Z¹ unabhängig eine zweiwertige Hydrocarbylgruppe mit 1 bis 10 Kohlenstoffatomen, -O-, -CO-, -SO-, -SO₂-, -S-, -S-S- -CR¹=CR¹-, -CR¹=CR¹-CR¹=CR¹-, -CR¹=N-N=CR¹-, -CR¹=CR¹-CO-O-(CHR¹)_{p'}-, -CR¹=CR¹-O-CO-(CHR¹)_{p'}-, -(CHR¹)_{p'}-O-CO-CR¹=CR¹-,-(CHR¹)_{p'}-CO-O-CR¹=CR¹-, -CR¹=CR¹-CO-O-, -O-CO-CR¹=CR¹-, -CO-NR¹-, -NR¹-CO-, -CO-NR¹-NR¹-CO-, -C≡C-, -C≡C-C≡C-, -CO-S-, -S-CO-, -CR¹=N-, -N=CR¹-, -O-CO-, -CO-O-, -CR¹=CR¹-CO-, -CO-CR¹=CR¹-, -CR¹=CR¹-O-CO-, -CO-O-CR¹=CR¹-, -CH₂-CH₂-CO-O-, -O-CO-CH₂-CH₂-, -N=N-, eine direkte Einzelbindung, 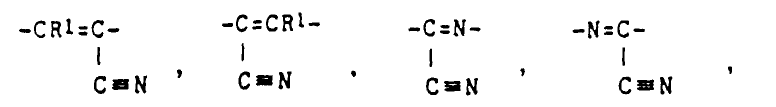 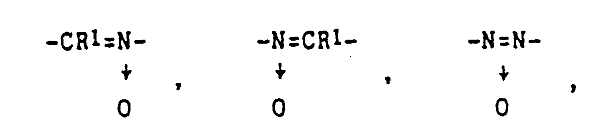 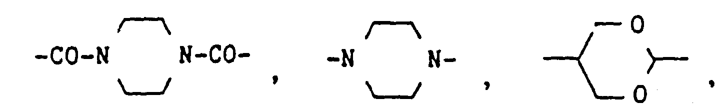 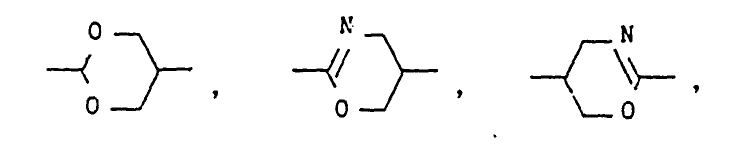 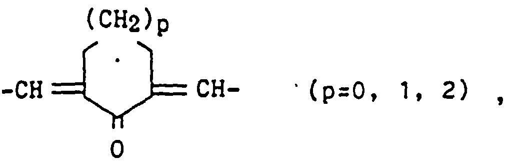 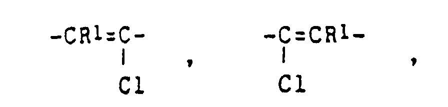 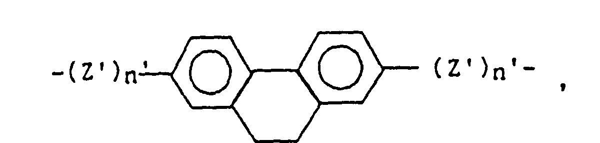 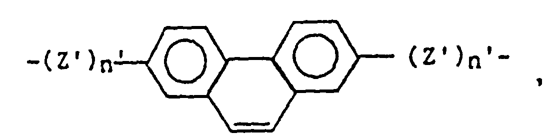 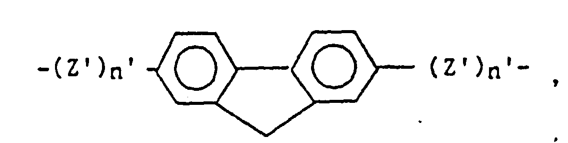 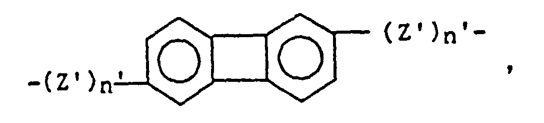 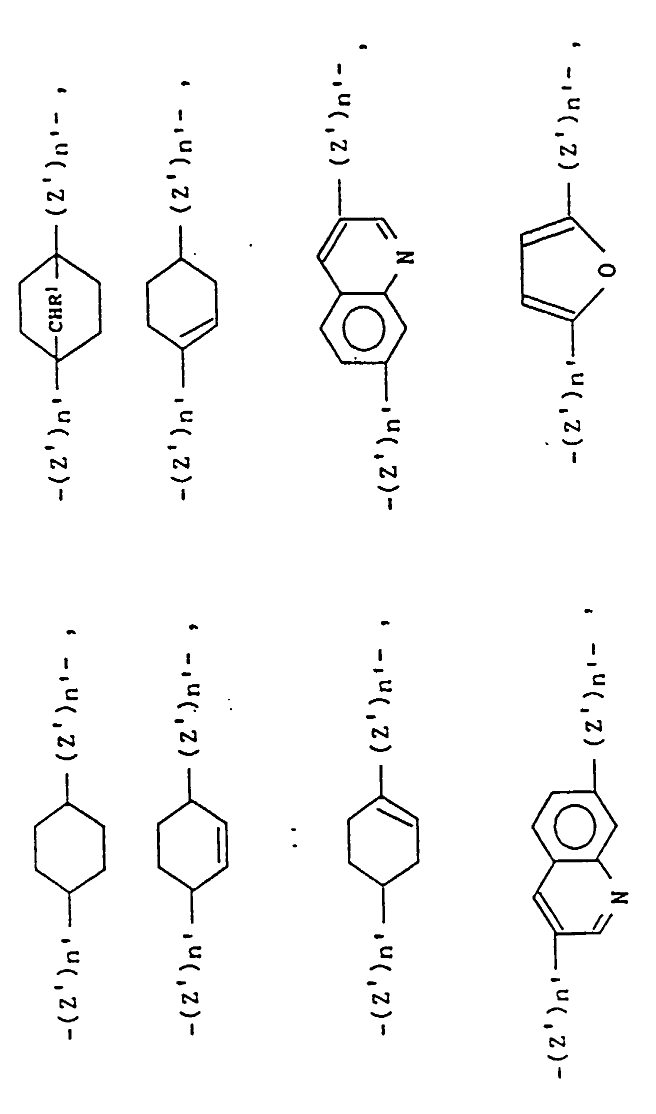 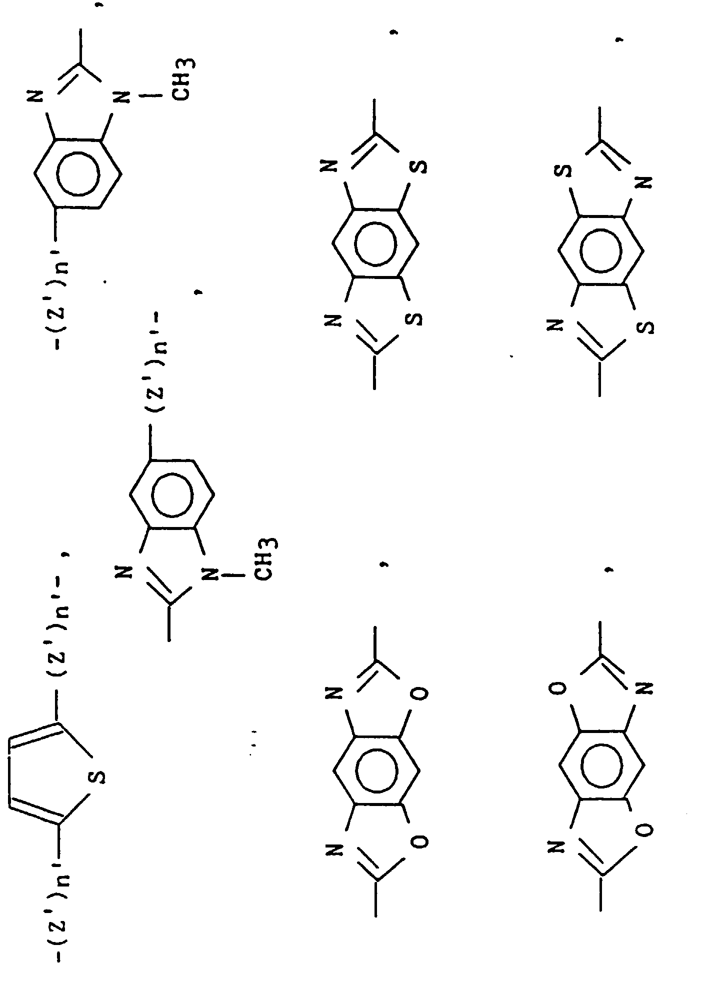 ist,
und jedes X unabhängig Wasserstoff, eine Hydrocarbyl- oder Hydrocarbyloxygruppe mit 1 bis 12 Kohlenstoffatomen, ein Halogenatom, -NO₂ oder -C≡N ist; Z² eine Gruppe ist, die durch ein cyclisches oder bicyclisches Ringsystem, das von 5 bis 12 Kohlenstoffatomen enthält und cycloaliphatisch, polycycloaliphatisch, aromatisch oder eine Kombination hiervon sein kann, wiedergegeben wird; n = null bis 2 ist; jedes Z' unabhängig eine -CO-, -O-CO-, -CO-O-, -CO-NR¹- oder -NR¹-CO-Gruppe ist; jedes R¹ unabhängig Wasserstoff oder eine aliphatische Kohlenwasserstoffgruppe mit 1 bis 4 Kohlenstoffatomen ist und jedes n' unabhängig einen Wert von null oder eins hat; oder eine beliebige Kombination von beliebigen zwei oder mehr Verbindungen, welche durch die zuvorgenannten Formeln XXV und XXVI wiedergegeben werden; und
(d) die Komponenten (A) und (B) in Mengen verwendet sind, welche ein Verhältnis von aktiven Wasserstoffatomen pro Epoxidgruppe von 0,05:1 bis 0,8:1 liefern; und
mit der Maßgabe, daß, falls die Komponente (A) eine Polyglycidylaminverbindung mit einem Durchschnitt von mehr als zwei Glycidylamingruppen pro Molekül enthält, nicht mehr als 10 % der Gesamtmenge der in Komponente (A) enthaltenen Glycidylgruppen durch diese Polyglycidylaminverbindung, welche einen Durchschnitt von mehr als zwei Glycidylamingruppen pro Molekül enthält, geliefert werden.

5. Verlängertes Polyglycidylamin nach Anspruch 3 oder 4, worin:
(a) Komponente (B) ist: Hydrochinon, Bisphenol-A, 4,4'-Dihydroxydiphenylmethan, 4,4'-Thiodiphenol, 4,4'-Sulfonyldiphenol, 4,4'-Dihydroxydiphenyloxid, 4,4'-Dihydroxybenzophenon, 1,1-Bis (4-hydroxyphenyl)-1-phenylethan, 3,3',5,5'-Tetrachlorbisphenol-A, 3,3'-Dimethoxybisphenol-A, 4,4'-Dihydroxybiphenyl, 4,4'-Dihydroxy-α,α'-diethylstilben, 4,4'-Dihydroxy-α-methylstilben, 4,4'-Dihydroxybenzanilid, 4,4'-Dihydroxy-2,2'-dimethylazoxybenzol, 4,4'-Dihydroxy-α-cyanostilben, Bis(4-hydroxyphenyl)terephthalat, N,N'-Bis-(4-hydroxyphenyl)terephthalamid, Bis(4'-hydroxybiphenyl)terephthalat, 4,4'-Dihydroxyphenylbenzoat, Bis(4'-hydroxyphenyl)-1,4-benzoldiimin, 4,4"-Dihydroxybiphenylbenzoat, 1,4-Bis(4'-hydroxyphenyl-1'-carboxamid)benzol, 1,4-Bis(4'-hydroxyphenyl-l'-carboxy)benzol, 4,4'-Bis (4"-hydroxyphenyl-1"-carboxy)biphenyl, Terephthalsäure, 4,4'-Benzaniliddicarbonsäure, 4,4'-Phenylbenzoatdicarbonsäure, 4,4'-Stilbendicarbonsäure, 4,4'-Dicarboxybiphenyl, 4,4'-Dicarboxydiphenylazomethin, Anilin, 4'-Sulfonamido-N-phenylbenzamid, 4'-Sulfonamido-N'-phenyl-4-chlorbenzamid, 4-Amino-1-phenylbenzoat, 4-Amino-N-phenylbenzamid, N-Phenyl-4-aminophenyl-1-carboxamid, Phenyl-4-aminobenzoat, Biphenyl-4-aminobenzoat, l-Phenyl-4'-aminophenylterephthalat, N,N'-Dimethyl-4,4'-diaminodiphenylmethan, N,N'-Diethyl-4,4'-diaminodiphenylmethan, N,N'-Dimethyl-4,4'-diaminobiphenyl, N,N'-Dimethyl-3,3'-dimethyl-4,4'-diaminobiphenyl, N,N'-Dimethyl-4,4'-diaminostilben, N,N'-Dimethylamino-4,4'-diaminodiphenylsulfon, N,N'-Dimethyl-4,4'-diaminobenzanilid, N,N'-Dimethyl-4,4'-diamino-N-methylbenzanilid und jede beliebige Kombination von beliebig zwei oder mehreren solcher Verbindungen; und
(b) die Komponenten (A) und (B) in Mengen verwendet sind, welche ein Verhältnis von aktiven Wasserstoffatomen pro Epoxidgruppe von 0,1:1 bis 0,5:1 liefern; und
mit der Maßgabe, daß, falls die Komponente (A) eine Polyglycidylaminverbindung mit einem Durchschnitt von mehr als zwei Glycidylgruppen pro Molekül enthält, nicht mehr als 5 % der Gesamtmenge der in Komponente (A) enthaltenen Glycidylgruppen durch diese Polyglycidylaminverbindung, welche einen Durchschnitt von mehr als zwei Glycidylgruppen pro Molekül enthält, geliefert werden.

6. Mischung, umfassend:
(A) eine oder mehrere Polyglycidylamin- oder Monoglycidylaminverbindungen, welche eine oder mehrere mesogene durch die Formeln I - VIII wiedergegebene Einheiten enthalten, mit der Maßgabe, daß das Polyglycidylamin der Formel II das Tetraglycidylamin von 4,4'-Diaminobiphenyl oder das Tetraglycidylamin von 3,3'-Dimethyl-4,4'-diaminobiphenyl einschließen kann; und
(B) eine oder mehrere Polyepoxidverbindungen, welche im wesentlichen frei von mesogenen Einheiten sind.

7. Mischung nach Anspruch 6, worin:
(a) wenn die Komponente (A) ein durch die Formeln I oder III wiedergegebenes Polyglycidylamin ist, R' von 1 bis 6 Kohlenstoffatome aufweist, und wenn X eine Hydrocarbyl- oder Hydrocarbyloxygruppe ist, sie von 1 bis 6 Kohlenstoffatome aufweist, und wenn X ein Halogenatom ist, es Chlor oder Brom ist;
(b) wenn die Komponente (A) ein durch die Formeln II, IV, V oder VI wiedergegebenes Polyglycidylamin ist, und wenn X eine Hydrocarbyl- oder Hydrocarbyloxygruppe ist, sie von 1 bis 6 Kohlenstoffatomen aufweist, und wenn X ein Halogenatom ist, es Chlor oder Brom ist;
(c) wenn die Komponente (A) ein durch die Formeln VII und VIII wiedergegebenes Monoglycidylamin ist, R' von 1 bis 6 Kohlenstoffatome aufweist, und wenn X eine Hydrocarbyl- oder Hydrocarbyloxygruppe ist, sie von 1 bis 6 Kohlenstoffatome aufweist, und wenn X ein Halogenatom ist, es Chlor oder Brom ist;
(d) die Komponente (B) ein Epoxyharz ist, das durch die folgenden Formeln IX - XV wiedergegeben wird: 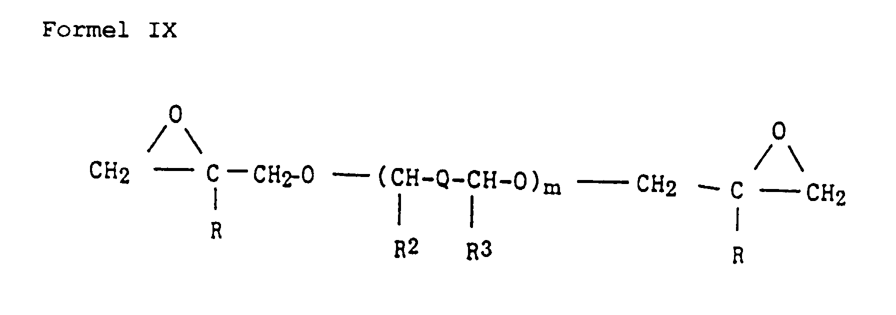 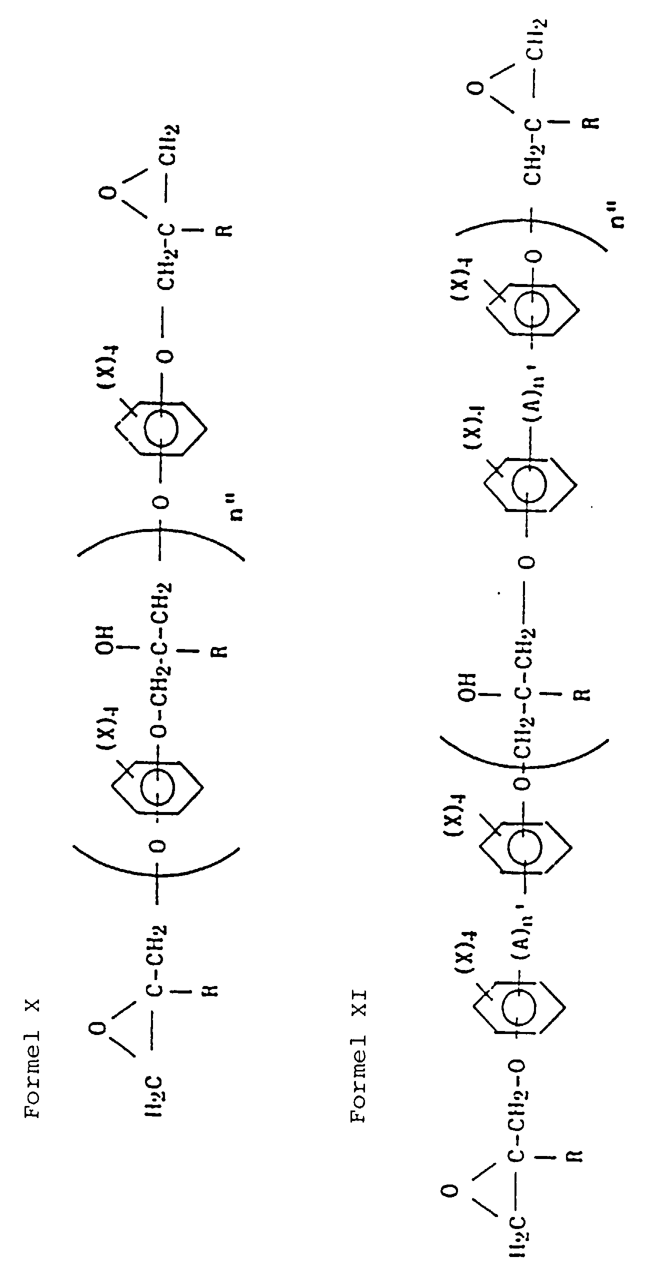 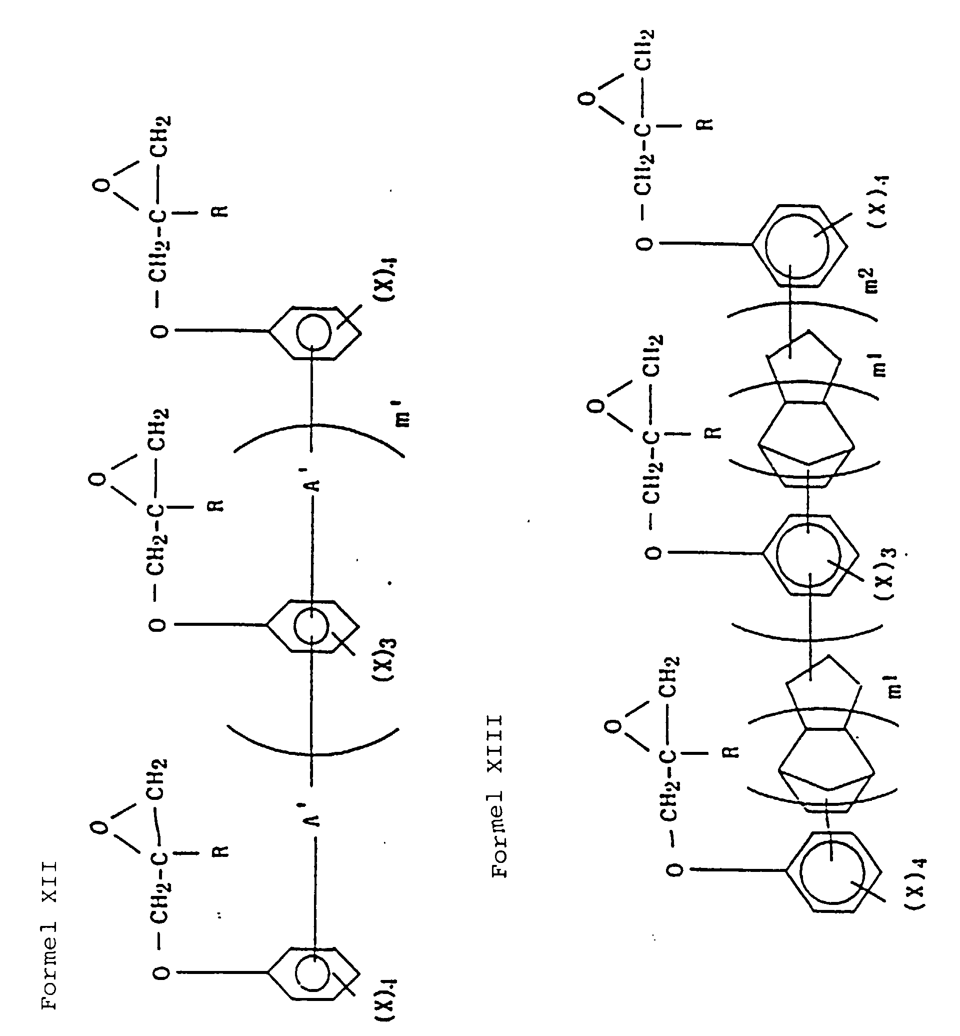 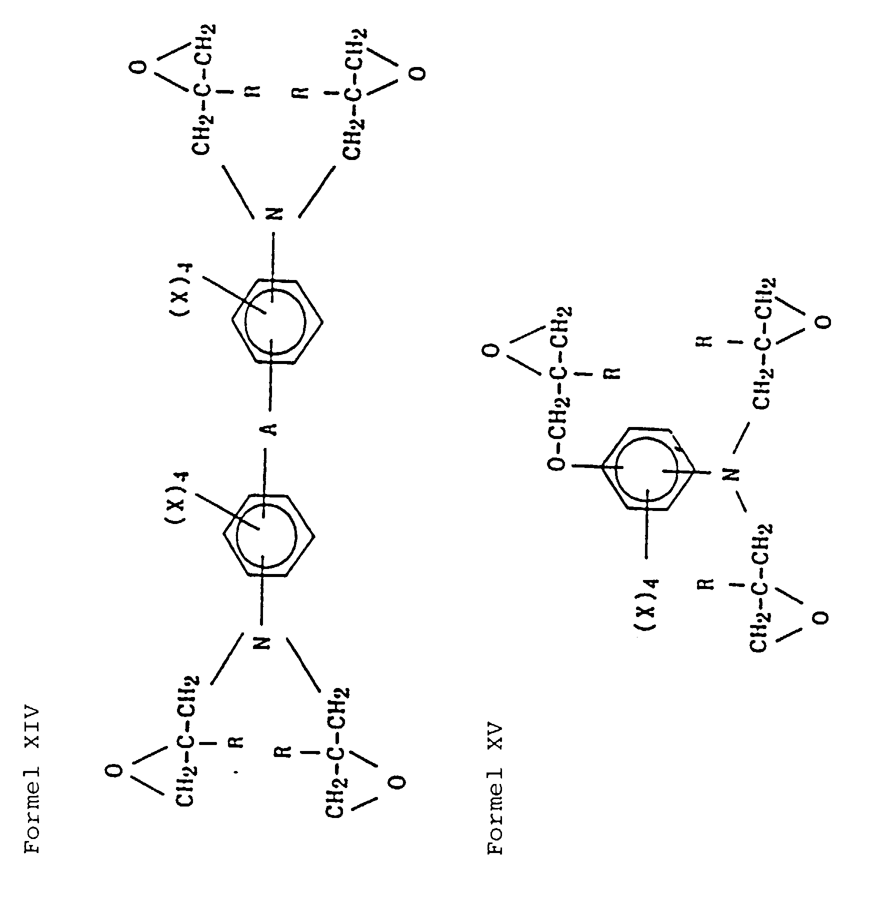 worin jedes A unabhängig eine zweiwertige Hydrocarbylgruppe mit 1 bis 12 Kohlenstoffatomen, -O-, -S-, -S-S-, -SO-, -SO₂- oder -CO- ist; jedes A' unabhängig eine zweiwertige Kohlenwasserstoffgruppe mit 1 bis 6 Kohlenstoffatomen ist; Q eine Einzelbindung, -CH₂-S-CH₂-, -(CH₂)ₙ¹ oder 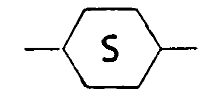 ist;
jedes R unabhängig Wasserstoff oder eine Alkylgruppe mit 1 bis 4 Kohlenstoffatomen ist; jedes R² oder R³ unabhängig Wasserstoff, eine Hydrocarbyl- oder Halogenhydrocarbylgruppe mit 1 bis 6 Kohlenstoffatomen ist, jedes X unabhängig Wasserstoff, eine Hydrocarbyl- oder Hydrocarbyloxygruppe mit 1 bis 12 Kohlenstoffatomen, ein Halogenatom, -NO₂ oder -C≡N ist; m einen Wert von 1 bis 10 hat; m' einen Durchschnittswert von 0,01 bis 12 hat; m¹ einen Durchschnittswert von 1 bis 12 hat; m² einen Wert von 1 bis 12 hat; n' einen Wert von null oder 1 hat; n" einen Durchschnittswert von null bis 3 hat und n¹ einen Durchschnittswert von 1 bis 10 hat; und
(e) die Komponente (A) in einer Menge von 1 bis 99 Gew.-%, bezogen auf die kombinierten Mengen der Komponenten (A) und (B), vorhanden ist, und die Komponente (B) in einer Menge von 99 bis 1 Gew.-%, bezogen auf die kombinierten Mengen der Komponenten (A) und (B), vorhanden ist.

8. Mischung nach Anspruch 7, worin die Komponente (A) ein Diglycidylamin von N,N'-Dimethyl-4,4'-diaminostilben, N,N'-Dimethyl-4,4'-diaminobiphenyl oder eine Kombination hiervon ist, und die Komponente (B) ein Diglycidylether von Bisphenol-A, ein Diglycidylether von 4,4'-Dihydroxydiphenylmethan, ein Diglycidylether von 4,4'-Dihydroxybenzophenon, ein Diglycidylether von 4,4'-Dihydroxythiodiphenol, ein Diglycidylether von 1,1,-Bis-(4-hydroxyphenyl)-1-phenylethan (Bisphenol-AP) oder eine beliebige Kombination hiervon ist.

9. Mischung, umfassend:
(A) eine verlängerte Polyglycidylaminzusammensetzung, hergestellt durch Umsetzung von:
(1) einem oder mehreren Polyglycidylaminen, welche eine oder mehrere mesogene Einheiten enthalten, wobei diese Polyglycidylamine solche durch die Formeln I - VI wiedergegebenen sind, mit der Maßgabe, daß das Polyglycidylamin der Formel II das Tetraglycidylamin von 4,4'-Diaminobiphenyl oder das Tetraglycidylamin von 3,3'-Dimethyl-4,4'-diaminobiphenyl einschließen kann; mit
(2) wenigstens einer Verbindung, welche einen Durchschnitt von mehr als einem aktiven Wasserstoffatom pro Molekül hat; und
worin die Komponenten (1) und (2) in Mengen verwendet sind, welche ein Verhältnis von aktiven Wasserstoffatomen pro Epoxidgruppe von 0,01:1 bis 0,95:1 ergeben, mit der Maßgabe, daß, falls die Komponente (A1) eine Polyglycidylaminverbindung mit einem Durchschnitt von mehr als zwei Glycidylamingruppen pro Molekül enthält, solche Verbindungen in einer Menge vorhanden sind, daß nicht mehr als 15 % der in Komponente (A1) enthaltenen Glycidylamingruppen durch diese Polyglycidylaminverbindung angeliefert werden, welche einen Durchschnitt von mehr als zwei Glycidylgruppen pro Molekül enthält, und
(B) ein oder mehrere Polyepoxide, welche frei von mesogenen Einheiten sind.

10. Mischung nach Anspruch 9, in welcher
(a) Komponente (2) ist: Hydrochinon, Bisphenol-A, 4,4'-Dihydroxydiphenylmethan, 4,4'-Thiodiphenol, 4,4'-Sulfonyldiphenol, 4,4'-Dihydroxydiphenyloxid, 4,4'-Dihydroxybenzophenon, 1,1-Bis (4-hydroxyphenyl)-1-phenylethan, 3,3',5,5'-Tetrachlorbisphenol-A, 3,3'-Dimethoxybisphenol-A, 4,4'-Dihydroxybiphenyl, 4,4'-Dihydroxy-α,α'-diethylstilben, 4,4'-Dihydroxy-α-methylstilben, 4,4'-Dihydroxybenzanilide, 4,4'-Dihydroxy-2,2'-dimethylazoxybenzol, 4,4'-Dihydroxy-α-cyanostilben, Bis(4-hydroxyphenyl)terephthalat, N,N'-Bis-(4-hydroxyphenyl)terephthalamid, Bis(4'-hydroxybiphenyl)terephthalat, 4,4'-Dihydroxyphenylbenzoat, Bis(4'-hydroxyphenyl)-1,4-benzoldiimin, 4,4"-Dihydroxybiphenylbenzoat, 1,4-Bis(4'-hydroxyphenyl-1'-carboxamid)benzol, 1,4-Bis(4'-hydroxyphenyl-1'-carboxy)benzol, 4,4'-Bis (4"-hydroxyphenyl-1"-carboxy)biphenyl, Terephthalsäure, 4,4'-Benzaniliddicarbonsäure, 4,4'-Phenylbenzoatdicarbonsäure, 4,4'-Stilbendicarbonsäure, 4,4'-Dicarboxybiphenyl, 4,4'-Dicarboxydiphenylazomethin, Anilin, 4'-Sulfonamido-N-phenylbenzamid, 4'-Sulfonamido-N'-phenyl-4-chlorbenzamid, 4-Amino-1-phenylbenzoat, 4-Amino-N-phenylbenzamid, N-Phenyl-4-aminophenyl-1-carboxamid, Phenyl-4-aminobenzoat, Biphenyl-4-aminobenzoat, 1-Phenyl-4'-aminophenyl terephthalat, N,N'-Dimethyl-4,4'-diaminodiphenylmethan, N,N'-Diethyl-4,4'-diaminodiphenylmethan, N,N'-Dimethyl-4,4'-diaminobiphenyl, N,N'-Dimethyl-3,3'-dimethyl-4,4'-diaminobiphenyl, N,N'-Dimethyl-4,4'-diaminostilben, N,N'-Dimethylamino-4,4'-diaminodiphenylsulfon, N,N'-Dimethyl-4,4'-diaminobenzanilid, N,N'-Dimethyl-4,4'-diamino-N-methylbenzanilid und jede beliebige Kombination on beliebig zwei oder mehreren solcher Verbindungen; und
(b) die Komponenten (1) und (2) in Mengen verwendet sind, welche ein Verhältnis von aktiven Wasserstoffatomen pro Epoxidgruppe von 0,1:1 bis 0,5:1 liefern;
(c) die Komponente (A) in einer Menge von 10 bis 50 Gew.-%, bezogen auf die kombinierten Mengen von Verbindungen (A) und (B), vorhanden ist; und
(d) die Komponente (B) in einer Menge von 90 bis 50 Gew.-%, bezogen auf die kombinierten Mengen von Verbindungen (A) und (B), vorhanden ist.

11. Mischung, umfassend:
(A) ein oder mehrere Polyglycidylamine entsprechend einer oder mehreren der Formeln I bis VI von Anspruch 1, enthaltend einen Durchschnitt von mehr als einer vicinalen Epoxidgruppe pro Molekül und eine oder mehrere mesogene Einheiten pro Molekül; und
(B) ein oder mehrere Monoglycidylamine entsprechend einer oder beiden der Formeln VII und VIII von Anspruch 1, enthaltend nur eine vicinale Epoxidgruppe pro Molekül und eine oder mehrere mesogene Einheiten pro Molekül.

12. Mischung nach Anspruch 11, in welcher Komponente (A) in einer Menge von 10 bis 50 Gew.-%, bezogen auf das kombinierte Gewicht der Komponenten (A) und (B), vorhanden ist, und Komponente (B) in einer Menge von 90 bis 50 Gew.-%, bezogen auf das kombinierte Gewicht der Komponenten (A) und (B), vorhanden ist.

13. Aushärtbare Zusammensetzung, umfassend wenigstens eine Zusammensetzung von Anspruch 1, 2, 3, 4, 5, 6, 7, 8, 9, 10, 11 oder 12 und eine aushärtende Menge eines Härters hierfür.

14. Produkt, resultierend aus dem Aushärten der aushärtbaren Zusammensetzung von Anspruch 13.

15. Produkt, resultierend aus der Orientierung einer aushärtbaren Zusammensetzung von Anspruch 14 vor dem Aushärten, während des Aushärtens oder sowohl vor als auch während des Aushärtens.

16. Produkt von Anspruch 15, in welchem diese Orientierung mittels eines elektrischen oder magnetischen Feldes oder durch Anwendung von Zieh- oder Scherkräften herbeigeführt worden ist.

17. Harz vom Phenoxytyp, hergestellt durch Umsetzung von:
(A) einem oder mehreren Polyglycidylaminen, enthaltend eine oder mehrere mesogene Einheiten, wobei diese Polyglycidylamine solche sind, die durch die Formeln I, III, V oder VI wiedergegeben werden;
(B) wenigstens einer Verbindung, welche einen Durchschnitt von mehr als einem aktiven Wasserstoffatom pro Molekül aufweist, worin die Komponenten (A) und (B) in Mengen verwendet sind, welche ein Verhältnis von aktiven Wasserstoffatomen pro Epoxidgruppe von 0,96:1 bis 1,05:1 ergeben.

## Revendications

1. Composition contenant une glycidylamine comportant un ou plusieurs fragments mésogènes, représentée par l'une des formules suivantes I à VIII : 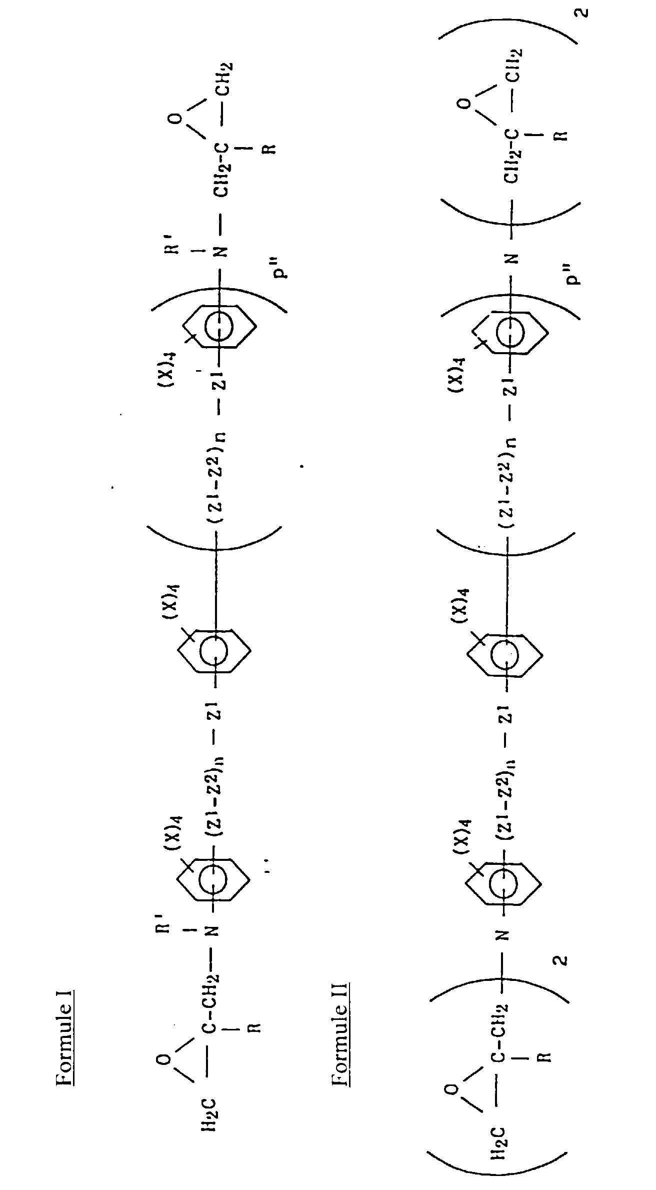 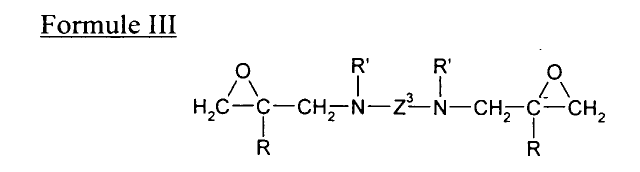 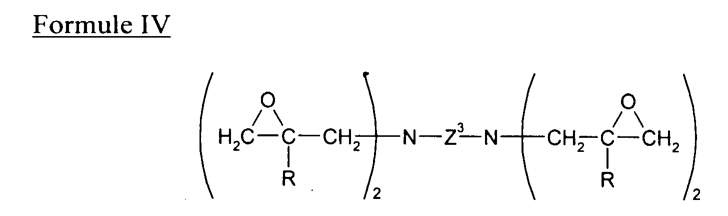 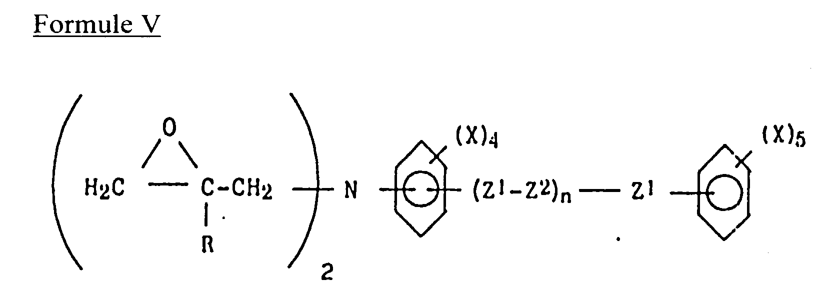 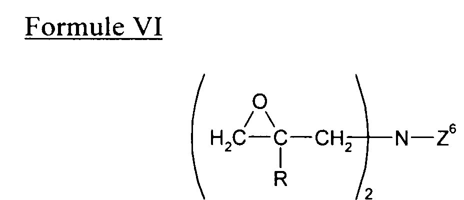 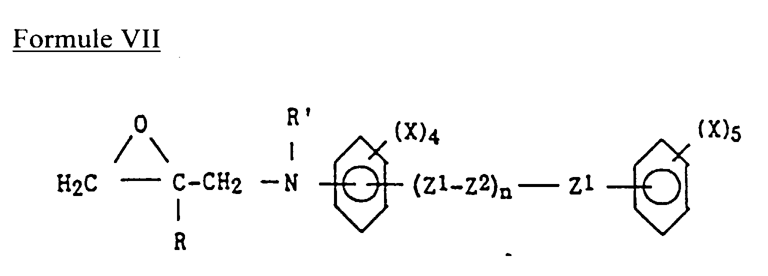 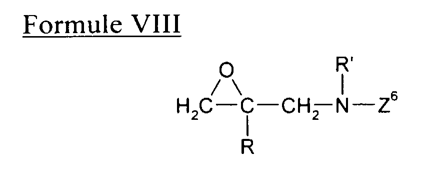 formules dans lesquelles :
au moins 80 % des enchaînements -(Z¹-Z²)ₙ-Z¹- et des groupes glycidylamine se trouvent en position para les uns par rapport aux autres ; chaque symbole R et R¹ représente indépendamment un atome d'hydrogène ou un groupe hydrocarboné aliphatique comportant de 1 à 4 atomes de carbone ;
chaque symbole R' représente indépendamment un groupe hydrocarbyle comportant de 1 à 12 atomes de carbone ;
chaque symbole X représente indépendamment un atome d'hydrogène, un groupe hydrocarbyle ou hydrocarbyloxy comportant avantageusement de 1 à 12 atomes de carbone, un atome d'halogène, ou un groupe -NO₂ ou -C≡N ;
X¹ représente un groupe hydrocarbyle comportant de 1 à 10 atomes de carbone, qui peut comporter un ou plusieurs hétéroatomes choisis parmi N, O et S, et qui peut être saturé ou insaturé ;
chaque symbole Z¹ représente indépendamment une liaison simple directe,
-CR¹=CR¹-, -CR¹=CR¹-CR¹=CR¹-, -CR¹=N-N=CR¹-, -CR¹=CR¹-CO-O-(CHR¹)_{p'}-, -CR¹=CR¹-O-CO-(CHR¹)_{p'}-, (CHR¹)_{p'}-O-CO-CR¹CR¹-, -(CHR¹)_{p'}-CO-O-CR¹=CR¹-, -CR¹=CR¹-CO-O-, -O-CO-CR¹=CR¹-, -CO-NR¹-, -NR¹-CO-, -CO-NR¹-NR¹-CO-, -C≡C-, -C≡C-C≡C-, -CO-S-, -S-CO-, -CR¹=N-, -N=CR¹-, -O-CO-, -CO-O-, -CR¹=CR¹-CO-, -CO-CR¹=CR¹-, -CR¹=CR¹-O-CO-, -CO-O-CR¹=CR¹-, -CH₂-CH₂-CO-O-, -O-CO-CH₂-CH₂-, -N=N-, 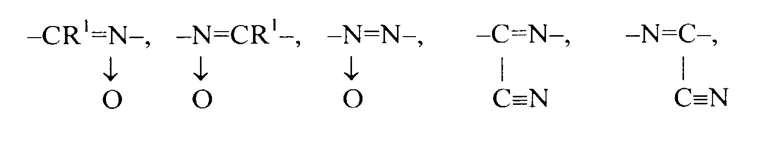 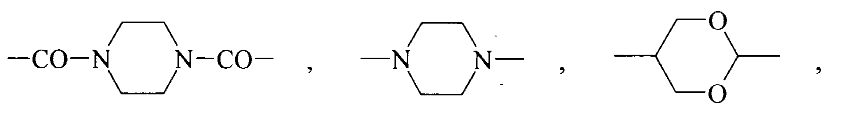 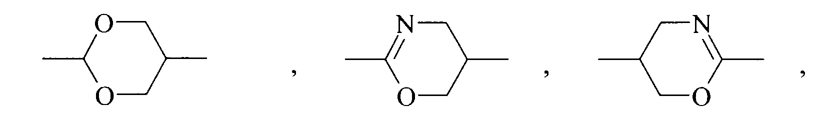 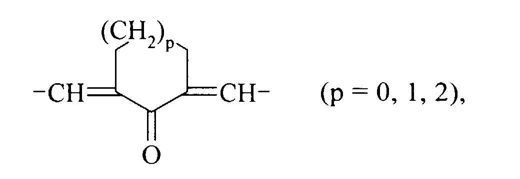 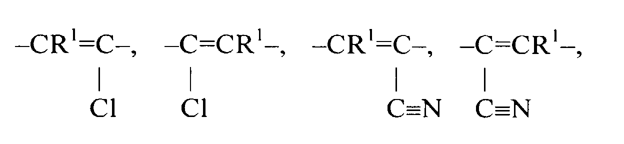 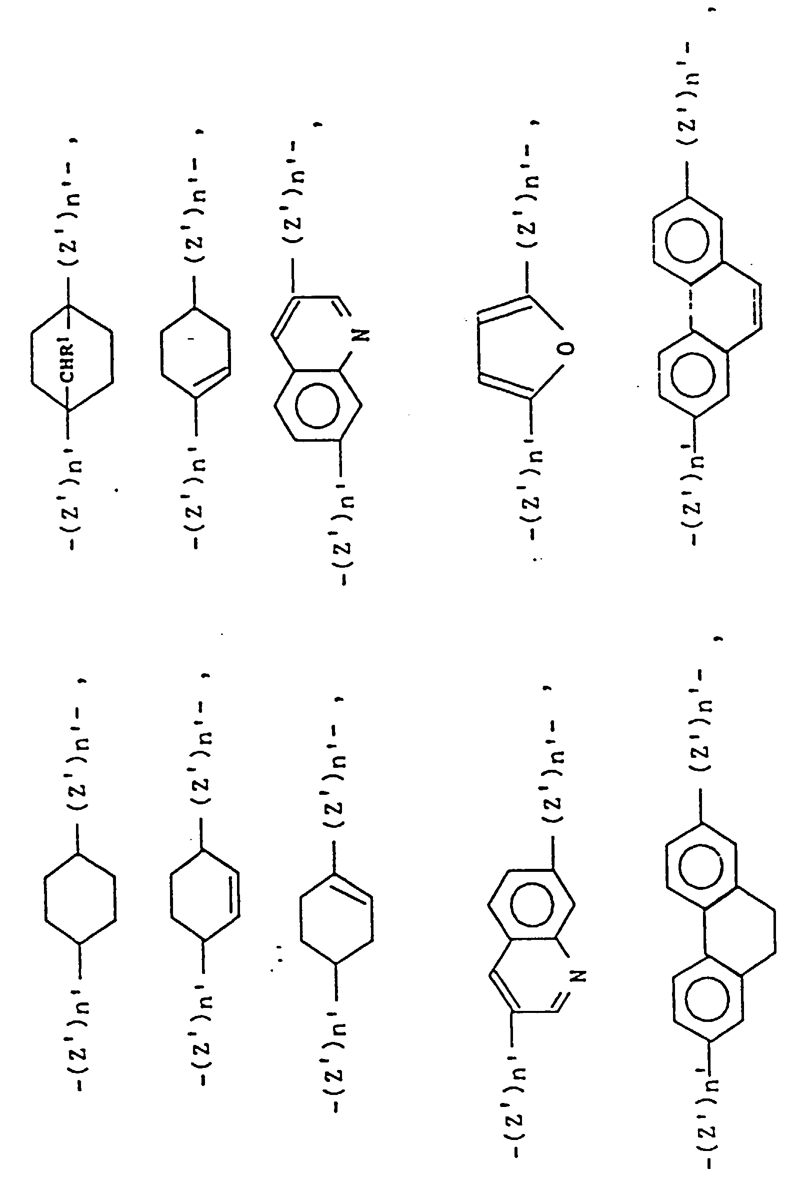 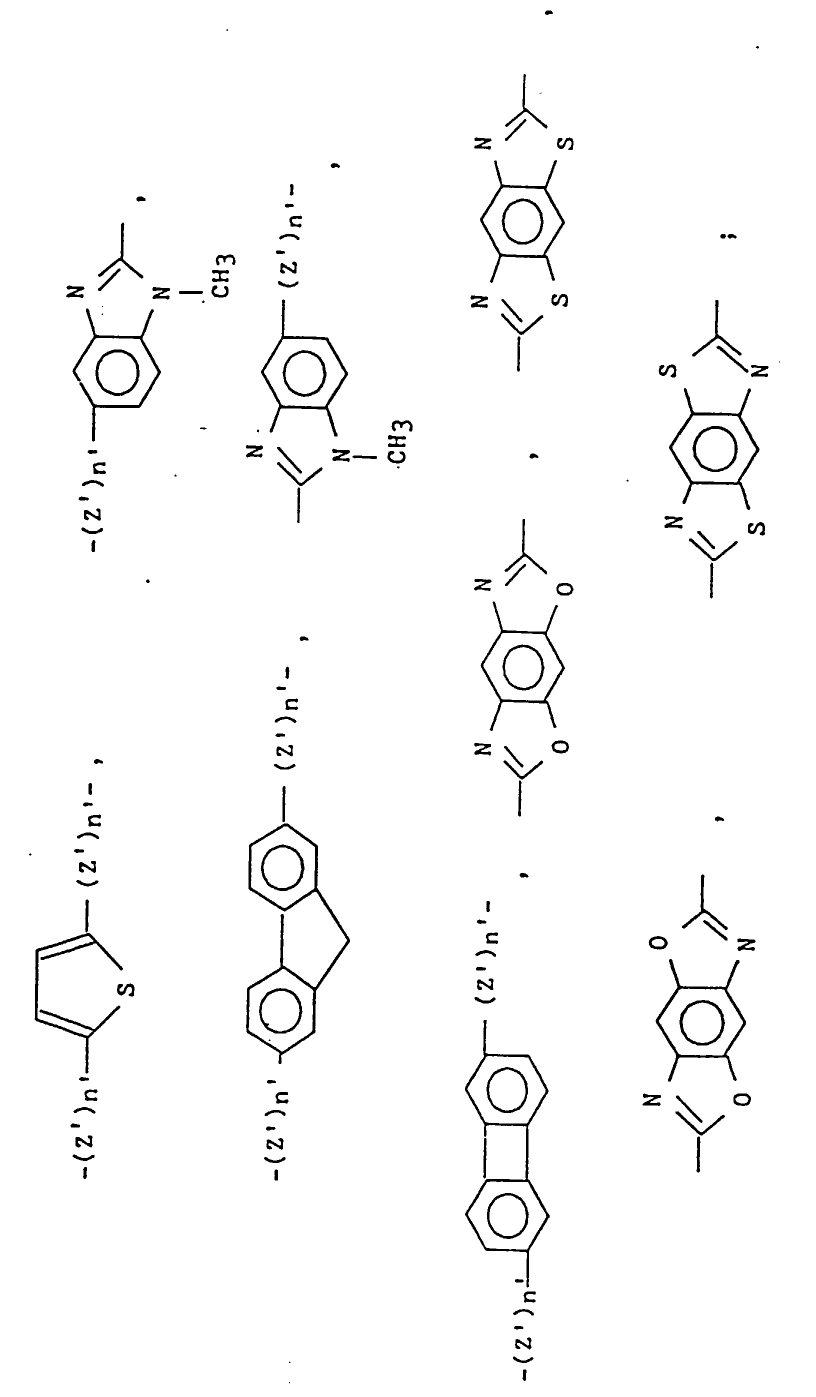
Z² représente un groupe qui est un système cyclique ou bicyclique comportant de 5 à 12 atomes de carbone et qui peut être de type cycloaliphatique, polycycloaliphatique, aromatique ou d'une combinaison de ces types ;
Z³ représente 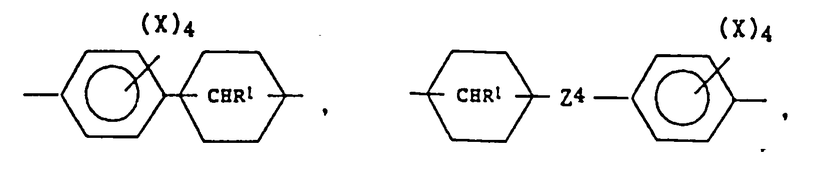 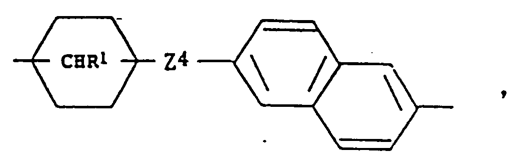 ou 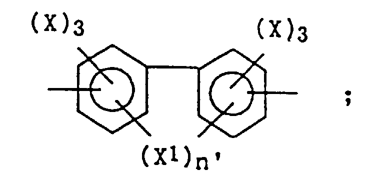
Z⁴ représente -CO-O-, -O-CO-, -NR¹-CO- ou -CO-NR¹ ;
Z⁶ représente : 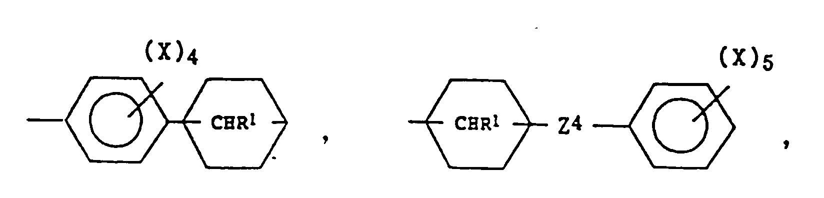 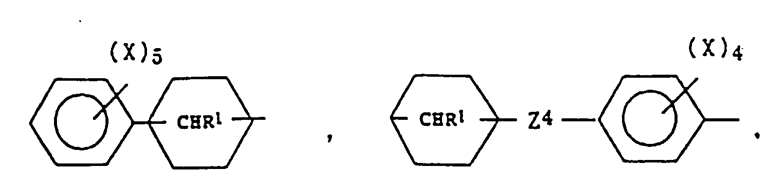 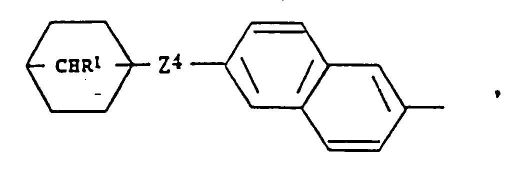 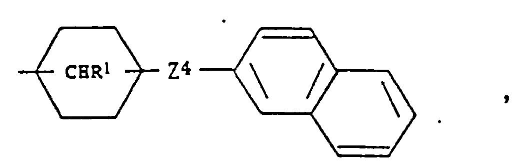 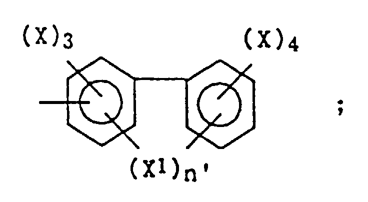
n vaut de O à 2 ;
n^{'} vaut 0 ou 1 ;
p' vaut 1 ou 2 ;
p" vaut de 0 à 100 ;
et chaque symbole Z' représente indépendamment un groupe -CO-, -O-CO-, -CO-O-, -CO-NR¹-, ou -NR¹-CO- ;
sous réserve que la polyglycidylamine de formule II ne soit pas la tétraglycidylamine dérivée du 4,4'-diaminobiphényle (où R représenterait H, X représenterait H, n vaudrait 0, p" vaudrait 0 et Z¹ représenterait une liaison simple directe), ni la tétraglycidylamine dérivée du 3,3'-diméthyl-4,4'-diaminobiphényle (où R représenterait H, X représenterait -CH₃ en position 3,3', et H dans toutes les autres positions, n vaudrait 0, p" vaudrait 0 et Z¹ représenterait une liaison simple directe), et que, dans la formule II, n et p" ne soient pas simultanément nuls.

2. Composition de polyglycidylamine comprenant une diglycidylamine dérivée du N,N'-diméthyl-4,4'-diaminostilbène, du N,N'-diméthyl-3,3'-diméthyl-4,4'-diaminobiphényle, ou de n'importe quelle combinaison de ceux-ci.

3. Composition de polyglycidylamine prépolymérisée, que l'on prépare en faisant réagir :
(A) une ou plusieurs polyglycidylamines comportant un ou plusieurs fragments mésogènes, lesdites polyglycidylamines étant de celles qui sont représentées par les formules I à VI, et la polyglycidylamine de formule II pouvant englober la tétraglycidylamine du 4,4'-diaminobiphényle ou la tétraglycidylamine du 3,3'-diméthyl-4,4'-diaminobiphényle, avec
(B) au moins un composé comportant en moyenne plus d'un atome d'hydrogène actif par molécule ;
les composants (A) et (B) étant utilisés en des quantités qui donnent un rapport du nombre d'atomes d'hydrogène actifs au nombre de groupes époxyde valant de 0,01/1 à 0,95/1 ;
sous réserve que, quand le composant (A) comporte des composés polyglycidylamines qui contiennent en moyenne plus de deux groupcs glycidyle par molécule, ces composés se trouvent en une quantité telle qu'il y ait moins de 15 % des groupes glycidyle qui proviennent de ces composés contenant en moyenne plus de deux groupes glycidyle par molécule.

4. Composition de polyglycidylamine prépolymérisée, conforme à la revendication 3, dans laquelle :
(a) si le composant (A) est une polyglycidylamine représentée par l'une des formules I et III, le groupe symbolisé par R' comporte de 1 à 6 atomes de carbone et, si X représente un groupe hydrocarbyle ou hydrocarbyloxy, celui-ci comporte de 1 à 6 atomes de carbone et, si X représente un atome d'halogène, il s'agit d'un atome de chlore ou de brome ;
(b) si le composant (A) est une polyglycidylamine représentée par l'une des formules II, IV, V et VI, et si X représente un groupe hydrocarbyle ou hydrocarbyloxy, celui-ci comporte de 1 à 6 atomes de carbone et, si X représente un atome d'halogène, il s'agit d'un atome de chlore ou de brome ;
(c) le composant (B) est un composé représenté par l'une des formules suivantes XXV et XXVI : 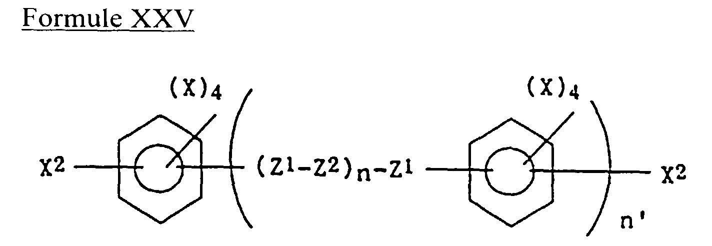 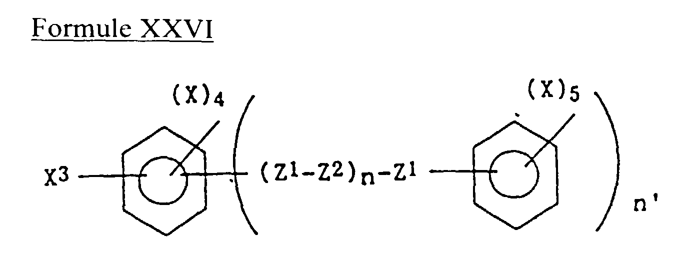 dans lesquelles
X² représente indépendamment un groupe hydroxy, carboxy, -SH ou-NHR', et chaque R' représente indépendamment un groupe hydrocarbyle comportant de 1 à 12 atomes de carbone ;
X³ représente -NH₂, NH₂-SO₂-, NH₂-CO- ou NH₂-Z⁵-O-, et Z⁵ représente un groupe alkyle ou cycloalkyle comportant de 1 à 12 atomes de carbone ;
Z¹ peut représenter, indépendamment, un groupe hydrocarbyle divalent comportant de 1 à 10 atomes de carbone, un chaînon -O-, -CO-, -SO-, -SO₂-, -S-, -S-S-, un groupe
-CR¹=CR¹-, -CR¹=CR¹-CR¹=CR¹-, -CR¹=N-N=CR¹-, -CR¹=CR¹-CO-O-(CHR¹)_{p'}-, -CR¹=CR¹-O-CO-(CHR¹)_{p'}-, -(CHR¹)_{p'}-O-CO-CR¹=CR¹-, -(CHR¹)_{p'}-CO-O-CR¹=CR¹-, -CR¹=CR¹-CO-O-, -O-CO-CR¹=CR¹-, -CO-NR¹-, -NR¹-CO-, -CO-NR¹-NR¹-CO-, -C≡C-, -C≡C-C≡C-, -CO-S-, -S-CO-, -CR¹=N-, -N=CR¹-, -O-CO-, -CO-O-, -CR¹=CR¹-CO-, -CO-CR¹=CR¹-, -CR¹=CR¹-O-CO-, -CO-O-CR¹=CR¹-, -CH₂-CH₂-CO-O-, -O-CO-CH₂-CH₂-, -N=N-,
une liaison simple directe, ou un groupe 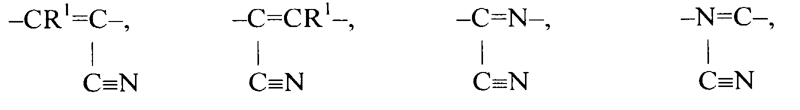 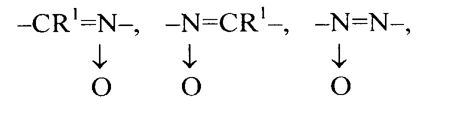 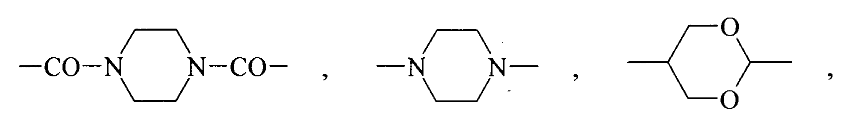 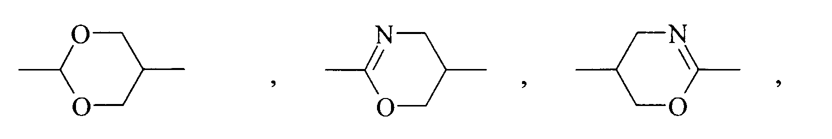 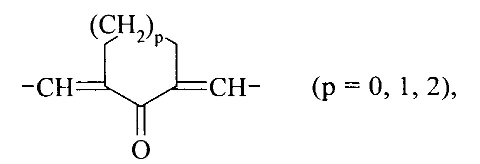 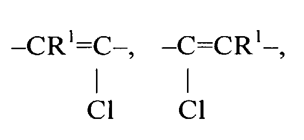 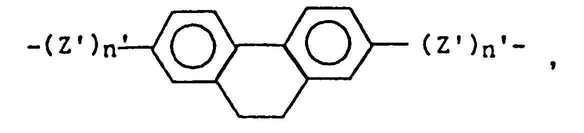 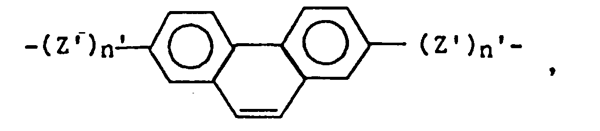 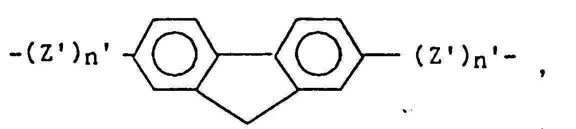 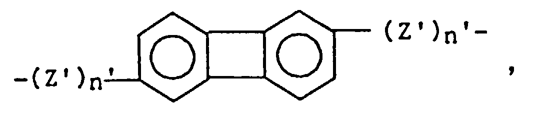 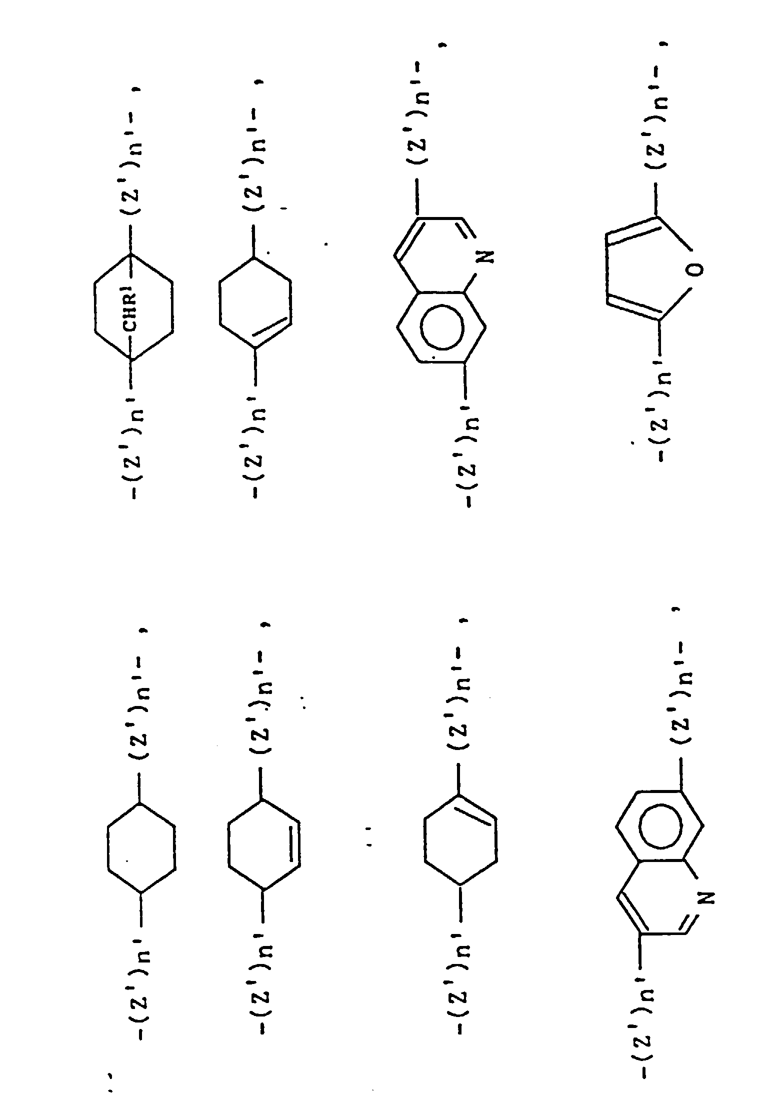 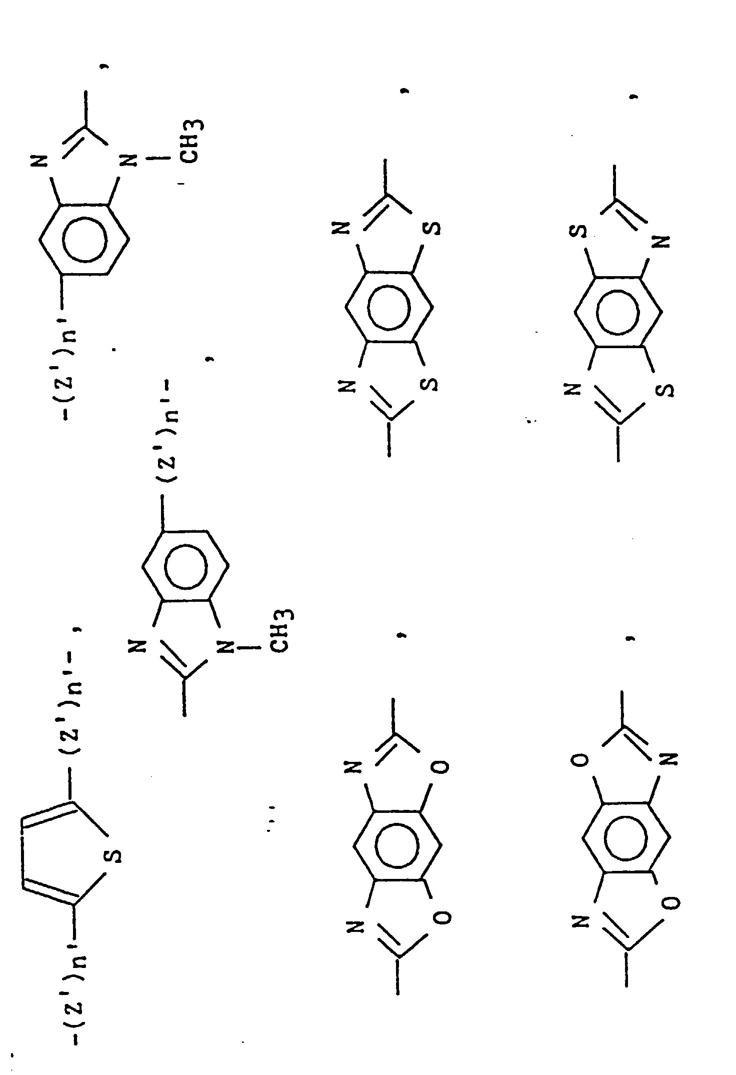
et chaque symbole X représente indépendamment un atome d'hydrogène ou un groupe hydrocarbyle ou hydrocarbyloxy comportant de 1 à 12 atomes de carbone, un atome d'halogène, ou un groupe -NO₂ ou -C≡N ;
Z² représente un groupe qui est un système cyclique ou bicyclique comportant de 5 à 12 atomes de carbone, qui peut être de type cycloaliphatique, polycycloaliphatique ou aromatique ou d'une combinaison de ces types ;
n vaut de 0 à 2 ;
chaque symbole Z' représente indépendamment un chaînon -CO-, -O-CO-, -CO-O-, -CO-NR¹- ou -NR¹-CO- ;
chaque symbole R¹ représente indépendamment un atome d'hydrogène ou un groupe hydrocarboné aliphatique comportant de 1 à 4 atomes de carbone ;
et chaque n' vaut indépendamment 0 ou 1 ;
ou toute combinaison de deux composés quelconques, ou plus, représentés par les formules XXV et XXVI ; et
(d) les composants (A) et (B) sont utilisés en des quantités qui fournissent un rapport du nombre d'atomes d'hydrogène actifs au nombre de groupes époxyde valant de 0,05/1 à 0,8/1 ;
sous réserve, si le composant (A) contient une polyglycidylamine comportant en moyenne plus de deux groupes glycidylamine par molécule, il n'y ait pas plus de 10 % du nombre total de groupes glycidyle contenus dans le composant (A) qui proviennent de ladite polyglycidylamine comportant en moyenne plus de deux groupes glycidylamine par molécule.

5. Polyglycidylamine prépolymérisée, conforme à la revendication 3 ou 4, dans laquelle :
(a) le composant (B) est l'un des suivants : hydroquinone, bisphénol A, 4,4'-dihydroxydiphénylméthane, 4,4'-thiodiphénol, 4,4'-sulfonyldiphénol, 4,4'-dihydroxydiphényléther, 4,4'-dihydroxybenzophénone, 1,1-bis(4-hydroxyphényl)-1-phényléthane, 3,3',5,5'-tétrachlorobisphénol A, 3,3'-diméthoxybisphénol A, 4,4'-dihydroxybiphényle, 4,4'-dihydroxy-α,α'-diéthylstilbène, 4,4'-dihydroxy-a-méthylstilbène, 4,4'-dihydroxybenzanilide, 4,4'-dihydroxy-2,2'-diméthylazoxybenzène, 4,4'-dihydroxy-α-cyanostilbène, téréphtalate de bis(4-hydroxyphényle), N,N'-bis(4-hydroxyphényl)téréphtalamide, téréphtalate de bis(4'-hydroxybiphényle), p-hydroxybenzoate de 4-hydroxyphényle, bis(4'-hydroxyphényl)-1,4-benzènediimine, p-hydroxybenzoate de 4'-hydroxybiphényle, 1,4-bis(4'-hydroxyphényl-1'-carboxamido)benzène, 1 ,4-bis(4'-hydroxyphényl-1'-carboxy)benzène, 4,4'-bis-(4"-hydroxyphényl-1"-carboxy)biphényle, acide téréphtalique, acide 4,4'-benzanilide-dicarboxylique, p-carboxybenzoate de 4-carboxyphényle, acide 4,4'-stilbène-dicarboxylique, 4,4'-dicarboxybiphényle, 4,4'-dicarboxydiphénylazométhine, aniline, 4'-sulfamyl-N-phénylbenzamide, 4'-sulfamyl-N-phényl-4-chlorobenzamide, benzoate de 4-aminophényle, 4-amino-N-phénylbenzamide, 4-(phénylamino)benzamide, 4-aminobenzoate de phényle, 4-aminobenzoate de biphényle, téréphtalate de phényle et de 4'-aminophényle, N,N'-diméthyl-4,4'-diaminodiphénylméthane, N,N'-diéthyl-4,4'-diaminodiphénylméthane, N,N'-diméthyl-4,4'-diaminobiphényle, N,N'-diméthyl-3,3'-diméthyl-4,4'-diaminobiphényle, N,N'-diméthyl-4,4'-diaminostilbène, N,N'-diméthyl-4,4'-diaminodiphénylsulfone, N,N'-diméthyl-4,4'-diaminobenzanilide, et N,N'-diméthyl-4,4'-diamino-N-méthylbenzanilide, ainsi que n'importe quelle combinaison de deux ou plus de ces composés ;
(b) les composants (A) et (B) sont utilisés en des quantités qui fournissent un rapport du nombre d'atomes d'hydrogène actifs au nombre de groupes époxyde valant de 0,1/1 à 0,5/1 ;
sous réserve, si le composant (A) contient une polyglycidylamine comportant en moyenne plus de deux groupes glycidylamine par molécule, il n'y ait pas plus de 5 % du nombre total de groupes glycidyle contenus dans le composant (A) qui proviennent de ladite polyglycidylamine comportant en moyenne plus de deux groupes glycidylamine par molécule.

6. Mélange comprenant :
(A) une ou plusieurs polyglycidylamines ou monoglycidylamines comportant un ou plusieurs fragments mésogènes, représentées par les formules I à VIII, la polyglycidylamine de formule II pouvant englober la tétraglycidylamine du 4,4'-diaminobiphényle et la tétraglycidylamine du 3,3'-diméthyl-4,4'-diaminobiphényle ; et
(B) un ou plusieurs polyépoxydes ne comportant pratiquement pas de fragments mésogènes.

7. Mélange conforme à la revendication 6, dans lequel :
(a) si le composant (A) est une polyglycidylamine représentée par l'une des formules I et III, le groupe symbolisé par R' comporte de 1 à 6 atomes de carbone et, si X représente un groupe hydrocarbyle ou hydrocarbyloxy, celui-ci comporte de 1 à 6 atomes de carbone et, si X représente un atome d'halogène, il s'agit d'un atome de chlore ou de brome ;
(b) si le composant (A) est une polyglycidylamine représentée par l'une des formules II, IV, V et VI, et si X représente un groupe hydrocarbyle ou hydrocarbyloxy, celui-ci comporte de 1 à 6 atomes de carbone et, si X représente un atome d'halogène, il s'agit d'un atome de chlore ou de brome ;
(c) si le composant (A) est une monoglycidylamine représentée par l'une des formules VII et VIII, le groupe symbolisé par R' comporte de 1 à 6 atomes de carbone et, si X représente un groupe hydrocarbyle ou hydrocarbyloxy, celui-ci comporte de 1 à 6 atomes de carbone et, si X représente un atome d'halogène, il s'agit d'un atome de chlore ou de brome ;
(d) le composant (B) est une résine époxy représentée par l'une des formules suivantes IX à XV : 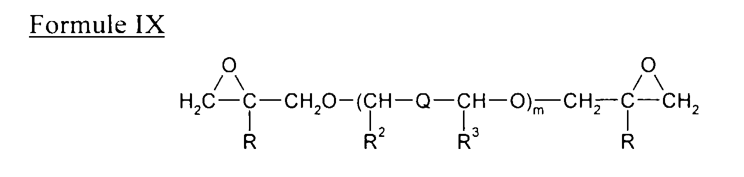 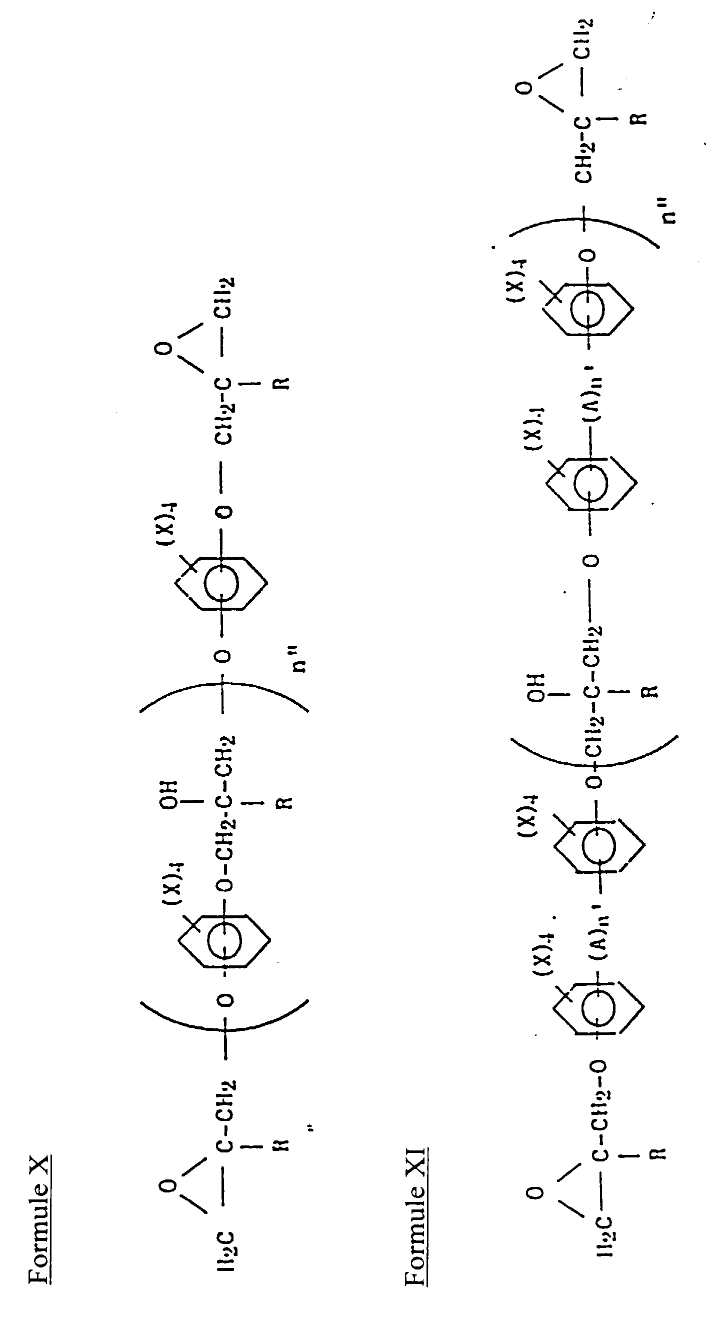 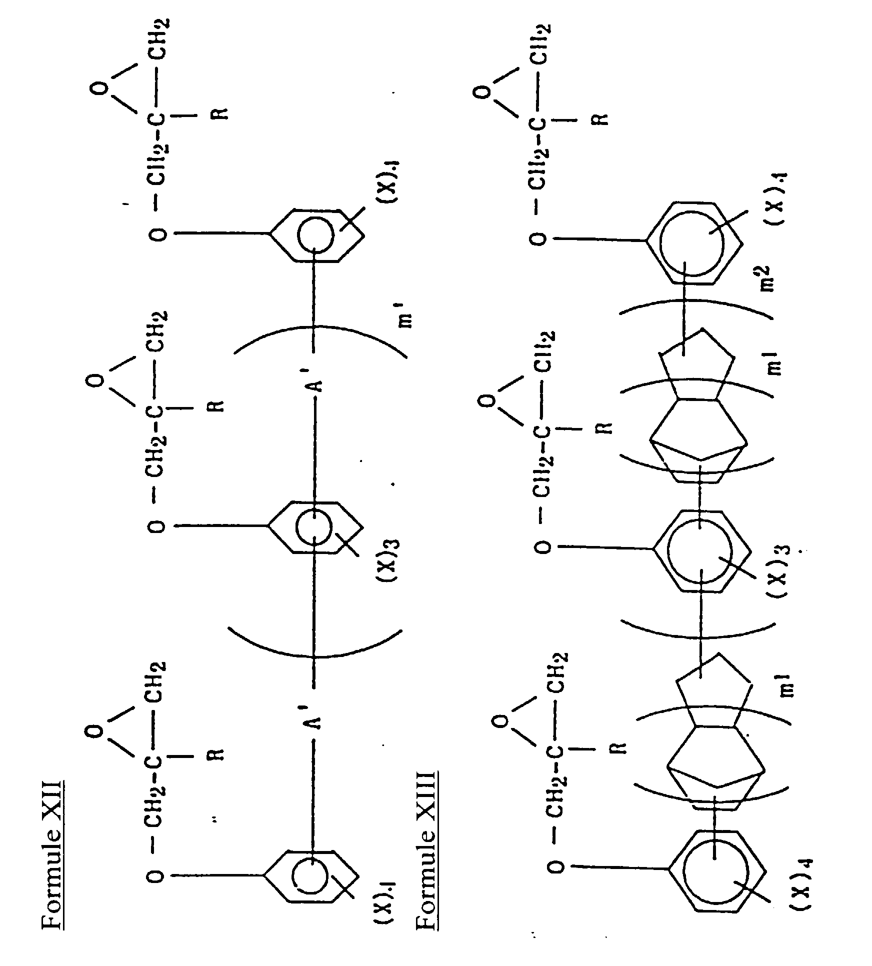 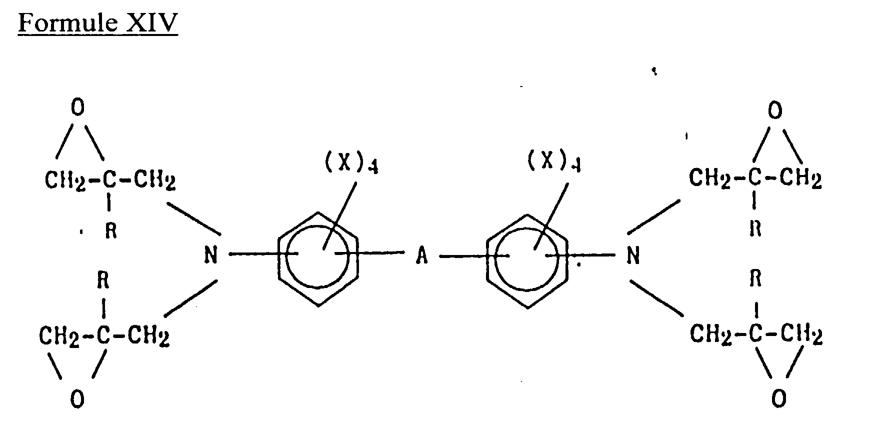 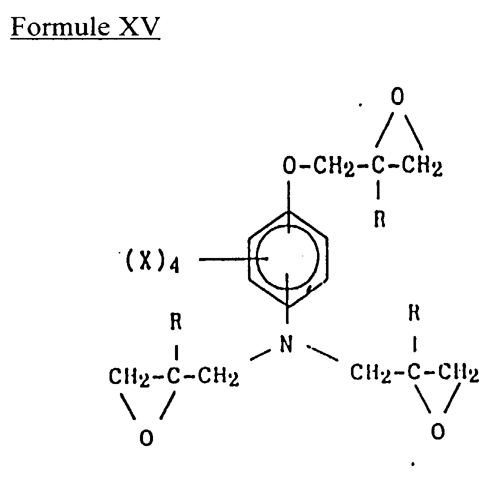 dans lesquelles formules :
chaque symbole A représente indépendamment un groupe hydrocarbyle divalent, comportant de 1 à 12 atomes de carbone, ou un chaînon -O-,-S-, -S-S-, -SO-, -SO₂-, ou -CO-;
chaque symbole A' représente indépendamment un groupe hydrocarboné divalent comportant de 1 à 6 atomes de carbone ;
Q représente une liaison simple ou un groupe -CH₂-S-CH₂-, -(CH₂)ₙ₁- ou 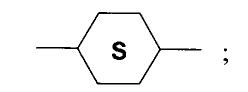
chaque symbole R représente indépendamment un atome d'hydrogène ou un groupe alkyle comportant de 1 à 4 atomes de carbone ;
chacun des symboles R² et R³ représente indépendamment un atome d'hydrogène ou un groupe hydrocarbyle ou halogénohydrocarbyle comportant de 1 à 6 atomes de carbone ;
chaque symbole X représente indépendamment un atome d'hydrogène ou un groupe hydrocarbyle ou hydrocarbyloxy comportant de 1 à 12 atomes de carbone, ou encore un atome d'halogène ou un groupe -NO₂ ou -C≡N ;
m vaut de 1 à 10 ;
m' vaut en moyenne de 0,01 à 12 ;
m¹ vaut en moyenne de 1 à 12 ;
m² vaut de 1 à 12 ;
n' vaut 0 ou 1 ;
n" vaut en moyenne de 0 à 3 ;
et n¹ vaut en moyenne de 1 à 10 ;
(e) le composant (A) se trouve présent en une quantité représentant de 1 à 99 % du poids total des composants (A) et (B), et le composant (B) se trouve présent en une quantité représentant de 99 à 1 % du poids total des composants (A) et (B).

8. Mélange conforme à la revendication 7, dans lequel le composant (A) est une diglycidylamine dérivée du N,N'-diméthyl-4,4'-diaminostilbène, du N,N'-diméthyl-4,4'-diaminobiphényle ou d'une de leurs combinaisons, et le composant (B) est l'éther diglycidylique du bisphénol A, l'éther diglycidylique du 4,4'-dihydroxydiphénylméthane, l'éther diglycidylique de la 4,4'-dihydroxybenzophénone, l'éther diglycidylique du 4,4'-thiodiphénol, ou l'éther diglycidylique du 1,1-bis(4-hydroxyphényl)-1-phényléthane (bisphénol AP), ou n'importe quelle combinaison de ces composés.

9. Mélange comprenant :
(A) une composition de polyglycidylamine prépolymérisée, qu'on a préparée en faisant réagir :
(1) une ou plusieurs polyglycidylamines comportant un ou plusieurs fragments mésogènes, lesdites polyglycidylamines étant de celles représentées par les formules I à VI, la polyglycidylamine de formule II pouvant englober la tétraglycidylamine du 4,4'-diaminobiphényle ou la tétraglycidylamine du 3,3'-diméthyl-4,4'-diaminobiphényle ; avec
(2) au moins un composé comportant en moyenne plus d'un atome d'hydrogène actif par molécule ;
les composants (1) et (2) étant utilisés en des quantités qui fournissent un rapport du nombre d'atomes d'hydrogène actifs au nombre de groupes époxyde valant de 0,01/1 à 0,95/1, sous réserve que, si le composant (A1) contient une polyglycidylamine comportant en moyenne plus de deux groupes glycidylamine par molécule, il n'y ait pas plus de 15 % du nombre total de groupes glycidyle contenus dans le composant (Al) qui proviennent de ladite polyglycidylamine comportant en moyenne plus de deux groupes glycidylamine par molécule ; et
(B) un ou plusieurs polyépoxydes ne comportant pratiquement pas de fragments mésogènes.

10. Mélange conforme à la revendication 9, dans lequel
(a) le composant (2) est l'un des suivants : hydroquinone, bisphénol A, 4,4'-dihydroxydiphénylméthane, 4,4'-thiodiphénol, 4,4'-sulfonyldiphénol, 4,4'-dihydroxydiphényléther, 4,4'-dihydroxybenzophénone, 1,1-bis(4-hydroxyphényl)-1-phényléthane, 3,3',5,5'-tétrachlorobisphénol A, 3,3'-diméthoxybisphénol A, 4,4'-dihydroxybiphényle, 4,4'-dihydroxy-a,a'-diéthylstilbène, 4,4'-dihydroxy-α-méthylstilbène, 4,4'-dihydroxybenzanilide, 4,4'-dihydroxy-2,2'-diméthylazoxybenzène, 4,4'-dihydroxy-α-cyanostilbène, téréphtalate de bis(4-hydroxyphényle), N,N'-bis(4-hydroxyphényl)téréphtalamide, téréphtalate de bis(4'-hydroxybiphényle), p-hydroxybenzoate de 4-hydroxyphényle, bis(4'-hydroxyphényl)-1,4-benzènediimine, p-hydroxybenzoate de 4'-hydroxybiphényle, 1,4-bis(4'-hydroxyphényl-1'-carboxamido)benzène, 1,4-bis(4'-hydroxyphényl-1'-carboxy)benzène, 4,4'-bis-(4"-hydroxyphényl-1"-carboxy)biphényle, acide téréphtalique, acide 4,4'-benzanilide-dicarboxylique, p-carboxybenzoate de 4-carboxyphényle, acide 4,4'-stilbène-dicarboxylique, 4,4'-dicarboxybiphényle, 4,4'-dicarboxydiphénylazométhine, aniline, 4'-sulfamyl-N-phénylbenzamide, 4'-sulfamyl-N-phényl-4-chlorobenzamide, benzoate de 4-aminophényle, 4-amino-N-phénylbenzamide, 4-(phénylamino)benzamide, 4-aminobenzoate de phényle, 4-aminobenzoate de biphényle, téréphtalate de phényle et de 4'-aminophényle, N,N'-diméthyl-4,4'-diaminodiphénylméthane, N,N'-diéthyl-4,4'-diaminodiphénylméthane, N,N'-diméthyl-4,4'-diaminobiphényle, N,N'-diméthyl-3,3'-diméthyl-4,4'-diaminobiphényle, N,N'-diméthyl-4,4'-diaminostilbène, N,N'-diméthyl-4,4'-diaminodiphénylsulfone, N,N'-diméthyl-4,4'-diaminobenzanilide, et N,N'-diméthyl-4,4'-diamino-N-méthylbenzanilide, ainsi que n'importe quelle combinaison de deux ou plus de ces composés ;
(b) les composants (1) et (2) sont utilisés en des quantités qui fournissent un rapport du nombre d'atomes d'hydrogène actifs au nombre de groupes époxyde valant de 0,1/1 à 0,5/1 ;
(c) le composant (A) se trouve présent en une quantité représentant de 10 à 50 % du poids total des composants (A) et (B) ; et
(d) le composant (B) se trouve présent en une quantité représentant de 90 à 50 % du poids total des composants (A) et (B).

11. Mélange comportant
(A) une ou plusieurs polyglycidylamines correspondant à une ou plusieurs des formules I à VI présentées dans la revendication 1, comportant en moyenne plus d'un groupe époxy vicinal par molécule et un ou plusieurs fragments mésogènes par molécule ; et
(B) une ou plusieurs monoglycidylamines, correspondant à l'une des formules VII et VIII présentées dans la revendication 1 ou à ces deux formules, ne comportant qu'un seul groupe époxy vicinal par molécule et comportant un ou plusieurs fragments mésogènes par molécule.

12. Mélange conforme à la revendication 11, dans lequel le composant (A) se trouve présent en une quantité représentant de 10 à 50 % du poids total des composants (A) et (B), et le composant (B) se trouve présent en une quantité représentant de 90 à 50 % du poids total des composants (A) et (B).

13. Composition durcissable comprenant au moins une composition conforme à la revendication 1, 2, 3, 4, 5, 6, 7, 8, 9, 10, 11 ou 12, et un agent durcissable approprié pour une telle composition, en une quantité qui lui permette de jouer efficacement son rôle de durcisseur.

14. Produit résultant du durcissement d'une composition durcissable conforme à la revendication 13.

15. Produit résultant de l'orientation d'une composition durcissable de la revendication 14, orientation effectuée soit avant le durcissement, soit pendant le durcissement, soit à la fois avant et pendant le durcissement.

16. Produit conforme à la revendication 15, dans lequel ladite orientation est effectuée au moyen d'un champ électrique ou d'un champ magnétique, ou par application de forces d'étirage ou de cisaillement.

17. Résine de type phénoxy, que l'on prépare en faisant réagir
(A) une ou plusieurs polyglycidylamines comportant un ou plusieurs fragments mésogènes, ces polyglycidylamines étant de celles qui sont représentées par les formules I, III, V et VI ; et
(B) au moins un composé comportant en moyenne plus d'un atome d'hydrogène actif par molécule ;
les composants (A) et (B) étant utilisés en des quantités qui fournissent un rapport du nombre des atomes d'hydrogène actifs au nombre de groupes époxyde valant de 0,96/1 à 1,05/1.
